(19) Europäisches Patentamt
European Patent Office
Office européen des brevets

(11) EP 3 508 197 A1

(12) **EUROPEAN PATENT APPLICATION**

(43) Date of publication:
**10.07.2019 Bulletin 2019/28**

(21) Application number: **18204805.8**

(22) Date of filing: **19.10.2010**

(51) Int Cl.:
***A61K 9/06*** $^{(2006.01)}$ ***A61K 47/10*** $^{(2017.01)}$
***A61K 31/506*** $^{(2006.01)}$ ***A61K 38/18*** $^{(2006.01)}$

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:
**21.10.2009 US 25378209 P**
**27.10.2009 US 25537909 P**
**28.10.2009 US 25578309 P**
**28.10.2009 US 25578009 P**
**21.01.2010 US 29417010 P**
**21.01.2010 US 29413810 P**
**14.07.2010 US 36428810 P**
**22.07.2010 US 36667710 P**

(62) Document number(s) of the earlier application(s) in accordance with Art. 76 EPC:
**10825524.1 / 2 490 722**

(71) Applicant: **Otonomy, Inc.**
**San Diego, CA 92121 (US)**

(72) Inventors:
- **YE, Qiang**
  **San Diego, CA 92130 (US)**
- **DELLAMARY, Luis, A.**
  **San Marcos, CA 92069 (US)**
- **PIU, Fabrice**
  **San Diego, CA 92131 (US)**

(74) Representative: **Campabadal i Monfà, Gemma**
**Wilson Sonsini Goodrich & Rosati LLP**
**Rue Montoyer 47**
**1000 Bruxelles (BE)**

Remarks:
•This application was filed on 07.11.2018 as a divisional application to the application mentioned under INID code 62.
•Claims filed after the date of filing of the application / after the date of receipt of the divisional application (Rule 68(4) EPC).

(54) **MODULATION OF GEL TEMPERATURE OF POLOXAMER-CONTAINING FORMULATIONS**

(57) Disclosed herein are methods for modulation of gel temperature of poloxamer-containing formulations. Also described herein are sustained release pharmaceutical formulations that gel upon contact with the body and are administered by direct application of these compositions and formulations onto or via perfusion into the targeted structure(s).

EP 3 508 197 A1

## Description

### CROSS-REFERENCE

This patent application claims the benefit of U.S. Provisional Application Ser. No. 61/253,782 filed October 21, 2009; U.S. Provisional Application Ser. No. 61/255,379 filed October 27, 2009;U.S. Provisional Application Ser. No. 61/255,780 filed October 28, 2009; U.S. Provisional Application Ser. No. 61/255,783 filed October 28, 2009; U.S. Provisional Application Ser. No. 61/297,138 filed January 21, 2010; U.S. Provisional Application Ser. No. 61/297,170 filed January 21, 2010; U.S. Provisional Application Ser. No. 61/364,288 filed July 14, 2010; and U.S. Provisional Application Ser. No. 61/366,677 filed July 22, 2010; all of which are incorporated by reference herein in their entirety.

### BACKGROUND OF THE INVENTION

Sustained release formulations that gel upon contact with the body are used in a variety of therapeutic applications.

### SUMMARY OF THE INVENTION

Described herein are sustained release formulations comprising thermosensitive polymers. Also described herein are methods wherein gelation temperature of formulations comprising thermosensitive polymers is manipulated with the addition of one or more gel temperature modifying agents to achieve a desired thereapeutically relevant gelation temperature (e.g., a formulation that gels upon contact with the body).

Provided herein, in some embodiments, are pharmaceutical formulations comprising an active agent, a thermosensitive polymer comprising polyoxyethylene and polyoxypropylene copolymers, and

a) having a syringable viscosity at time of administration suitable for administration via a 25 - 31 gauge needle;
b) having a gelation temperature between about 14 °C and about 42 °C ;
c) providing in vivo sustained release of a therapeutically effective amount of the active agent for a period of at least 3 days; and
(d) having less than 50 cfu of microbial agents per gram of the formulation;

provided that

(i) the formulation comprises less than 14.5% of the thermosensitive polymer by weight of the formulation and further comprises one or more gelation temperature increasing agents; or
(ii) the formulation comprises more than 25% of the thermosensitive polymer by weight of the formulation and further comprises one or more gelation temperature decreasing agents; or
(iii) the formulation comprises between about 5% and about 20% of the thermosensitive polymer by weight of the formulation, wherein the thermosensitive polymer has been purified, and optionally further comprises one or more gelation temperature increasing or gelation temperature decreasing agents; or
(iv) the formulation comprises between about 14.5% and about 25% of the thermosensitive polymer by weight of the formulation and further comprises one or more gelation temperature increasing or gelation temperature decreasing agents.

In some embodiments, the formulation provides an in vivo sustained release of a therapeutically effective amount of the active agent for a period of at least 5 days. In some embodiments, the formulation provides an in vivo sustained release of a therapeutically effective amount of the active agent for a period of at least 7 days. In some embodiments, the formulation provides an in vivo sustained release of a therapeutically effective amount of the active agent for a period of at least 10 days. In some embodiments, the formulation provides an in vivo sustained release of a therapeutically effective amount of the active agent for a period of at least 14 days.

In some embodiments, the formulation is administered at or in the vicinity of the round window membrane of the ear. In some embodiments, the in vivo sustained release occurs in the inner ear.

In some embodiments, the formulation is administered in the middle ear, away from the round window membrane. In some embodiments, the in vivo sustained release occurs in the middle ear.

In some embodiments, the formulation is administered into or in the vicinity of one or more sinonasal cavities. In some embodiments, the in vivo sustained release occurs in one or more sinonasal cavities or in the vicinity of one or more sinonasal cavities.

In some embodiments, the thermosensitive polymer is P407. In some embodiments, the formulation is substantially free of additional preservatives. In some embodiments, the formulation is substantially free of pyrogens. In some embodiments, the formulation comprises less than about 5 endotoxin units (EU) per kg of body weight of a subject. In some embodiments, the formulation is substantially free of additional tonicity agents.

In some embodiments, the formulation comprises a suspension of one or more multiparticulate active agents. In some embodiments, the multiparticulate active agent is a micronized active agent sterilized by dry-heat, irradiation or steam sterilization.

In some embodiments, the formulation has any individual product related impurity of no more than 1% by weight of the formulation. In some embodiments, the formulation has total product related impurities of no more than 2% by weight of the formulation.

In some embodiments, the active agent is a corticosteroid, or a salt or prodrug or solvate thereof.

In some embodiments, the corticosteroid is 21-acetoxypregnenolone, alclometasone, algestone, amcinonide, beclomethasone, betamethasone, budesonide, chloroprednisone, clobetasol, clobetasone, clocortolone, cloprednol, corticosterone, cortisone, cortivazol, deflazacort, desonide, desoximetasone, dexamethasone, diflorasone, diflucortolone, difluprednate, enoxolone, fluazacort, flucloronide, flumethasone, flunisolide, fluocinolone acetonide, fluocinonide, fluocortin butyl, fluocortolone, fluorometholone, fluperolone acetate, fluprednidene acetate, fluprednisolone, flurandrenolide, fluticasone propionate, formocortal, halcinonide, halobetasol propionate, halometasone, halopredone acetate, hydrocortamate, hydrocortisone, loteprednol etabonate, mazipredone, medrysone, meprednisone, methylprednisolone, mometasone furoate, paramethasone, prednicarbate, prednisolone, prednisolone 25-diethylamino-acetate, prednisolone sodium phosphate, prednisone, prednival, prednylidene, rimexolone, tixocortol, triamcinolone, triamcinolone acetonide, triamcinolone benetonide, or triamcinolone hexacetonide, or salt or prodrug thereof.

In some embodiments, the corticosteroid is dexamethasone, prednisolone, methylprednisolone, triamcinolone, or a salt or prodrug or solvate thereof, or a combination thereof. In some embodiments, the corticosteroid is dexamethasone, or a salt or prodrug or solvate thereof. In some embodiments, the dexamethasone is dexamethasone sodium phosphate or dexamethasone acetate.

In some embodiments, the dexamethasone, or salt or prodrug or solvate thereof, is present in an amount from about 0.05% to about 40% by weight of the formulation. In some embodiments, the dexamethasone, or salt or prodrug or solvate thereof, is present in an amount from about 0.1% to about 30% by weight of the formulation. In some embodiments, the dexamethasone, or salt or prodrug or solvate thereof, is present in an amount from about 0.5% to about 15% by weight of the formulation.

In some embodiments, the formulation provides an in vivo sustained release of a therapeutically effective amount of dexamethasone for a period of at least 5 days. In some embodiments, the formulation provides an in vivo sustained release of a therapeutically effective amount of dexamethasone for a period of at least 7 days. In some embodiments, the formulation provides an in vivo sustained release of a therapeutically effective amount of dexamethasone for a period of at least 10 days. In some embodiments, the formulation provides an in vivo sustained release of a therapeutically effective amount of dexamethasone for a period of at least 14 days.

In some embodiments, the active agent is an antimicrobial agent. In some embodiments, the antimicrobial agent is an antibiotic.

In some embodiments, the antibiotic is amikacin, gentamicin, kanamycin, neomycin, netilmicin, streptomycin, tobramycin, paromycin, geldanamycin, herbimycin, loracarbef, ertapenem, doripenem, imipenem, meropenem, cefaclor, cefamandole, cefotoxin, cefprozil, cefuroxime, cefixime, cefdinir, cefditoren, cefpodoxime, ceftazidime, ceftibuten, ceftizoxime, ceftriaxone, cefepime, ceftobirprole, vancomycin, azithromycin, clarithromycin, dirithromycin, erythromycin, roxithromycin, troleandomycin, telithromycin, spectinomycin, aztreonam, amoxicillin, ampicillin, azociling, carbenicillin, cloxacillin, dicloxacillin, flucloxacillin, mezlocillin, meticillin, nafcillin, oxacillin, peperacillin, ticarcillin, bacitracin, colistin, polymyxin B, ciprofloxacin, clavulanic acid, enoxacin, gatifloxacin, levofloxacin, lomefloxacin, moxifloxacin, nonfloxacin, ofloxacin, trovafloxacin, grepafloxacin, sparfloxacin, AL-15469A, AL-38905, OP-145, afenide, prontosil, sulfacetamide, sulfamethiazole, sulfanilimide, sulfasalazine, sulfisoxazole, trimethoprim, cotrimoxazole, demeclocycline, doxycycline, minocycline, oxytetracycline, tetraycline, linezolid, arsogebanubem chloramphenicol, clindamycin, lincomycin, ethambutol, fosfomycin, fusidic acid, furazolidone, isoniazid, linezolid, metronidazole, mupirocin, nitrofurantoin, platensimycin, pyrazinamide, quinupristin, dalfopristin, rifampicin, thamphenicol, tinidazole, amoxicillin+clavulanic acid, Maximin H5, Dermcidin, Cecropins, andropin, moricin, ceratotoxin, melittin, Magainin, dermaseptin, bombinin, brevinin-1,esculentins and buforin II, CAP18, LL37 , abaecin, apidaecins, prophenin, indolicidin, brevinins, protegrin, tachyplesins, defensins, drosomycin, alamethicin, pexiganan or MSI-78, MSI-843, MSI-594, polyphemusin, colicin, pyocin, klebicin, subtilin, epidermin, herbicolacin, brevicin, halocin , agrocin, alveicin, carnocin, curvaticin, divercin ,enterocin, enterolysin, erwiniocin, glycinecin, lactococin, lacticin, leucoccin, mesentericin, pediocin, plantaricin, sakacin, sulfolobicin, vibriocin, warnerinand, nisin, or a salt or cocrystal, or prodrug or solvate thereof, or a combination thereof.

In some embodiments, the antibiotic agent is ciprofloxacin, amoxicillin, amoxicillin+clavulanic acid, moxifloxacin or ofloxacin. In some embodiments, the antibiotic agent is ciprofloxacin or ciprofloxacin hydrate. In some embodiments, the ciprofloxacin or ciprofloxacin hydrate is present in an amount between about 0.1 to about 20% by weight of the

formulation.

In some embodiments, the formulation provides an in vivo sustained release of a therapeutically effective amount of ciprofloxacin for a period of at least 5 days. In some embodiments, the formulation provides an in vivo sustained release of a therapeutically effective amount of ciprofloxacin for a period of at least 7 days. In some embodiments, the formulation provides an in vivo sustained release of a therapeutically effective amount of ciprofloxacin for a period of at least 10 days. In some embodiments, the formulation provides an in vivo sustained release of a therapeutically effective amount of ciprofloxacin for a period of at least 14 days.

In some embodiments, the gel temperature increasing agent or gel temperature decreasing agent is selected from P188, P338, cyclodextrin, Tween 20, Tween 40, Tween 65, Tween 80, Tween 85, sodium oleate, sodium caprate, sodium caprylate and PEG.

Also provided herein are kits comprising (a) sterilized multiparticulate active agent powder and (b) a solution comprising a thermosensitive polymer, wherein (a) and (b), when combined, form a formulation described above.

In some embodiments, the formulations described above comprise a higher concentration of an active agent than the actual administered dose. In some of such embodiments, the formulation is diluted prior to administration. Accordingly, in some embodiments, the percentage by weight amount of active agent in the administered formulation is different from the percentage by weight amount of active agent in the prepared formulation.

In one aspect, provided herein are pharmaceutical formulations comprising

(a) less than 14.5% of a thermosensitive polymer by weight of the formulation and further comprising one or more gelation temperature increasing agents;
(b) water;
(c) between about 0.2% and about 20% of micronized dexamethasone by weight of the administered formulation;
(d) having a gelation temperature between about 14 °C and about 42 °C;
(e) having less than 50 cfu of microbial agents per gram of the formulation; and
(f) having a syringable viscosity at time of administration suitable for administration via a 25 - 31 gauge needle.

In one aspect, provided herein are pharmaceutical formulations comprising

(a) more than 25% of a thermosensitive polymer by weight of the formulation and further comprising one or more gelation temperature decreasing agents;
(b) water;
(c) between about 0.2% and about 20% of micronized dexamethasone by weight of the administered formulation;
(d) having a gelation temperature between about 14 °C and about 42 °C;
(e) having less than 50 cfu of microbial agents per gram of the formulation; and
(f) having a syringable viscosity at time of administration suitable for administration via a 25 - 31 gauge needle.

In one aspect, provided herein are pharmaceutical formulations comprising

(a) between about 5% to about 20% of a purified thermosensitive polymer by weight of the formulation, and optionally further comprising one or more gelation temperature increasing agents;
(b) water;
(c) between about 0.2% and about 20% of micronized dexamethasone by weight of the administered formulation;
(d) having a gelation temperature between about 14 °C and about 42 °C;
(e) having less than 50 cfu of microbial agents per gram of the formulation; and
(f) having a syringable viscosity at time of administration suitable for administration via a 25 - 31 gauge needle.

In one aspect, provided herein are pharmaceutical formulations comprising

(a) between about 14.5% and about 25% of a thermosensitive polymer by weight of the formulation and further comprising one or more gelation temperature increasing or gelation temperature decreasing agents;
(b) water;
(c) between about 0.2% and about 20% of micronized dexamethasone by weight of the administered formulation;
(d) having a gelation temperature between about 14 °C and about 42 °C;
(e) having less than 50 cfu of microbial agents per gram of the formulation; and
(f) having a syringable viscosity at time of administration suitable for administration via a 25 - 31 gauge needle.

In one aspect, provided herein are pharmaceutical formulations comprising

(a) less than 14.5% of a thermosensitive polymer by weight of the formulation and further comprising one or more gelation temperature increasing agents;
(b) water;
(c) between about 0.2% and about 20% of ciprofloxacin by weight of the administered formulation;
(d) having a gelation temperature between about 14 °C and about 42 °C;
(e) having less than 50 cfu of microbial agents per gram of the formulation; and
(f) having a syringable viscosity at time of administration suitable for administration via a 25 - 31 gauge needle.

In one aspect, provided herein are pharmaceutical formulations comprising

(a) more than 25% of a thermosensitive polymer by weight of the formulation and further comprising one or more gelation temperature decreasing agents;
(b) water;
(c) between about 0.2% and about 20% of ciprofloxacin by weight of the administered formulation;
(d) having a gelation temperature between about 14 °C and about 42 °C;
(e) having less than 50 cfu of microbial agents per gram of the formulation; and
(f) having a syringable viscosity at time of administration suitable for administration via a 25 - 31 gauge needle.

In one aspect, provided herein are pharmaceutical formulations comprising

(a) between about 5% to about 20% of a purified thermosensitive polymer by weight of the formulation, and optionally further comprising one or more gelation temperature increasing agents;
(b) water;
(c) between about 0.2% and about 20% ciprofloxacin by weight of the administered formulation;
(d) having a gelation temperature between about 14 °C and about 42 °C;
(e) having less than 50 cfu of microbial agents per gram of the formulation; and
(f) having a syringable viscosity at time of administration suitable for administration via a 25 - 31 gauge needle.

In one aspect, provided herein are pharmaceutical formulations comprising

(a) between about 14.5% and about 25% of a thermosensitive polymer by weight of the formulation and further comprising one or more gelation temperature increasing or gelation temperature decreasing agents;
(b) water;
(c) between about 0.2% and about 20% of ciprofloxacin by weight of the administered formulation;
(d) having a gelation temperature between about 14 °C and about 42 °C;
(e) having less than 50 cfu of microbial agents per gram of the formulation; and
(f) having a syringable viscosity at time of administration suitable for administration via a 25 - 31 gauge needle.

In one aspect, provided herein are pharmaceutical formulations comprising

(a) less than 14.5% of a thermosensitive polymer by weight of the formulation and further comprising one or more gelation temperature increasing agents;
(b) water;
(c) between about 0.001% and about 5% of micronized dexamethasone by weight of the administered formulation;
(d) between about 0.1% and about 10% of ciprofloxacin, moxifloxacin or ofloxacin by weight of the administered formulation.
(e) having a gelation temperature between about 14 °C and about 42 °C;
(f) having less than 50 cfu of microbial agents per gram of the formulation; and
(g) having a syringable viscosity at time of administration suitable for administration via a 25 - 31 gauge needle.

In one aspect, provided herein are pharmaceutical formulations comprising

(a) more than 25% of a thermosensitive polymer by weight of the formulation and further comprising one or more gelation temperature decreasing agents;
(b) water;
(c) between about 0.001% and about 5% of micronized dexamethasone by weight of the administered formulation;
(d) between about 0.1% and about 10% of ciprofloxacin, moxifloxacin or ofloxacin by weight of the administered formulation.

(e) having a gelation temperature between about 14 °C and about 42 °C;
(f) having less than 50 cfu of microbial agents per gram of the formulation; and
(g) having a syringable viscosity at time of administration suitable for administration via a 25 - 31 gauge needle.

In one aspect, provided herein are pharmaceutical formulations comprising

(a) between about 5% to about 20% of a purified thermosensitive polymer by weight of the formulation, and optionally further comprising one or more gelation temperature increasing agents;
(b) water;
(c) between about 0.001% and about 5% of micronized dexamethasone by weight of the administered formulation;
(d) between about 0.1% and about 10% of ciprofloxacin, moxifloxacin or ofloxacin by weight of the administered formulation.
(e) having a gelation temperature between about 14 °C and about 42 °C;
(f) having less than 50 cfu of microbial agents per gram of the formulation; and
(g) having a syringable viscosity at time of administration suitable for administration via a 25 - 31 gauge needle.

In one aspect, provided herein are pharmaceutical formulations comprising

(a) between about 14.5% and about 25% of a thermosensitive polymer by weight of the formulation and further comprising one or more gelation temperature increasing or gelation temperature decreasing agents;
(b) water;
(c) between about 0.001% and about 5% of micronized dexamethasone by weight of the administered formulation;
(d) between about 0.1% and about 10% of ciprofloxacin, moxifloxacin or ofloxacin by weight of the administered formulation.
(e) having a gelation temperature between about 14 °C and about 42 °C;
(f) having less than 50 cfu of microbial agents per gram of the formulation; and
(g) having a syringable viscosity at time of administration suitable for administration via a 25 - 31 gauge needle.

Further provided herein is the use of any formulation described above in the manufacture of a medicament for treatment of any otic and/or sinonasal and/or nasopharyngeal disorder described herein.

Provided herein, in some embodiments, are methods for treating an otic disorder selected from Meniere's disease, sudden sensorineural hearing loss, noise induced hearing loss, age-related hearing loss, vertigo, tinnitus, otosclerosis, autoimmune ear disease (AIED), otitis media, and otitis externa comprising administration of any formulation described herein to an individual in need thereof.

Provided herein, in some embodiments, are methods for treating a sinonasal or nasopharyngeal disorder selected from sinonasal polyposis, allergic fungal sinusitis, nasal polyps, paranasal sinus cancers, nasopharyngeal cancers, epistaxis, anosmia, repiratory papilloma, papilloma virus induced tumors (e.g., inverting papillomas), recurrent respiratory papillomas, reduction of post-surgical complications associated with sinonasal surgery (inferior turbinate removal), chronic sinusitis, and/or chronic rhinosinusitis comprising administration of any formulation described herein to an individual in need thereof.

**BRIEF DESCRIPTION OF FIGURES**

**Figure 1.** is an illustrative comparison of non-sustained release and sustained release formulations.
**Figure 2** are illustrative predicted tunable releases of an active agent from four compositions.
**Figure 3** are illustrative inner ear pharmacokinetics with increasing concentrations of a steroid drug in sustained release formulations.
**Figure 4** is an illustration of in vitro mean dissolution time with increasing concentrations of steroid drug in sustained release formulations.
**Figure 5** is an illustration of in vitro mean dissolution time of high versus low solubility drug substances and solution versus gel formulations.
**Figure 6** is an illustrative comparison of in vitro release of zoledronate from a formulation comprising zoledronate versus a formulation comprising a zoledronate-calcium complex.
**Figure 7** illustrates the mean dissolution time (MDT) for certain formulations.
**Figure 8** illustrates the MRT for dexamethasone (Dex), dexamethasone sodium phosphate (DSP), and dexamethasone acetate (DA) from certain formulations following intratympanic injection in guinea pigs.

**Figure 9** illustrates the MRT for soluble form or methylprednisolone (MPS) and insoluble form of methylprednisolone (MP) from certain formulations following intratympanic injection in guinea pigs.

**Figure 10** illustrates the MRT for 0.6% L-701324 in 17% poloxamer 407 formulation following intratympanic injection in guinea pigs.

**Figure 11** illustrates the MRT for 0.5% SP-600125 in 17% poloxamer 407 formulation following intratympanic injection in guinea pigs.

**Figure 12** illustrates the MRT for 2% meclizine in 17% poloxamer 407 formulation following intratympanic injection in guinea pigs.

**Figure 13** illustrates a substantially uniform distribution of dexamethasone in the chochlea from a formulation comprising a thermosensitive polymer and the uneven distribution of dexamethasone in the cochlea from a dexamethasone solution not containing a thermosensitive polymer following intratympanic injection.

**Figure 14** illustrates the effect of poloxamer 407 formulations comprising varying concentrations of dexamethasone on the ABR hearing thresholds in guinea pigs following intratympanic administration. Hearing was tested by recording the brainstem activity in response to a known auditory stimulus, under general anesthesia, in a sound isolation booth. An earphone (EC1, Tucker Davis Technologies) was fitted into the ear just above the external auditory canal orifice. Three subcutaneous needle electrodes were used to measure the brainstem activity, placed in the postauricular area of the ear (reference), on the vertex of the skull (active) and in the hind leg (ground). The acoustic stimulus was generated using the SigGen system (Tucker Davis Technologies) and consisted of 10ms auditory clicks (frequency range 100Hz - 30 KHz). Responses were averaged from 512 presentations with sound level up to 90 dB SPL with increments of 5 dB SPL. Responses were acquired using BioSig (Tucker Davis Technologies) and threshold was determined as the average between the non observable and smallest observable intensity.

**Figure 15** illustrates a comparison of in vitro release characteristics of otic agents from 15-18% poloxamer formulations comprising water and 50% poloxamer formulation comprising water+ethanol as solvent upon administration to the middle ear in guinea pigs.

**Figure 16** illustrates a comparison of in vivo release characteristics of otic agents from 15-18% poloxamer formulations comprising water and 50% poloxamer formulation comprising water+ethanol as solvent upon administration to the middle ear in guinea pigs.

**Figure 17** illustrates middle ear drug concentration of ciprofloxacin and dexamethasone from 15-18% poloxamer formulations comprising water and 50% poloxamer formulation comprising water+ethanol as solvent upon administration to the middle ear in guinea pigs in dry ear conditions.

**Figure 18** illustrates middle ear drug concentration of ciprofloxacin and dexamethasone from 15-18% poloxamer formulations comprising water and 50% poloxamer formulation comprising water+ethanol as solvent upon administration to the middle ear in guinea pigs in wet ear conditions.

**Figure 19** illustrates middle ear fluid levels of ciprofloxacin and dexamethasone from 15-18% poloxamer formulations comprising water and 50% poloxamer formulation comprising water+ethanol as solvent upon administration to the middle ear in guinea pigs in dry ear conditions.

**Figure 20** is a comparison of release profile for formulations described herein and Ciprodex® Otic solution.

**Figure 21** illustrates effect of formulations described herein and Ciprodex® Otic solution on auditory function in guinea pigs following intratympanic administration in guinea pigs. Administration of Ciprodex® Otic causes transient hearing shift of 20-25dB, improving by day 7. Administration of a formulation comprising dexamethasone, ciprofloxacin, 15-18% P407 and water causes minimal hearing shift (5-10 dB), resolved by day 7. Administration of a formulation comprising dexamethasone, ciprofloxacin, 50% P407 and water+ethanol causes transient hearing shift of 40-50 dB, resolved by day 3.

**Figure 22** illustrates middle ear pharmacokinetics following intratympanic administration of varying poloxamer concentrations all containing 0.5% Cipro and 0.1% DEX. Guinea pigs (n=4) received a single intratympanic injection (50 $\mu$l). Free drug levels in the middle ear were quantified at the indicated times. Data are presented as mean $\pm$ SEM. Poloxamer concentrations are as follows: 16% poloxamer (diamond), 17% poloxamer (square), 19% poloxamer (triangle) and 21% poloxamer (circle).

**Figure 23** illustrates middle ear pharmacokinetics following intratympanic administration of various doses of P407 formulations. Guinea pigs (n=4) received a single intratympanic injection (50 $\mu$l) of either 0.5% Cipro 0.1% DSP (circle) or 1% Cipro 0.1% DSP (square). Free drug levels in the middle ear were quantified at the indicated times. Data are presented as mean $\pm$ SEM.

**Figure 24** illustrates middle ear pharmacokinetics following intratympanic administration of various doses of P407 formulations. Guinea pigs (n=4) received a single intratympanic injection (50 $\mu$l) of either 0.3% Cipro 0.1% DEX (diamond), 0.6% Cipro 0.2% DEX (circle), 2% Cipro 0.7% DEX (triangle) or 6% Cipro, 2% DEX (square). Free drug levels in the middle ear were quantified at the indicated times. Data are presented as mean $\pm$ SEM.

**Figure 25** illustrates tissue-bound middle ear drug levels following intratympanic administration of P407 formulations. Guinea pigs (n=4) received a single intratympanic injection (50 $\mu$l) of 0.3% Cipro 0.1% DEX. Tissue-bound drug

levels in the middle ear epithelium were quantified at the indicated times. Data are presented as mean ± SEM. Black bars: ciprofloxacin, white bars: dexamethasone.

**Figure 26** illustrates middle ear pharmacokinetics following intratympanic administration of various volumes of a formulation. Guinea pigs (n=4) received a single intratympanic injection of either 25 μl (circle), 50 μl (square) or 75 μl (triangle) of 0.3% Cipro 0.1% DEX. Drug levels in the middle ear were quantified at the indicated times. Data are presented as mean ± SEM.

**Figure 27** illustrates Hearing evaluation of P407 formulations administered intratympanically. Guinea pigs (n=4) received a single intratympanic administration (50 μl) of 0.2% CiproHCl, 0.2% DEX. Hearing evaluation was conducted using Auditory Brainstem Response across frequencies. Data are presented as mean + SEM (n=4) of ABR threshold.

**Figure 28 A-G** illustrates the hemolysis in guinea pig red blood cells when exposed to serially diluted poloxamer solutions.

## DETAILED DESCRIPTION OF THE INVENTION

Local administration of active agents reduces toxicities and/or side effects associated with systemic administration. The ability to provide sustained release of active agents at localized sites in the body is desirable for current treatment modalites that require multiple daily dosing and/or prolonged dosing because such sustained release treatment regimens reduce dosing frequency thereby improving patient compliance. Provided herein are sustained release active agent pharmaceutical formulations that gel upon contact with the body. Such formulations are suitable for local administration at various target sites in the body, including and not limited to the ear, the eye, the sinonasal cavities, the gastrointestinal tract, the buccal cavity, the intrathecal and/or intracranial cavities, synovial cavities or the like.

Provided herein are formulations that are manufactured with low bioburden or sterilized with stringent sterilty requirements and are suitable for administration in vivo. In some embodiments, the biocompatible compositions described herein are substantially free of pyrogens and/or microbes.

Where the current standard of care requires multiple administrations of drops or injections (e.g. intratympanic injections) over several days (e.g., up to two weeks), including schedules of receiving multiple injections per day, formulations described herein are administered at reduced dosing frequency compared to the current standard of care. In certain instances, a reduced frequency of administration alleviates discomfort caused by multiple injections in individuals undergoing treatment for a disease, disorder or condition and/or improves patient compliance during long-term therapy. In some embodiments, the formulations described herein are administered locally at a target site and prolong residence time of an active agent at the site of administration.

Localized administration allows an active agent to reach a target organ (e.g., inner ear) and reduces or eliminates systemic accumulation of the active agent. In some instances, local administration provides a higher therapeutic index for an active agent that would otherwise have dose-limiting systemic toxicity. In addition, localized treatment also affords the use of previously undesired therapeutic agents, including agents with poor pK profiles, poor uptake, low systemic release, and/or toxicity issues.

In some instances, a disadvantage of liquid formulations is their propensity to wash way in physiological media and cause rapid clearance of the formulation from the site of administration. Provided herein, in certain embodiments, are formulations comprising polymers that gel at about body temperature and remain in contact with the target surfaces (e.g., the sinonasal epithelium) for extended periods of time. Formulations described herein avoid attenuation of therapeutic benefit due to drainage or washing away of active agents.

Accordingly, provided herein are pharmaceutical formulations that meet stringent criteria including pH, ionic balance, and/or sterility. Such formulations are designed to be isotonic with biological fluids. In some embodiments, the biocompatible compositions described herein are formulated with minimum excipients and thus reduce or eliminate irritation or toxicity at the site of administration. Further, the formulations comprise thermosensitive polymers that are biocompatible and/or otherwise non-toxic. In some embodiments, the thermosensitive gel is biodegradable and/or bioeliminated (e.g., the copolymer is eliminated from the body by a biodegradation or bioelimination process, e.g., elimination in the urine, the feces or the like).

### Administration in the ear

In some embodiments, the sustained release formulations described herein are suitable for administration to the ear for the treatment of otic disorders including and not limited to Meniere's disease, sudden sensorineural hearing loss, noise induced hearing loss, age-related hearing loss, vertigo, tinnitus, otosclerosis, autoimmune ear disease (AIED), otitis media, otitis externa, ear infections and the like.

The environment of the inner ear is an isolated environment. The endolymph and the perilymph are static fluids and are not in contiguous contact with the circulatory system. In certain instances, even trace amounts of pyrogens

and/or microbes can trigger infections and related physiological changes in the isolated microenvironment of the inner ear. When the tympanic membrane is intact, the air of the middle ear is not in direct contact with the atmosphere outside the body. In certain instances, even trace amounts of pyrogens and/or microbes can trigger infections and related physiological changes in the isolated microenvironment of the inner and/or middle ear. The compositions described herein are sterile compositions suitable for administration to the isolated environment of the inner ear and/or into the middle ear and provide sustained release of an active agent at the target site.

In some embodiments, for application to the inner ear, the formulations described herein are administered (e.g., via intratympanic injection, as ear drops in the ear canal, direct perfusion during otic surgery) behind and/or through the tympanic membrane at or near the round window membrane and/or the ossicular chain. In some embodiments, sustained release formulations described herein are injected as a liquid into the tympanic cavity in the vicinity of the round window membrane and gel and/or form thickened liquids upon contact with auditory surfaces.

In some other embodiments, for application to the middle ear, the formulations described herein are administered (e.g., via intratympanic injection, as ear drops in the ear canal, direct perfusion during otic surgery) behind and/or through the tympanic membrane so that they are not in contact with the round window membrane and/or the ossicular chain. In some embodiments, sustained release formulations described herein are administered in the tympanic cavity, away from the round window membrane. In some embodiments, the formulations are deposited, by injection, on the walls of the middle ear and gel and/or form thickened liquids upon contact with auditory surfaces.

In other embodiments, the formulations are administered as a paint (e.g., the formulations are smeared on the walls of the tympanic cavity using a cotton-tipped stick). In some embodiments, the formulations are sprayed (e.g., as a fluid, a foam or the like) into the middle ear cavity (e.g., when the tympanic membrane has ruptured). In some embodiments, the formulations are administered on the auditory walls and not on auditory bones (e.g., the ossicles). In some embodiments, the compositions described herein are administered in the outer ear, e.g., in the ear canal.

In some embodiments, formulations described herein are low viscosity liquid compositions suitable for administration as ear drops. Following administration, the formulations form thickened liquids and/or gels that do not wash away from the middle ear and/or the round window membrane and provide sustained release of active agents, even in the presence of biological fluids such as middle ear fluids present in individuals suffering from otitis media with effusion. By way of example, when formulations comprising a copolymer of polyoxyethyelene and polyoxypropylene are administered to an individual suffering from otitis media with effusion, the formulations do not wash away, and remain in contact with the walls of the middle ear preventing infection and/or further accumulation of mucus. In certain other embodiments, the formulations are deposited on auditory bones (e.g., as a treatment for otosclerosis).

**Administration in sinonasal structures**

In some embodiments, the sustained release formulations described herein are suitable for instrasinusoidal, intranasal, and/or intranasopharyngeal administration for the treatment of sinusoidal, nasal, and/or nasopharynx disorders including and not limited, sinonasal polyposis, allergic fungal sinusitis, nasal polyps, paranasal cancers, nasopharyngeal cancers, epistaxis, anosmia, respiratory papilloma, papilloma virus induced tumors (e.g., inverting papillomas), recurrent respiratory papillomas, reduction of post-surgical complications associated with sinonasal surgery (inferior turbinate removal), chronic sinusitis, chronic rhinosinusitis and the like.

In some embodiments, sustained release formulations described herein are administered in a sinusoidal cavity and/or in the vicinity of the sinusoidal cavities, including the the ethmoid, maxillary, frontal and/or sphenoid sinusoidal cavities and other anatomical or physiological structures located within the sinonasal cavities such as nasal polyps, turbinates, site of surgical wound or the like. There is considerable anatomical variation in sinuses amongst individuals. Current treatment regimens for sinusodial conditions include nasal sprays and/or nasal irrigation for topical drug administration into the paranasal sinuses. However, nasal sprays and/or nasal irrigation are not effective in delivering a solution in the paranasal sinuses and/or the sinusoidal cavities. Moreover, the solutions drain out of the nasal passages. In some other embodiments, the sustained release formulations described herein are administered in nasal cavities and provide sustained release without attenuation of therapeutic benefit due to drainage of formulation via nasal passages. In yet other embodiments, the sustained release formulations described herein are administered in the nasopharyngeal region.

In some embodiments, sustained release formulations described herein are administerd in conjunction with a surgical procedure, e.g., in combination with tympanostomy, sinonasal polypectomy, balloon rhinoplasty or the like. In some embodiments, sustained release formulations described herein are suitable for use with certain devices such as AdvaCoat™ Sinus Dressing and the AdvaCoat™ Rx for chronic rhinosinusitis (available from Carbylan BioSurgery, Inc), catheter-based tools such as the Balloon Sinuplasty™ devices available from Acclarent, or bioabsorbable drug eluting stents such as a stent available from Intersect ENT, Inc.

In some embodiments, a formulation described herein comprises at least about 5.0% and not more than about 50% of a thermosensitive polymer (e.g., polyoxyethylene-polyoxypropylene triblock copolymer) by weight of the composition. In some embodiments, a formulation described herein comprises at least about 5.0% and not more than about

40% of a thermosensitive polymer (e.g., polyoxyethylene-polyoxypropylene triblock copolymer) by weight of the composition. In some embodiments, a formulation described herein comprises at least about 10.0% and not more than about 35% of a thermosensitive polymer (e.g., polyoxyethylene-polyoxypropylene triblock copolymer) by weight of the composition. In some embodiments, a formulation described herein comprises at least about 10.0% and not more than about 30% of a thermosensitive polymer (e.g., polyoxyethylene-polyoxypropylene triblock copolymer) by weight of the composition. In some embodiments, a formulation described herein comprises at least about 10.0% and not more than about 25% of a thermosensitive polymer (e.g., polyoxyethylene-polyoxypropylene triblock copolymer) by weight of the composition. In some embodiments, a formulation described herein comprises at least about 12.0% and not more than about 25% of a thermosensitive polymer (e.g., polyoxyethylene-polyoxypropylene triblock copolymer) by weight of the composition. In some embodiments, a formulation described herein comprises at least about 10% and not more than about 20% of a thermosensitive polymer (e.g., polyoxyethylene-polyoxypropylene triblock copolymer) by weight of the composition. In some embodiments, a formulation described herein comprises at least about 12% and not more than about 20% of a thermosensitive polymer (e.g., polyoxyethylene-polyoxypropylene triblock copolymer) by weight of the composition. In some of such embodiments, the thermosensitive polymer is a purified polymer. In other embodiments, the thermosensitive polymer is un-purified.In any of the aforementioned embodiments, the formulations further comprise a gel temperature modulating agent.

In some embodiments, a formulation described herein comprises at least about 5% and not more than about 20% of a thermosensitive polymer (e.g., polyoxyethylene-polyoxypropylene triblock copolymer) by weight of the composition. In some embodiments, a formulation described herein comprises at least about 10% and not more than about 20% of a thermosensitive polymer (e.g., polyoxyethylene-polyoxypropylene triblock copolymer) by weight of the composition. In some embodiments, a formulation described herein comprises at least about 10% and not more than about 18% of a thermosensitive polymer (e.g., polyoxyethylene-polyoxypropylene triblock copolymer) by weight of the composition. In some embodiments, a formulation described herein comprises at least about 10% and not more than about 16% of a thermosensitive polymer (e.g., polyoxyethylene-polyoxypropylene triblock copolymer) by weight of the composition. In some embodiments, a formulation described herein comprises at least about 10% and not more than about 15% of a thermosensitive polymer (e.g., polyoxyethylene-polyoxypropylene triblock copolymer) by weight of the composition. In some embodiments, a formulation described herein comprises at least about 12% and not more than about 14% of a thermosensitive polymer (e.g., polyoxyethylene-polyoxypropylene triblock copolymer) by weight of the composition. In some embodiments, a formulation described herein comprises at least about 10% and not more than about 13% of a thermosensitive polymer (e.g., polyoxyethylene-polyoxypropylene triblock copolymer) by weight of the composition. In some embodiments, a formulation described herein comprises at least about 5 % and not more than about 15%, 16%, 17%, 18%, 19% or 20% of a thermosensitive polymer (e.g., polyoxyethylene-polyoxypropylene triblock copolymer) by weight of the composition. In some of such embodiments, the thermosensitive polymer is a purified polymer. In other embodiments, the thermosensitive polymer is un-purified. In any of the aforementioned embodiments, the formulations further comprise a gel temperature modulating agent.

In some embodiments, a formulation described herein comprises at least about 5.0%, 10.0%, 10.5%, 11.0%, 11.5%, 12.0%, 12.5%, 13.0%, 13.5%, 14.0%, 14.5%, 15.0%, 15.5%, 16.0%, 16.5%, 17.0%, 17.5%, or 18.0% and not more than about 14.5%, 15.0%, 15.5%, 16.0%, 16.5%, 17.0%, 17.5%, 18.0%, 18.5%, 19.0%, 20.0%, 21.0%, 25.0%, 30%, 40% or 50% of P407 by weight of the composition.

In some embodiments, formulations described above have a gelation temperature between about 5 °C and about 42 °C and comprise between about 5% to about 50% of a thermosensitive polymer by weight of the composition. In some embodiments, formulations described above have a gelation temperature between about 14 °C and about 42 °C and comprise between about 5% to about 40% of a thermosensitive polymer by weight of the composition. In some embodiments, the about 5% to about 40% of a thermosensitive polymer comprises a polyoxyethylene-polyoxypropylene triblock copolymer by weight of the composition. In some embodiments, the thermosensitive polymer (e.g., polyoxyethylene-polyoxypropylene triblock copolymer) is purified. In some embodiments, the thermosensitive polymer (e.g., polyoxyethylene-polyoxypropylene triblock copolymer) is un-purified (e.g., commercially available P407 NF from BASF). In some embodiments, the about 5% to about 40% of a thermosensitive polymer comprises a polyoxyethylene-polyoxypropylene triblock copolymer and the formulation further comprises a gel temperature modulating agent. By way of example, in certain embodiments, a gel temperature modulating agent is selected from, for example, cyclodextrin, PEG, P188, P338, carboxymethyl cellulose, hyaluronic acid, Carbopol®, chitosan, or the like.

In some embodiments of the formulations described above, the formulations comprise purified poloxamer. In some embodiments, a formulation comprising a purified poloxamer contains a lower poloxamer concentration compared to a formulation comprising non-purified poloxamer while retaining the ability to gel at a temperature between about 14 °C and about 42 °C. By way of example, a micronized dexamethasone formulation comprising between about 10% and about 12% of fractionated poloxamer 407 gels at a temperature between about 14 °C and about 42 °C, and a micronized dexamethasone formulation comprising between about 14.5% and about 25% of un-purified poloxamer 407 also gels at a temperature between about 14 °C and about 42 °C. Thus use of purified poloxamer allows for use of a lower amount

of the thermosensitive polymer while retaining the gel temperature and sustained release properties of the formulation.

Accordingly, also contemplated within the scope of embodiments described herein are active compositions comprising primarily a thermosensitive polymer comprising polyoxyethylene and polyoxyethylene copolymers as a major component polymer and a gel temperature modifying agent as a minor component polymer such that the formulation retains the ability to gel at temperatures between about 14 °C and about 42 °C. By way of example, a composition comprising about 30% of non-purified P407 by weight of the composition, and about 3% P188 by weight of the composition gels at about body temperature.

In some embodiments, the formulations described herein are free or substantially free of additional preservatives that cause irritation and/or toxicity. Additional preservatives do not include trace amounts of antioxidants (e.g., Butylated hydroxytoluene (BHT)) that stabilize thermosensitive polymers, and which are typically provided commercially with thermosensitive polymers. Examples of additional preservatives include benzethonium chloride, benzalkonium chloride, and thiomersal. In some embodiments, a formulation disclosed herein comprises less than about 50 ppm of each of benzethonium chloride, benzalkonium chloride, and thiomersal. In some embodiments, a formulation disclosed herein comprises less than about 25 ppm of each of benzethonium chloride, benzalkonium chloride, and thiomersal. In some embodiments, a formulation disclosed herein comprises less than about 20 ppm of each of benzethonium chloride, benzalkonium chloride, and thiomersal. In some embodiments, a formulation disclosed herein comprises less than about 10 ppm of each of benzethonium chloride, benzalkonium chloride, and thiomersal. In some embodiments, a formulation disclosed herein comprises less than about 5 ppm of each of benzethonium chloride, benzalkonium chloride, and thiomersal. In some embodiments, a formulation disclosed herein comprises less than about 1 ppm of each of benzethonium chloride, benzalkonium chloride, and thiomersal.

In some embodiments, the formulations described herein are free or substantially free of additional tonicity agents that cause irritation and/or toxicity. Examples of additional tonicity agents include propylene glycol. Thus, in some embodiments, a formulation described herein is free or substantially free of propylene glycol. In some embodiments, a formulation disclosed herein comprises less than about 50 ppm of propylene glycol. In some embodiments, a formulation disclosed herein comprises less than about 25 ppm of propylene glycol. In some embodiments, a formulation disclosed herein comprises less than about 20 ppm of propylene glycol. In some embodiments, a formulation disclosed herein comprises less than about 10 ppm of propylene glycol. In some embodiments, a formulation disclosed herein comprises less than about 5 ppm of propylene glycol. In some embodiments, a formulation disclosed herein comprises less than about 1 ppm of propylene glycol.

In some embodiments, the formulations described herein are free or substantially free of additional moisture retention agents. Examples of moisture retention agents include glycerin. Thus, in some embodiments, a formulation described herein is free or substantially free of glycerin. In some embodiments, a formulation disclosed herein comprises less than about 50 ppm of glycerin. In some embodiments, a formulation disclosed herein comprises less than about 25 ppm of glycerin. In some embodiments, a formulation disclosed herein comprises less than about 20 ppm of glycerin. In some embodiments, a formulation disclosed herein comprises less than about 10 ppm of glycerin. In some embodiments, a formulation disclosed herein comprises less than about 5 ppm of glycerin. In some embodiments, a formulation disclosed herein comprises less than about 1 ppm of glycerin.

The formulations described herein are substantially free of degradation products of the active agent and/or the polymer components. As used herein, "substantially free of degradation products" means less than 5% by weight of the active agent and/or the polymer components are degradation products of the active agent and/or the polymer components. In further embodiments, the term means less than 3% by weight of the active agent and/or the polymer components are degradation products of the active agent and/or the polymer components. In yet further embodiments, the term means less than 2% by weight of the active agent and/or the polymer components are degradation products of the active agent and/or the polymer components. In further embodiments, the term means less than 1% by weight of the active agent and/or the polymer components are degradation products of the active agent and/or the polymer components.

In some embodiments, the formulations described herein are free or substantially free of additional thickening agents. Examples of additional thickening agents include chitosan, or polyethylene glycol (PEG). In some embodiments, a formulation disclosed herein comprises less than about 5% by weight of chitosan. In some embodiments, a formulation disclosed herein comprises less than about 4% by weight of chitosan. In some embodiments, a formulation disclosed herein comprises less than about 3% by weight of chitosan. In some embodiments, a formulation disclosed herein comprises less than about 2% by weight of chitosan. In some embodiments, a formulation disclosed herein comprises less than about 1% by weight of chitosan. In some embodiments, a formulation disclosed herein comprises less than about 0.5% by weight of chitosan.

In some embodiments, the formulations described herein are free or substantially free of additional mucoadhesives. Examples of additional mucoadhesives include hyaluronic acid. In some embodiments, a formulation described herein comprises less than about 5% by weight of hyaluronic acid. In some embodiments, a formulation disclosed herein comprises less than about 4% by weight of hyaluronic acid. In some embodiments, a formulation disclosed herein comprises less than about 3% by weight of hyaluronic acid. In some embodiments, a formulation disclosed herein

comprises less than about 2% by weight of hyaluronic acid. In some embodiments, a formulation disclosed herein comprises less than about 1% by weight of hyaluronic acid. In some embodiments, a formulation disclosed herein comprises less than about 0.5% by weight of hyaluronic acid.

In some embodiments, the formulations described herein are free or substantially free of additional common solvents that cause irritation and/or toxicity. Examples of additional solvents include ethanol, propylene glycol, DMSO, N-Methyl-2-pyrrolidone, and cyclohexane. Thus, in some embodiments, a formulation described herein is free or substantially free of ethanol, propylene glycol, DMSO, N-Methyl-2-pyrrolidone, and cyclohexane. In some embodiments, a formulation disclosed herein comprises less than about 50 ppm of each of ethanol, propylene glycol, DMSO, N-Methyl-2-pyrrolidone, and cyclohexane. In some embodiments, a formulation disclosed herein comprises less than about 25 ppm of each of ethanol, propylene glycol, DMSO, N-Methyl-2-pyrrolidone, and cyclohexane. In some embodiments, a formulation disclosed herein comprises less than about 20 ppm of each of ethanol, propylene glycol, DMSO, N-Methyl-2-pyrrolidone, and cyclohexane. In some embodiments, a formulation disclosed herein comprises less than about 10 ppm of each of ethanol, propylene glycol, DMSO, N-Methyl-2-pyrrolidone, and cyclohexane. In some embodiments, a formulation disclosed herein comprises less than about 5 ppm of each of ethanol, propylene glycol, DMSO, N-Methyl-2-pyrrolidone, and cyclohexane. In some embodiments, a formulation disclosed herein comprises less than about 1 ppm of each of ethanol, propylene glycol, DMSO, N-Methyl-2-pyrrolidone, and cyclohexane.

In some embodiments, the formulations described herein are free or substantially free of additional antiseptics that are commonly used to disinfect any component of an active preparation and that are potentially toxic. Examples of additional antiseptics that are known to be toxic include acetic acid, iodine and merbromin. Additionally, chlorhexidene, a commonly used antiseptic, that is used to disinfect components of an active preparation (including devices used to administer the preparation) is highly toxic in minute concentrations (e.g., 0.05%). Thus, in some embodiments, a formulation disclosed herein is free or substantially free of acetic acid, iodine, merbromin, and chlorhexidene. In some embodiments, a formulation disclosed herein comprises less than about 50 ppm of each of acetic acid, iodine, merbromin, and chlorhexidene. In some embodiments, a formulation disclosed herein comprises less than about 25 ppm of each of acetic acid, iodine, merbromin, and chlorhexidene. In some embodiments, a formulation disclosed herein comprises less than about 20 ppm of each of acetic acid, iodine, merbromin, and chlorhexidene. In some embodiments, a formulation disclosed herein comprises less than about 10 ppm of each of acetic acid, iodine, merbromin, and chlorhexidene. In some embodiments, a formulation disclosed herein comprises less than about 5 ppm of each of acetic acid, iodine, merbromin, and chlorhexidene. In some embodiments, a formulation disclosed herein comprises less than about 1 ppm of each of acetic acid, iodine, merbromin, and chlorhexidene.

Further, certain preparations (e.g., preparations for inner ear administration, intrathecal administration) require particularly low concentrations of several potentially-common contaminants that are known to be toxic. Other dosage forms, while seeking to limit the contamination attributable to these compounds, do not require the stringent precautions that such preparations require. For example, in some embodiments, the formulations described herein are free or substantially free of contaminants such as arsenic, lead, mercury, and tin. Thus, in some embodiments, a formulation disclosed herein is free or substantially free of arsenic, lead, mercury, and tin. In some embodiments, a formulation disclosed herein comprises less than about 50 ppm of each of arsenic, lead, mercury, and tin. In some embodiments, a formulation disclosed herein comprises less than about 25 ppm of each of arsenic, lead, mercury, and tin. In some embodiments, a formulation disclosed herein comprises less than about 20 ppm of each of arsenic, lead, mercury, and tin. In some embodiments, a formulation disclosed herein comprises less than about 10 ppm of each of arsenic, lead, mercury, and tin. In some embodiments, a formulation disclosed herein comprises less than about 5 ppm of each of arsenic, lead, mercury, and tin. In some embodiments, a formulation disclosed herein comprises less than about 1 ppm of each of arsenic, lead, mercury, and tin.

## Certain Definitions

"Thermosensitive polymers" or " thermosetting polymers"are polymers that undergo a reversible temperature-dependent phase transtion (e.g., a liquid to gel transition, a gel to liquid transition, or the like). Example of thermosensitive polymers that form thermosensitive gels include and are not limited to poloxamers (e.g., Pluronics F68®, F88®, and F108®, F127®, or the like) or any other thermosetting polymer described herein.

As used herein, a "purified" thermosensitive polymer is a commercially purchased thermosensitive polymer that is subjected to further steps prior to preparation of formulations described herein. A purified thermosensitive polymer has lower polydispersity (i.e., a narrower distribution of molecular weights amongst the individual polymer chains therein) and/or lower ethylene content and/or less unsaturation and/or weight% oxyethylene values compared to a commercially available sample of the same polymer. Purification is carried out using any suitable technique including and not limited to fractionation, chromatography, washing and/or decantation, purification using supercritical fluid (*See*, for example, U.S. Patent Appl. Pub. No. 2008/0269449, disclosure of purification of polymers by use of supercritical fluid described therein is incorporated herein by reference), reverse precipitation (*See*, for example, U.S. Patent No. 7,148,320, disclosure of reverse precipitation described therein is incorporated herein by reference), salt extraction and liquid phase separation (*See* for example, U.S. Patent No. 5,800,711, disclosure of poloxamer purification described therein is incorporated herein by reference), or the like. Other processes for purification and/or fractionation of polymers are described in, for example, US 6,977,045 and US 6,761,824 which processes described therein are incorporated herein by reference

By way of example, in some embodiments, purified poloxamer 407 is fractionated P407 having a lower polydispersity index compared to a commercially purchased batch of P407 grade NF from BASF. By way of example, the commercially purchased P407 has a polydispersity index of about 1.2. In some embodiments, the polydispersity index of fractionated P407 as described herein is between about 1 and about 1.15. In other embodiments, the polydispersity index of fractionated P407 as described herein is between about 1 and about 1.1. In yet other embodiments, the polydispersity index of fractionated P407 as described herein is between about 1 and about 1.05. As used herein, the calculated polydispersity index (PDI) is the weight average molecular weight divided by the number average molecular weight of polymeric chains ($M_w/M_n$). It indicates the distribution of individual molecular masses in a batch of polymers.

A "syringable viscosity" is a viscosity that is low enough such that a pharmaceutical formulation described herein is a liquid that is capable of being administered (e.g., syringed) via a narrow gauge needle or cannula or catheter using normal finger pressure (e.g., by a physician using normal finger pressure on the plunger of the syringe, such that the needle of the syringe can accurately and stably deliver the pharmaceutical formulation at the targeted site (e.g., round window membrane of inner ear, sinonasal cavities or the like). Thus in some embodiments, formulations described herein are dispensed through a 18-31 gauge needle or cannula or catheter. In some embodiments, formulations described herein are dispensed through a 20-26 gauge needle or cannula or catheter. In some embodiments, formulations described herein are dispensed through a 25-31 gauge needle or cannula or catheter. In some embodiments, formulations described herein are dispensed through a 27-31 gauge needle or cannula or catheter. In some embodiments, formulations described herein are syringable through a 27 gauge needle or cannula or catheter. In some embodiments, formulations described herein are syringable through a 29 gauge needle or cannula or catheter. In some embodiments, formulations described herein are syringable through a 31 gauge needle or cannula or catheter.

A "gelation temperature modifying agent" or a "gel temperature modifying agent" is an additive added to any formulation described herein, and changes the gelation temperature of the formulation such that the gel temperature of the formulation is maintained, in some embodiments, between about 5 °C and about 42 °C. In some other embodiments, a gel temperature modifying agent changes the gelation temperature of the formulation such that the gel temperature of the formulation is maintained, in some embodiments, between about 14 °C and about 42 °C. In some embodiments, a gel temperature modifying agent increases the gelation temperature of the formulation compared to the gelation temperature in the absence of the gel temperature modifying agent. In some embodiments, a gel temperature modifying agent decreases the gelation temperature of the formulation compared to the gelation temperature in the absence of the gel temperature modifying agent.

The terms "effective amount" or "therapeutically effective amount," as used herein, refer to a sufficient amount of the active agent or active agent (e.g., an active agent, an anti-inflammatory agent) being administered that would be expected to relieve to some extent one or more of the symptoms of the disease or condition being treated. For example, the result of administration of an active agent disclosed herein is reduction and/or alleviation of the signs, symptoms, or causes of tinnitus or balance disorders. For example, an "effective amount" for therapeutic uses is the amount of active agent, including a formulation as disclosed herein required to provide a decrease or amelioration in disease symptoms without undue adverse side effects. The term "therapeutically effective amount" includes, for example, a prophylactically effective amount. An "effective amount" of an active agent disclosed herein is an amount effective to achieve a desired pharmacologic effect or therapeutic improvement without undue adverse side effects. It is understood that "an effective amount" or "a therapeutically effective amount" varies, in some embodiments, from subject to subject, due to variation in metabolism of the compound administered, age, weight, general condition of the subject, the condition being treated, the severity of the condition being treated, and the judgment of the prescribing physician. It is also understood that "an effective amount" in an extended-release dosing format may differ from "an effective amount" in an immediate release dosign format based upon pharmacokinetic and pharmacodynamic considerations.

As used herein, the term "active agent" refers to active agents that treat, or reduce or ameliorate severity of any active disorder described herein. Suitable "active agents" may be antimicrobial agents (e.g., antibacterial agents (effective against bacteria), antiviral agents (effective against viruses), antifungal agents (effective against fungi), antiprotozoal (effective against protozoa), and/or antiparasitic to any class of microbial parasites), corticosteroids, or any other active agent described herein. "Active agents" may work by any suitable mechanism, non-limiting examples of which include by being anti-inflammatory, antimicrobial, toxic, cytostatic, immunomodulatory agents, ion channgel modulators, anti-angiogenic agents and the like.

The mean residence time (MRT) is the average time that molecules of an active agent reside in an active structure after administration of a dose.

A "prodrug" refers to an active agent that is converted into the parent drug *in vivo.* In certain embodiments, a prodrug is enzymatically metabolized by one or more steps or processes to the biologically, pharmaceutically or therapeutically active form of the compound. To produce a prodrug, a pharmaceutically active compound is modified such that the active compound will be regenerated upon *in vivo* administration. In one embodiment, the prodrug is designed to alter the metabolic stability or the transport characteristics of a drug, to mask side effects or toxicity, or to alter other characteristics or properties of a drug. Compounds provided herein, in some embodiments, are derivatized into suitable prodrugs.

Other objects, features, and advantages of the methods and compositions described herein will become apparent from the following detailed description. It should be understood, however, that the detailed description and the specific examples, while indicating specific embodiments, are given by way of illustration only.

## Active Agents

Provided herein are active agent compositions and formulations that are suitable for localized administration and provide sustained release of an active agent at the target site.

## Antimicrobial agents

In some embodiments, the active agent suitable for use in the formulations and methods disclosed herein is an antimicrobial agent including an antibacterial agent, an antifungal agent, an antiviral agent, an antiprotozoal agent, and/or an antiparasitic agent. In some embodiments, the antimicrobial agent is a protein, a peptide, an antibody, DNA, an siRNA, a carbohydrate, an inorganic molecule, or an organic molecule. In certain embodiments, the active agents are antimicrobial small molecules.

## Antibacterial agents

In some embodiments, the active agent is an antibacterial agent. In some embodiments, the antibacterial agent treats infections caused by gram positive bacteria. In some embodiments, the antibacterial agent treats infections caused by gram negative bacteria. In some embodiments, the antibacterial agent treats infections caused by mycobacteria. In some embodiments, the antibacterial agent treats infections caused by giardia.

In some embodiments, the antibacterial agent treats infections by inhibiting bacterial protein synthesis. In some embodiments, the antibacterial agent treats infections by disrupting synthesis of bacterial cell wall. In some embodiments, the antibacterial agent treats infections by changing permeability of bacterial cell membranes. In some embodiments, the antibacterial agent treats infections by disrupting DNA replication in bacteria.

In some embodiments, the antibacterial agent is an antibiotic. In some embodiments, the antibiotic is an aminoglycoside. Examples of aminoglycoside antibiotics include and are not limited to amikacin, gentamicin, kanamycin, neomycin, netilmicin, streptomycin, tobramycin, paromycin or the like. In some embodiments, the antibiotic is an ansamycin. Examples of ansamycins include and are not limited to geldanamycin, herbimycin or the like. In some embodiments, the antibiotic is a carbacephem. Examples of carbecephems include and are not limited to loracarbef or the like. In some embodiments, the antibiotic is a carbapenem. Examples of carbapenems include and are not limited to ertapenem, doripenem, imipenem (cilostatin), meropenem or the like. In some embodiments, the antibiotic is a cephalosporin (including, for example, first, second, third, fourth or fifth generation cephalosporins). Examples of cephalosporins include and are not limited to cefaclor, cefamandole, cefotoxin, cefprozil, cefuroxime, cefixime, cefdinir, cefditoren, cefpodoxime, ceftazidime, ceftibuten, ceftizoxime, ceftriaxone, cefepime, ceftobirprole or the like. In some embodiments, the antibiotic is a glycopeptide. Examples of glycopeptides include and are not limited to vancomycin or the like. In some embodiments, the antibiotic is a macrolide antibiotic. Examples of macrolides include and are not limited to azithromycin, clarithromycin, dirithromycin, erythromycin, roxithromycin, troleandomycin, telithromycin, spectinomycin, or the like. In some embodiments, the antibiotic is a monobactam. Examples of monobactams include and are not limited to aztreonam or the like. In some embodiments, the antibiotic is a beta-lactamase inhibitor and/or penicillin. Examples of beta-lactamase inhibitors include clavulanic acid and/or pencillins and/or beta-lactams. Examples of penicillins include and are not limited to amoxicillin, ampicillin, azociling, carbenicillin, cloxacillin, dicloxacillin, flucloxacillin, mezlocillin, meticillin, nafcillin, oxacillin, peperacillin, ticarcillin, amoxcillin+ clavulanic acid (Augmentin®), or the like. In some embodiments, the antibiotic is a quinolone. Examples of quinolones include and are not limited to ciprofloxacin, enoxacin, gatifloxacin, levofloxacin, lomefloxacin, moxifloxacin, nonfloxacin, ofloxacin, trovafloxacin, grepafloxacin, sparfloxacin, AL-15469A, AL-38905 or the like. In some embodiments, the antibiotic is a sulfonamide. Examples of suflonamides include and are not limited to afenide, prontosil, sulfacetamide, sulfamethiazole, sulfanilimide, sulfasalazine, sulfisoxazole, trimethoprim, cotrimoxazole or the like. In some embodiments, the antibiotic is a tetracycline antibiotic. Examples of tetracyclines include and are not limited to demeclocycline, doxycycline, minocycline, oxytetracycline, tetraycline or the like. In some embodiments, the antibiotic is an oxazolidinone antibiotic. Examples of oxazolidinone antibiotics include and are not limited to linezolid or the like. In some embodiments, the antibiotic is arsogebanubem chloramphenicol, clindamycin, lincomycin, ethambutol,

fosfomycin, fusidic acid, furazolidone, isoniazid, linezolid, metronidazole, mupirocin, nitrofurantoin, platensimycin, pyrazinamide, quinupristin, dalfopristin, rifampicin, thamphenicol, tinidazole or the like.

In some embodiments, an antibiotic compatible with the compositions described herein is a broad spectrum antibiotic. In some embodiments, an antibiotic compatible with the compositions described herein is effective in treating infections that are resistant to other classes of antibiotics. For example, in some instances, vancomycin is effective in treating infections caused by methicillin resistant staphyloccocus aureus bacteria.

In some embodiments, an active antibacterial agent is a peptide or a lantibiotic including, by way of non-limiting example, Maximin H5, Dermcidin, Cecropins, andropin, moricin, ceratotoxin, melittin, Magainin, dermaseptin, bombinin, brevinin-1,esculentins and buforin II, CAP18, LL37 , abaecin, apidaecins, prophenin, indolicidin, brevinins, protegrin, tachyplesins, defensins, drosomycin, alamethicin, pexiganan or MSI-78, and other MSI peptides like MSI-843 and MSI-594, polyphemusin, Class I II and III bacterocins like: colicin, pyocin, klebicin, subtilin, epidermin, herbicolacin, brevicin, halocin , agrocin, alveicin, carnocin, curvaticin, divercin ,enterocin, enterolysin, erwiniocin, glycinecin, lactococin, lacticin, leucoccin, mesentericin, pediocin, plantaricin, sakacin, sulfolobicin, vibriocin, warnerinand, nisin or the like. In some embodiments, the antibiotic is a polypeptide or peptide. Examples of polypeptide antibiotics include and are not limited to bacitracin, colistin, polymyxin B or the like. Examples of peptide antibacterial agents include and are not limited to OP-145 (Octoplus).

In specific embodiments, an antibiotic used in formulations described herein is ciprofloxacin (Cipro®). In specific embodiments, an antibiotic used in formulations described herein is amoxicillin. In specific embodiments, an antibiotic used in formulations described herein is, amoxicillin + clavulanic acid (Augmentin®). In specific embodiments, an antibiotic used in formulations described herein is moxifloxacin.

Localized administration of antibiotic compositions reduces the risk of development of resistance to antibiotics compared to the risk for development of antibiotic resistance when an antibiotic is administered systemically. The compositions described herein are effective for recurring active diseases or conditions including, for example, recurring ear infections in children without the need for changing treatment regimens (e.g., in response to development of antibiotic resistance).

**Antiviral Agents**

In some embodiments, the active agent is an antiviral agent. In some embodiments, the antiviral agents include but are not limited to acyclovir, famciclovir and valacyclovir. Other antiviral agents include abacavir, aciclovir, adfovir, amantadine, amprenavir, arbidol, atazanavir, artipla, brivudine, cidofovir, combivir, edoxudine, efavirenz, emtricitabine, enfuvirtide, entecavir, fomvirsen, fosamprenavir, foscarnet, fosfonet, ganciclovir, gardasil, ibacitabine, imunovir, idoxuridine, imiquimod, indinavir, inosine, integrase inhibitors, interferons, including interferon type III, interferon type II, interferon type I, lamivudine, lopinavir, loviride, MK-0518, maraviroc, moroxydine, nelfinavir, nevirapine, nexavir, nucleoside analogues, oseltamivir, penciclovir, peramivir, pleconaril, podophyllotoxin, protease inhibitors, reverse transcriptase inhibitors, ribavirin, rimantadine, ritonavir, saquinavir, stavudine, tenofovir, tenofovir disoproxil, tipranavir, trifluridine, trizivir, tromantadine, truvada, valganciclovir, vicriviroc, vidarabine, viramidine, zalcitabine, zanamivir, zidovudine, and combinations thereof.

**Antifungal Agents**

In some embodiments, the active agent is an antifungal agent. In some embodiments, the antifungal agents include but are not limited to amrolfine, utenafine, naftifine, terbinafine, flucytosine, fluconazole, itraconazole, ketoconazole, posaconazole, ravuconazole, voriconazole, clotrimazole, econazole, miconazole, oxiconazole, sulconazole, terconazole, tioconazole, nikkomycin Z, caspofungin, micafungin, anidulafungin, amphotericin B, liposomal nystastin, pimaricin, griseofulvin, ciclopirox olamine, haloprogin, tolnaftate, undecylenate, clioquinol, and combinations thereof.

Antiparasitic agents include amitraz, amoscanate, avermectin, carbadox, diethylcarbamizine, dimetridazole, diminazene, ivermectin, macrofilaricide, malathion, mitaban, oxamniquine, permethrin, praziquantel, prantel pamoate, selamectin, sodium stibogluconate, thiabendazole, and combinations thereof.

Antimicrobial agents also include antibacterial, antiviral, antifungal, antiprotozoal and/or anti-parasitic agents described in U.S. Appl. Nos. 12/427,663, 12/466,310, 12/472,034, 12/486,697, 12/493,611, 12/494,156, 12/500,486, 12/504,553, 12/506,091, 12/506,127, 12/506,573, 12/506,616, and 12/506,664, the disclosure of antimicrobial agents described therein is incorporated herein by reference. Antimicrobial agents that are not disclosed herein but which are useful in sustained release formulations described herein are expressly included and intended within the scope of the embodiments presented.

**Anti-inflammatory Agents**

Corticosteroids (including agents that act at glucocorticoid receptors) or other anti-inflammatory steroids are compatible with the formulations disclosed herein. One advantage of the use of a formulation described herein is the greatly reduced systemic exposure to anti-inflammatory glucocorticoid steroids.

In one embodiment is the active pharmaceutical ingredient of the formulation described herein is prednisolone. In another embodiment the active pharmaceutical ingredient of the formulation described herein is dexamethasone. In an additional embodiment, the active pharmaceutical ingredient of the formulation described herein is beclomethasone. In an additional embodiment, the active pharmaceutical ingredient of the formulation described herein is triamcinolone. In a further embodiment, the active pharmaceutical ingredient of the formulation described herein is selected from 21-acetoxypregnenolone, alclometasone, algestone, amcinonide, beclomethasone, betamethasone, budesonide, chloroprednisone, clobetasol, clobetasone, clocortolone, cloprednol, corticosterone, cortisone, cortivazol, deflazacort, desonide, desoximetasone, dexamethasone, diflorasone, diflucortolone, difluprednate, enoxolone, fluazacort, flucloronide, flumethasone, flunisolide, fluocinolone acetonide, fluocinonide, fluocortin butyl, fluocortolone, fluorometholone, fluperolone acetate, fluprednidene acetate, fluprednisolone, flurandrenolide, fluticasone propionate, formocortal, halcinonide, halobetasol propionate, halometasone, halopredone acetate, hydrocortamate, hydrocortisone, loteprednol etabonate, mazipredone, medrysone, meprednisone, methylprednisolone, mometasone furoate, paramethasone, prednicarbate, prednisolone, prednisolone 25-diethylamino-acetate, prednisolone sodium phosphate, prednisone, prednival, prednylidene, rimexolone, tixocortol, triamcinolone, triamcinolone acetonide, triamcinolone benetonide, triamcinolone hexacetonide, or combinations thereof.

Anti-inflammatory agents that are not disclosed herein but which are useful in sustained release formulations described herein are expressly included and intended within the scope of the embodiments presented.

**Bisphosphonates**

In some embodiments, bisphosphonates are used in the formulations disclosed herein, including for the treatment of otosclerosis. Bisphosphonates are contemplated as modulators of bone remodeling in the active capsule (e.g., in the treatment of otosclerosis). Examples of bisphosphonates include and are not limited to etidronate (DIDRONEL®); clodronate (BONEFOS®); tiludronate (SKELID®); pamidronate (APD, AREDIA®); neridronate; olpadronate; alendronate (FOSFAMAX®); ibandronate (BONIVA®); risedronate (ACTONEL®); zoledronate (ZOMETA®), or the like. In certain embodiments, a bisphosphonate is zoledronatae or risedronatae.

Relative potency of bisphosphonates is shown in Table A below:

Table A

| Bisphosphonate | Relative Potency to Etidronate |
|---|---|
| Pamidronate (APD, Aredia) | 100 |
| Neridronate | 100 |
| Olpadronate | 500 |
| Alendronate (Fosamax) | 500 |
| Ibandronate (Boniva) | 1000 |
| Risedronate (Actonel) | 2000 |
| Zoledronate (Zometa, Aclasta) | 10000 |

Bisphosphonates and/or other bone-remodeling agents that are not disclosed herein but which are useful in sustained release formulations described herein are expressly included and intended within the scope of the embodiments presented.

**Immunomodulating agents**

*TNF-$\alpha$ modulators*

Contemplated for use with the formulations disclosed herein are active agents which reduce or ameliorate symptoms or effects as a result of an autoimmune disease and/or inflammatory disorder. Accordingly, some embodiments of the methods and compositions described herein incorporate the use of agents which block the effects of TNF-$\alpha$,

including anti-TNF agents for treatment of sinonasal and/or otic conditions associated with autoimmune disease and/or inflammation. By way of example only, anti-TNF agents include protein-based therapeutics, such as etanercept (ENBREL®), infliximab (REMICADE®), adalimumab (HUMIRA®) and golimumab (CNTO 148), and small molecule therapeutics, such as TACE inhibitors, IKK inhibitors or calcineurin inhibitors or combinations thereof. Calcineurin inhibitors are a group of structurally diverse small molecule immunomodulators which function through the inhibition of calcineurin function. Examples of calcineurin modulators include tacrolimus, pimecrolimus, cyclosporine or the like. IKK inhibitors are yet another structurally diverse group of small molecule immunomodulators, examples of which include and are not limited to PC-839, PS-1145, BMS-345541, SC-514 or the like.

Other immunomodulator agents suitable for use in the methods and compositions described herein include and are not limited to:

*TACE inhibitors:*

Examples of TACE inhibitors include and are not limited to Nitroarginine analog A, GW3333, TMI-1, BMS-561392, DPC-3333, TMI-2, BMS-566394, TMI-005, apratastat, GW4459, W-3646, IK-682, GI-5402, GI-245402, BB-2983, DPC-A38088, DPH-067517, R-618, CH-138 or the like.

*Interleukin inhibitors*

Examples of Interleukin inhibitors include and are not limited to WS-4 (an antibody against IL-8), SB 265610 (N-(2-Bromophenyl)-N'-(7-cyano-1H-benzotriazol-4-yl)urea); SB 225002 (N-(2-Bromophenyl)-N'-(2-hydroxy-4-nitrophenyl)urea); SB203580 (4-(4-Fluorophenyl)-2-(4-methylsulfinyl phenyl)-5-(4-pyridyl) 1H-imidazole); SB272844 (GlaxoSmithKline); SB517785 (GlaxoSmithKline); SB656933 (GlaxoSmithKline); Sch527123 (2-hydroxy-N,N-dimethyl-3-{2-[[(R)-1-(5-methyl-furan-2-yl)-propyl]amino]-3,4-dioxo-cyclobut-1-enylamino}-benzamide); PD98059(2-(2-amino-3-methoxyphenyl)-4H-1-Benzopyran-4-one); reparixin; N-[4-chloro-2-hydroxy-3-(piperazine-1-sulfonyl)phenyl]-N'-(2-chloro-3-fluorophenyl)urea p-toluenesulfonate, basiliximab; cyclosporin A; SDZ RAD (40-O-(2-hydroxyethyl)-rapamycin); FR235222 (Astellas Pharma); daclizumab; anakinra; AF12198 (Ac-Phe-Glu-Trp-Thr-Pro-Gly-Trp-Tyr-Gln-L-azetidine-2-carbonyl-Tyr-Ala-Leu-Pro-Leu-NH2) or the like.

*Platelet Activating Factor antagonists*

Examples of platelet activating factor antagonists include and are not limited to kadsurenone, phomactin G, ginsenosides, apafant (4-(2- chlorophenyl)-9-methyl-2[3(4-morpholinyl)-3-propanol-1-yl[6H-thieno[3.2-f[[1.2.4]triazolo]4,3-1]]1.4]diazepine), A-85783, BN-52063, BN-52021, BN-50730 (tetrahedra-4,7,8,10 methyl-1 (chloro-1 phenyl)-6 (methoxy-4 phenyl-carbamoyl)-9 pyrido [4',3'-4,5] thieno [3,2-f] triazolo-1,2,4 [4,3-a] diazepine-1,4), BN 50739, SM-12502, RP-55778, Ro 24-4736, SR27417A, CV-6209, WEB 2086, WEB 2170, 14-deoxyandrographolide, CL 184005, CV-3988, TCV-309, PMS-601, TCV-309 or the like.

*Toll like receptor inhibitors*

Examples of toll like receptor inhibitors include and are not limited to E5531 ((6-O-{2-deoxy-6-O-methyl-4-O-phosphono-3-O-[(R)-3-Z-dodec-5-endoyloxydecl]-2-[3-oxo-tetradecanoylamino]-β-O-phosphono-α-D-glucopyranose tetrasodium salt); E5564 (α-D-Glucopyranose,3-O-decyl-2-deoxy-6-O-[2-deoxy-3-O-[(3R)-3-methoxydecyl]-6-O-methyl-2-[[(11Z)-1-oxo-11-octadecenyl]amino]-4-O-phosphono-β-D-glucopyranosyl]-2-[(1,3-dioxotetradecyl)amino]-1-(dihydrogen phosphate), tetrasodium salt); compound 4a (hydrocinnamoyl-L-valyl pyrrolidine; *see* PNAS, June 24, 2003, vol. 100, no. 13, 7971-7976 which is herein incorporated by reference for disclosures related to compound 4a); CPG 52364 (Coley Pharmaceutical Group); LY294002 (2-(4-Morpholinyl)-8-phenyl-4H-1-benzopyran-4-one); PD98059 (2-(2-amino-3-methoxyphenyl)-4H-1-Benzopyran-4-one); chloroquine or the like.

*Progesterone Receptor Modulators*

Examples progesterone receptor modulators include and are not limited to RU-486 ((11b,17b)-11-[4-(Dimethylamino)phenyl]-17-hydroxy-17-(1-propynyl)-estra-4,9-dien-3-one); CDB-2914 (17α-acetoxy-11β-[4-N,N-dimethylaminophenyl]-19-norpregna-4,9-diene-3,20-dione); CDB-4124 (17α-acetoxy-21-methoxy-11β-[4-N,N-dimethylaminophenyl]-19-norpregna-4,9- diene-3,20-dione); CDB-4453 (17α-acetoxy-21-methoxy-11β-[4-N-methylaminophenyl]-19-norpregna-4,9-diene-3,20-dione); RTI 3021-022 (Research Triangle Institute); ZK 230211 (11-(4-acetylphenyl)-17-hydroxy-17-(1,1,2,2,2-pentafluoroethyl)estra-4,9-dien-3-one); ORG 31710 (11-(4-dimethylaminophenyl)-6-methyl-4',5'-dihydro(estra-4,9-diene-17,2'-(3H)-furan)-3-one); ORG 33628 (Organon); onapristone (ZK 98299); asoprisnil; ulipristal; a

anti-progesterone antibody; an anti-progesterone receptor antibody or the like.

*Prostaglandins*

Examples of prostaglandins and/or analogs thereof include and are not limited to naturally occurring prostaglandins, Prostaglandin analogues, such as latanoprost, travoprost, unoprostone, minprostin F2 alpha and bimtoprost, SQ29548, JB004/A or the like.

*Adenosine Receptor Modulators*

Examples of adenosine receptor modulators include and are not limited to ATL313 (4-(3-(6-amino-9-(5-cyclopropylcarbamoyl-3,4-dihydroxytetrahydrofuran-2-yl)-9H-purin-2-yl)prop-2-ynyl)piperidine-1-carboxylic acid methyl ester); GW328267X ((2R,3R,4S,5R)-2-{6-amino-2-[(1-benzyl-2-hydroxyethyl)amino]-9H-purin-9-yl}-5-(2-ethyl-2H-tetrazol-5-yl)tetrahydrofuran-3,4-diol); CGS 21680 hydrochloride (4-[2-[[6-Amino-9-(N-ethyl-b-D-ribofuranuronamidosyl)-9H-purin-2-yl]amino]ethyl]benzenepropanoic acid hydrochloride); CV 1808 (2-Phenylaminoadenosine); p-DITC-APEC (2-[4-[2-[2-[(4-Isothiocyanatophenyl)thiocarbonylamino]e thylaminocarbonyl]ethyl]phenethylamino]-5'-N-ethylcarbo xamidadenosine); SDZ WAG994 (N-Cyclohexyl-2'-O-methyladenosine); CVT-3146 (regadenoson; 1-(9-(3,4-dihydroxy-5-(hydroxymethyl)oxolan-2-yl)-6-aminopurin-2-yl)pyrazol-4-yl)-N-methylcarboxamide); ATL-146e (4-{3-[6-Amino-9-(5-ethylcarbamoyl-3,4-dihydroxy-tetrahydro-furan-2-yl)-9H-purin-2-yl]-prop-2-ynyl}-cyclohexanecarboxylic acid methyl ester); 5'-n-Ethyl-carboxamidoadenosine; tecadenoson; CVT-510 (N-(3(R)-tetrahydrofuranyl)-6-aminopurine riboside); CCPA (2-Chloro-N6-cyclopentyladenosine); CPA (N6-Cyclopentyladenosine); GR 79236 (N-[(1S,2S)-2-Hydroxycyclopentyl]adenosine); 2'-MeCCPA; PD 81723 ((2-Amino-4,5-dimethyl-3-thienyl)-[3-(trifluoromethyl)ph enyl]methanone); PSB 36 (1-Butyl-8-(hexahydro-2,5-methanopentalen-3a(1H)-yl)-3,7-dihydro-3-(3-hydroxypropyl)-1H-purine-2,6-dione); ribavirin; CHA (N6-cyclohexyladenosine); GW493838 (GSK); (-)-N6-(2-phenylisopropyl) adenosine; GW684067 ((2R,3R,4S,5R)-5-ethynyl-2-[6-tetrahydro-2H-pyran-4-ylamino)-9H-purin-9-yl]tetrahydrofuran-3,4-diol); CVT-3619 (2-(6-((2-hydroxycyclopentyl)amino)purin-9-yl)-5-((2-fluorophenylthio)methyl)oxolane-3,4-diol); 2-Cl-IB-MECA (CF102; 2-chloro-$N^6$-(3-iodobenzyl)-5'-N-methylcarbamoyladenosine); HEMADO; IB-MECA (CF101; $N^6$-(3-iodobenzyl)-5'-*N*-methylcarbamoyladenosine); CP-532903 ($N^6$-(2,5-Dichlorobenzyl)-3'-aminoadenosine-5'-N-methylcarboxamide); CF502 (Can-Fite BioPharma); LJ-529 (2-chloro-N(6)-(3-iodobenzyl)-5'-N-methylcarbamoyl-4'- thioadenosine); BAA (8-butylaminoadenosine); 6-Amino-2-chloropurine riboside; 2-Chloroadenosine; NECA (5'-N-ethylcarboxamidoadenosine); APNEA (N6-2-(4-aminophenyl)ethyladenosine); or the like.

Other immunomodulating agents are described in, for example, U.S. Appl. Nos. 12/472,034 and 12/427,663, which agents are incorporated herein by reference and are contemplated as being within the scope of embodiments presented herein.

**Cytotoxic agents and/or Chemotherapeutic Agents**

Contemplated for use with the formulations disclosed herein are active agents which reduce or ameliorate symptoms or effects as a result of a cell proliferation disorder. Accordingly, some embodiments of the methods and compositions described herein incorporate the use of cytotoxic agents for treatment of sinonasal and/or otic conditions including and not limited to cancers.

Examples of cytotoxic agents include and are not limited to methotrexate (RHEUMATREX®, Amethopterin) cyclophosphamide (CYTOXAN®), thalidomide (THALIDOMID®), acridine carboxamide, actimid®, actinomycin, 17-N-allylamino-17-demethoxygeldanamycin, aminopterin, amsacrine, anthracycline, antineoplastic, antineoplaston, 5-azacytidine, azathioprine, BL22, bendamustine, biricodar, bleomycin, bortezomib, bryostatin, busulfan, calyculin, camptothecin, capecitabine, carboplatin, chlorambucil, cisplatin, cladribine, clofarabine, cytarabine, dacarbazine, dasatinib, daunorubicin, decitabine, dichloroacetic acid, discodermolide, docetaxel, doxorubicin, epirubicin, epothilone, eribulin, estramustine, etoposide, exatecan, exisulind, ferruginol, floxuridine, fludarabine, fluorouracil, fosfestrol, fotemustine, gemcitabine, hydroxyurea, IT-101, idarubicin, ifosfamide, imiquimod, irinotecan, irofulven, ixabepilone, laniquidar, lapatinib, lenalidomide, lomustine, lurtotecan, mafosfamide, masoprocol, mechlorethamine, melphalan, mercaptopurine, mitomycin, mitotane, mitoxantrone, nelarabine, nilotinib, oblimersen, oxaliplatin, PAC-1, paclitaxel, pemetrexed, pentostatin, pipobroman, pixantrone, plicamycin, procarbazine, proteasome inhibitors (e.g., bortezomib), raltitrexed, rebeccamycin, revlimid®, rubitecan, SN-38, salinosporamide A, satraplatin, streptozotocin, swainsonine, tariquidar, taxane, tegafur-uracil, temozolomide, testolactone, thioTEPA, tioguanine, topotecan, trabectedin, tretinoin, triplatin tetranitrate, tris(2-chloroethyl)amine, troxacitabine, uracil mustard, valrubicin, vinblastine, vincristine, vinorelbine, vorinostat, zosuquidar. or the like.

Other cytotoxic agents are described in, for example, U.S. Appl. No. 12/493,611, which agents are incorporated herein by reference.

**Estrogen Receptors Modulators**

Contemplated for use with the formulations disclosed herein are active agents which modulate estrogen receptors. Accordingly, some embodiments of the methods and compositions described herein incorporate the use of estrogen receptor modulators for treatment of sinonasal and/or otic conditions including and not limited to polyps and/or cancers in the sinonasal and/or otic structures. Examples of estrogen receptor modulators include and are not limited to, PPT (4,4',4"-(4-Propyl-[1H]-pyrazole-1,3,5-triyl)trisphenol); SKF-82958 (6-chloro-7,8-dihydroxy-3-allyl-1-phenyl-2,3,4,5-tetrahydro-1H-3-benzazepine); estrogen; estradiol; estradiol derivatives, including but not limited to 17-β estradiol, estrone, estriol, synthetic estrogen compositions or combinations thereof. In some embodiments, the ERβ agonist is ERβ-131, phytoestrogen, MK 101 (bioNovo); VG-1010 (bioNovo); DPN (diarylpropiolitrile); ERB-041; WAY-202196; WAY-214156; genistein; estrogen; estradiol or the like.

**Growth factors**

Contemplated for use with the formulations disclosed herein are agents which modulate epithelial cell growth. Accordingly, some embodiments of the methods and compositions described herein incorporate the use of growth factors and/or modulators of growth factors for treatment of sinonasal and/or otic conditions associated with aberrant growth in otic, sinonasal and/or nasopharyngeal regions. Examples of growth factors contemplated for incorporation in compositions described herein include, for example, fibroblast growth factor (FGF), insulin-like growth factor (IGF), epidermal growth factor (EGF), a platlet-derived growth factor (PGF), agonists of epidermal growth factor (EGF) receptor, hepatocyte growth factor (HGF), Transforming growth factor alpha (TGF-α), Transforming growth factor beta (TGF-β), modulators of Vascular endothelial growth factor (VEGF), neutorophic factors or the like.

**Apoptosis modulators**

Contemplated for use with the formulations disclosed herein are agents which reduce or ameliorate symptoms or effects as a result of apoptosis. Accordingly, some embodiments of the methods and compositions described herein incorporate the use of apoptosis modulators for treatment of sinonasal and/or otic conditions associated with aberrant apoptosis. Inhibitors of apoptosis include inhibitors of the MAPK/JNK signaling cascade AKT inhibitors, IKK inhibitors, JAK inhibitors, PI3 kinase inhibitors, NF-κB inhibitors, p38 inhibitors, ERK inhibitors, Src inhibitors or the like that are involved in apoptotic pathways. Other modulators of apoptotic pathways included modulators of caspases or sirtuin.

*JNK modulators*

In some embodiments, the anti-apoptotic agent is an agent which inhibits (partially or fully) the activity of the MAPK/JNK signaling cascade. In some embodiments, the anti-apoptotic agent is minocycline; SB-203580 (4-(4-Fluorophenyl)-2-(4-methylsulfinyl phenyl)-5-(4-pyridyl)1H-imidazole); PD 169316 (4-(4-Fluorophenyl)-2-(4-nitrophenyl)-5-(4-pyridyl)-1H-imidazole); SB 202190 (4-(4-Fluorophenyl)-2-(4-hydroxyphenyl)-5-(4-pyridyl)1H-imidazole); RWJ 67657 (4-[4-(4-fluorophenyl)-1-(3-phenylpropyl)-5-(4-pyridinyl)-1H-imidazol-2-yl]-3-butyn-1-ol); SB 220025 (5-(2-Amino-4-pyrimidinyl)-4-(4-fluorophenyl)-1-(4-piperidinlyl)imidazole); or combinations thereof. In some embodiments, the agent which antagonizes the MAPK/JNK signaling cascade is D-JNKI-1 ((D)-hJIP$_{175-157}$-DPro-DPro-(D)-HIV-TAT$_{57-48}$), AM-111 (Auris), SP600125 (anthra[1,9-cd]pyrazol-6(2H)-one), JNK Inhibitor I ((L)-HIV-TAT$_{48-57}$-PP-JBD$_{20}$), JNK Inhibitor III ((L)-HIV-TAT$_{47-57}$-gaba-c-Junδ$_{33-57}$), AS601245 (1,3-benzothiazol-2-yl(2-[[2-(3-pyridinyl)ethyl] amino]-4 pyrimidinyl) acetonitrile), JNK Inhibitor VI ($H_2N$-RPKRPTTLNLF-$NH_2$), JNK Inhibitor VIII (N-(4-Amino-5-cyano-6-ethoxypyridin-2-yl)-2-(2,5-dimethoxyphenyl)acetamide), JNK Inhibitor IX (N-(3-Cyano-4,5,6,7-tetrahydro-1-benzothien-2-yl)-1-naphthamide), dicumarol (3,3'-Methylenebis(4-hydroxycoumarin)), SC-236 (4-[5-(4-chlorophenyl)-3-(trifluoromethyl)-1*H*-pyrazol-1-yl]benzene-sulfonamide), CEP-1347 (Cephalon), CEP-11004 (Cephalon); or combinations thereof. In some embodiments, the anti-apoptotic agent is AM-111 (Auris).

*JAK (Janus Kinase) modulators*

Contemplated for use with the formulations disclosed herein are active agents that fully or partially inhibit JAK kinases. In some embodiments, the anti-apoptotic agent is VX-680, TG101348, TG101209, INCB018424, XL019, CEP-701, AT9283, or combinations thereof.

*Akt modulators*

In some embodiments, the anti-apoptotic agent is an agent that inhibits (partially or fully) the activity of Akt1.

In some embodiments, the anti-apoptotic agent is a growth factor. In some embodiments, the growth factor is EGF.

*PI3 Kinases modulators*

In some embodiments, the anti-apoptotic agent is an agent that inhibits (partially or fully) the activity of PI3 kinases. In some embodiments, the anti-apoptotic agent is 740 Y-P; SC 3036 (KKHTDDGYMPMSPGVA); PI 3-kinase Activator (Santa Cruz Biotechnology, Inc.), wortmannin, wortmannin analogs (e.g., PX-866); or combinations thereof.

*NF-kB modulators*

Some embodiments incorporate the use of active agents that modulate an NF-kB transcription factor. In certain instances, the agent that modulates an NF-kB transcription factor is an antagonist, partial agonist, inverse agonist, neutral or competitive antagonist, allosteric antagonist, and/or orthosteric antagonist of NF-kB. In some embodiments, the NF-kB transcription factor agonist, partial agonist, and/or positive allosteric modulator is $Pam_3Cys$ ((S)-(2,3-bis(palmitoyloxy)-(2RS)-propyl)-N-palmitoyl-(R)-Cys-(S)-Ser(S)-Lys4-OH, trihydrochloride); Act1 (NF-kB activator 1); Acetyl-11-keto-b-Boswellic Acid; Andrographolide; Caffeic Acid Phenethyl Ester (CAPE); Gliotoxin; Isohelenin; NEMO-Binding Domain Binding Peptide (DRQIKIWFQNRRMKWKKTALDWSWLQTE); NF-kB Activation Inhibitor (6-Amino-4-(4-phenoxyphenylethylamino)quinazoline); NF-kB Activation Inhibitor II (4-Methyl-N1-(3-phenylpropyl)benzene-1,2-diamine); NF-kB Activation Inhibitor III (3-Chloro-4-nitro-N-(5-nitro-2-thiazolyl)-benzamide); NF-kB Activation Inhibitor IV ((E)-2-Fluoro-4'-methoxystilbene); NF-kB Activation Inhibitor V (5-Hydroxy-(2,6-diisopropylphenyl)-1H-isoindole-1,3-dione); NF-kB SN50 (AAVALLPAVLLALLAPVQRKRQKLMP); Oridonin; Parthenolide; PPM-18 (2-Benzoylamino-1,4-naphthoquinone); Ro106-9920; Sulfasalazine; TIRAP Inhibitor Peptide (RQIKIWFNRRMKWKKLQLRDAAPGGAIVS); Withaferin A; Wogonin; BAY 11-7082 ((E)3-[(4-Methylphenyl)sulfonyl]-2-propenenitrile); BAY 11-7085 ((E)3-[(4-t-Butylphenyl)sulfonyl]-2-propenenitrile); (E)-Capsaicin; or combinations thereof.

*p38 modulators*

Some embodiments incorporate the use of active agents that modulate p38. In some embodiments, the agent that modulates p38 is a p38 antagonist, partial agonist, inverse agonists, neutral or competitive antagonists, allosteric antagonists, and/or orthosteric antagonists. In some embodiments, the p38 antagonist, partial agonist, inverse agonists, neutral or competitive antagonist, allosteric antagonist, and/or orthosteric antagonist is ARRY-797 (Array BioPharma); SB-220025 (5-(2-Amino-4-pyrimidinyl)-4-(4-fluorophenyl)-1-(4-piperidinlyl)imidazole); SB-239063 (trans-4-[4-(4-Fluorophenyl)-5-(2-methoxy-4-pyrimidinyl)-1H-imidazol-1-yl]cyclohexanol); SB-202190 (4-(4-Fluorophenyl)-2-(4-hydroxyphenyl)-5-(4-pyridyl)1H-imidazole); JX-401 (-[2-Methoxy-4-(methylthio)benzoyl]-4-(phenylmethyl)piperidine); PD-169316 (4-(4-Fluorophenyl)-2-(4-nitrophenyl)-5-(4-pyridyl)-1H-imidazole); SKF-86002 (6-(4-Fluorophenyl)-2,3-dihydro-5-(4-pyridinyl)imidazo[2,1-b]thiazole dihydrochloride); SB-200646 (N-(1-Methyl-1H-indol-5-yl)-N'-3-pyridinylurea); CMPD-1 (2'-Fluoro-N-(4-hydroxyphenyl)-[1,1'-biphenyl]-4-butanamide); EO-1428 ((2-Methylphenyl)-[4-[(2-amino-4-bromophenyl)amino]-2-ch lorophenyl]methanone);SB-253080 (4-[5-(4-Fluorophenyl)-2-[4-(methylsulfonyl)phenyl]-1H-imidazol-4-yl]pyridine); SD-169 (1H-Indole-5-carboxamide); SB-203580 (4-(4-Fluorophenyl)-2-(4-methylsulfinylphenyl)-5-(4-pyridyl)1H-imidazole); or combinations thereof.

*Src modulators*

Contemplated for use in the methods and compositions described herein are Src modulators. In some embodiments, the Src antagonist, partial agonist, inverse agonist, neutral or competitive antagonist, allosteric antagonist, and/or orthosteric antagonist is 1-Naphthyl PP1 (1-(1,1-Dimethylethyl)-3-(1-naphthalenyl)-1H-pyrazolo[3,4-d]pyrimidin-4-amine); Lavendustin A (5-[[(2,5-Dihydroxyphenyl)methyl][(2-hydroxyphenyl)methy 1]amino]-2-hydroxybenzoic acid); MNS (3,4-Methylenedioxy-b-nitrostyrene); PP1 (1-(1,1-Dimethylethyl)-1-(4-methylphenyl)-1H-pyrazolo[3, 4-d]pyrimidin-4-amine); PP2 (3-(4-chlorophenyl) 1-(1,1-dimethylethyl)-1H-pyrazolo [3,4-d]pyrimidin-4-amine); KX1-004 (Kinex); KX1-005 (Kinex); KX1-136 (Kinex); KX1-174 (Kinex); KX1-141 (Kinex); KX2-328 (Kinex); KX1-306 (Kinex); KX1-329 (Kinex); KX2-391 (Kinex); KX2-377 (Kinex); ZD4190 (Astra Zeneca; N-(4-bromo-2-fluorophenyl)-6-methoxy-7-(2-(1H-1,2,3-triazol-1-yl)ethoxy)quinazolin-4-amine); AP22408 (Ariad Pharmaceuticals); AP23236 (Ariad Pharmaceuticals); AP23451 (Ariad Pharmaceuticals); AP23464 (Ariad Pharmaceuticals); AZD0530 (Astra Zeneca); AZM475271 (M475271; Astra Zeneca); Dasatinib (N-(2-chloro-6-methylphneyl)-2-(6-(4-(2-hydroxyethyl)-piperazin-1-yl)-2-methylpyrimidin-4-ylamino) thiazole-5-carboxamide); GN963 (trans-4-(6,7-dimethoxyquinoxalin-2ylamino)cyclohexanol sulfate); Bosutinib (4-((2,4-dichloro-5-methoxyphenyl)amino)-6-methoxy-7-(3-(4-methyl-1-piperazinyl)propoxy)-3-quinolinecarbonitrile); or combinations thereof.

*Caspase modulators*

In some embodiments, an antagonist, partial agonist, inverse agonist, neutral or competitive antagonist, allosteric antagonist, and/or orthosteric antagonist of a caspase target, including but not limited to caspase-8 and/or caspase-9, is suitable for use in methods and compositions described herein. In some embodiments, the caspase inhibitor is z-VAD-FMK (Benzyloxycarbonyl-Val-Ala-Asp(OMe)-fluoromethylketone); z-LEHD-FMK (benzyloxycarbonyl-Leu-Glu(OMe)-His-Asp(OMe)-fluoromethylketone); B-D-FMK (boc-aspartyl(Ome)-fluoromethylketone); Ac-LEHD-CHO (N-acetyl-Leu-Glu-His-Asp-CHO); Ac-IETD-CHO (N-acetyl-Ile-Glu-Thr-Asp-CHO); z-IETD-FMK (benzyloxycarbonyl-Ile-Glu(OMe)-Thr-Asp(OMe)-fluoromethy lketone); FAM-LEHD-FMK (benzyloxycarbonyl Leu-Glu-His-Asp-fluoromethyl ketone); FAM-LETD-FMK (benzyloxycarbonyl Leu-Glu-Thr-Asp-fluoromethyl ketone); Q-VD-OPH (Quinoline-Val-Asp-$CH_2$-O-Ph); or combinations thereof.

*Sirtuin Modulators modulators*

Some embodiments incorporate the use of one or more antagonists, partial agonists, inverse agonists, neutral or competitive antagonists, allosteric antagonists, and/or orthosteric antagonists of sirtuins as active agents. In some embodiments, the agonist, partial agonist, and/or positive allosteric modulator of sirtuin activity is a stilbene, flavone, isoflavone, flavanone, catechin, free radical protective compound, isonicotinamide, dipyridamole, ZM 336372 (3-(dimethylamino)-N-[3-[(4-hydroxybenzoyl)-amino]-4-met hylphenyl]benzamide), camptothecin, coumestrol, nordihydroguaiaretic acid, esculetin, SRT-1720 (Sirtris), SRT-1460 (Sirtris), SRT-2183 (Sirtris), resveratrol, piceatannol, rhapontin, deoxyrhapontin, butein, a chalcone (e.g.,chalcon; isoliquirtigen; butein; 4,2',4'-trihydroxychalcone; 3,4,2',4',6'-pentahydroxychalcone); morin, fisetin; luteolin; quercetin; kaempferol; apigenin; gossypetin; myricetin; 6-hydroxyapigenin; 5-hydroxyflavone; 5,7,3',4',5'-pentahydroxyflavone; 3,7,3',4',5'-pentahydroxyflavone; 3,6,3',4'-tetrahydroxyflavone; 7,3',4',5'-tetrahydroxyflavone; 3,6,2',4'-tetrahydroxyflavone; 7,4'-dihydroxyflavone; 7,8,3',4'-tetrahydroxyflavone; 3,6,2',3'-tetrahydroxyflavone; 4'-hydroxyflavone; 5-hydroxyflavone; 5,4'-dihydroxyflavone; 5,7-dihydroxyflavone; or combinations thereof.

Other pro-apoptotic and anti-apoptotic agents are described in U.S. Appl. No. 12/500,486 which agents are incorporated herein by reference and are contemplated as being within the scope of embodiments presented herein.

## Antihistamines

Contemplated for use with the formulations disclosed herein are agents which reduce or ameliorate symptoms or effects as a result of wheal and flare in sinonasal passages. Accordingly, some embodiments of the methods and compositions described herein incorporate the use of antihistamines for treatment of sinonasal conditions, Examples of antihistamines suitable for methods and compositions described herein include, but are not limited to, meclizine, diphenhydramine, loratadine, levocetirizine, fexofenadine, quetiapine, mepyramine, piperoxan, antazoline, carbinoxamine, doxylamine, clemastine, dimenhydrinate, pheniramine, chlorphenamine, chlorpheniramine, dexchlorpheniramine, brompheniramine, triprolidine, cyclizine, chlorcyclizine, hydroxyzine, promethazine, alimemazine, trimeprazine, cyproheptadine, azatadine, ketotifen, oxatomide, meclizine hydrochloride, promethazine hydrochloride, hydroxyzine pamoate, chlorperazine, or the like.

Other antihistamines are described in U.S. Appl. Nos. U.S. Appl. Nos. 12/472,034 and 12/427,663, which agents are incorporated herein by reference and are contemplated as being within the scope of embodiments presented herein.

## Ion Channel modulators

*NMDA receptor modulators*

Contemplated for use with the formulations disclosed herein are agents which reduce or ameliorate symptoms or effects as a result of aberrant ion channel activity in epithelial cells lining sinusoidal cavities and/or in auris hair cells. In some instances, aberrant NMDA receptor activity is associated with influx of $Ca^{2+}$ and/or $Na^+$ ions in epithelial cells. Accordingly, some embodiments of the methods and compositions described herein incorporate the use of NMDA receptor antagonists or NMDA receptor agonists for treatment of sinonasal and/or otic conditions associated with aberrant ion channel activity. Examples of NMDA receptor antagonists include and are not limited to aminoadamantane, dextromethorphan, dextrorphan, ibogaine, ketamine (including R or S ketamine), nitrous oxide, phencyclidine, riluzole, tiletamine, memantine, neramexane, dizocilpine, aptiganel, remacimide, 7-chlorokynurenate, DCKA (5,7-dichlorokynurenic acid), kynurenic acid, 1-aminocyclopropanecarboxylic acid (ACPC), AP7 (2-amino-7-phosphonoheptanoic acid), APV (R-2-amino-5-phosphonopentanoate), CPPene (3-[(R)-2-carboxypiperazin-4-yl]-prop-2-enyl-1-phosphonic

acid); (+)-(1S,2S)-1-(4-hydroxyphenyl)-2-(4-hydroxy-4-phenylpiperidino)-1-pro-panol; (1S,2S)-1-(4-hydroxy-3-methoxyphenyl)-2-(4-hydroxy-4-phenylpiperi-dino)-1-propanol; (3R,4S)-3-(4-(4-fluorophenyl)-4-hydroxypiperidin-1-yl-)-chroman-4,7-diol; (1R*,2R*)-1-(4-hydroxy-3-methylphenyl)-2-(4-(4-fluoro-phenyl)-4-hydroxypiperidin-1-yl)-propan-1-ol-mesylate; AM-101, L-701324, dextrometorphan, eliprodil, and/or combinations thereof.

*ENaC Receptor Modulators*

In some embodiments, an active agent modulates ion channel activity (e.g., in auris hair cells, in sinonasal epithelia) and is a modulator of ENaC channels. The epithelial sodium channel (ENaC, sodium channel non-neuronal 1 (SCNN1) or amiloride sensitive sodium channel (ASSC)) is a membrane-bound ion-channel that is permeable for $Li^+$-ions, protons and $Na^+$-ions. The ENaC is located in the apical membrane of polarized epithelial cells and is involved in transepithelial $Na^+$-ion transport. $Na^+$/K+-ATPase is also involved in $Na^+$ transport and ion homeostasis. Examples of modulators of the activity of ENaC include, by way of example, the mineralcorticoid aldosterone, triamterene, and amiloride.

*Calcium channel modulators*

In some embodiments, an active agent modulates ion channel activity (e.g., in auris hair cells, in sinonasal epithelia) and is a calcium channel agonist or antagonist. In some embodiments, the calcium channel antagonist is cinnarizine, flunarizine, or nimodipine. Other calcium channel blockers include and are not limited to verapamil, diltiazem, omega-conotoxin, GVIA, amlodipine, felodipine, lacidipine, mibefradil, NPPB (5-Nitro-2-(3-phenylpropylamino)benzoic Acid), flunarizine, and/or combinations thereof

*Potassium channel modulators*

In some embodiments, an active agent modulates ion channel activity (e.g., in auris hair cells, in sinonasal epithelia) and is a potassium channel agonist or antagonist. In some embodiments, the the agonist of a potassium channel is nicorandil; minoxidil, levcromakalim; lemakalim; cromakalim; L-735,334 (14-hydroxy CAF-603 oleate); retigabine; flupirtine; BMS-204352 (3S)-(+)-(5-Chloro-2-methoxyphenyl)-1,3-dihydro-3-fluoro-6-(trifluoromethyl)-2H-indole-2-one); DMP-543 (10,10-bis((2-fluoro-4-pyridinyl)methyl)-9(10H)-anthracenone); or combinations thereof.

In some embodiments, an active agent modulates potassium channel activity (e.g., in auris hair cells, in sinonasal epithelia) and is an antagonist of a potassium channel (e.g. a potassium channel blocker). In some embodiments, the antagonist of a potassium channel is linopirdine; XE991 (10,10-bis(4-pyridinylmethyl)-9(10H)-anthracenone); 4-AP (4-aminopyridine); 3,4-DAP (3,4-Diaminopyridine); E-4031 (4'-[[1-[2-(6-methyl-2-pyridyl)ethyl]-4-piperidinyl]carbonyl]-methanesulfonanilide); DIDS (4,4'-diisothiocyanostilbene-2,2'-disulfonic acid); Way 123,398 (N-methyl-N-(2-(methyl(1-methyl-1H-benzimidazol-2-yl)amino)ethyl)-4-((methylsulfonyl)amino) benzenesulfonamide HCl); CGS-12066A (7-Trifluoromethyl-4-(4-methyl-1-piperazinyl)pyrrolo-[1,2-a]quinoxaline); dofetilide; sotalol; apamin; amiodarone; azimilide; bretylium; clofilium; tedisamil; ibutilide; sematilide; nifekalant; tamulustoxin and combinations thereof.

*Sodium channel modulators*

In some embodiments, an active agent modulates ion channel activity (e.g., in auris hair cells, in sinonasal epithelia) and is a sosium channel agonist or antagonist. In some embodiments, a $Na^+$ channel blocker is vinpocetine ((3a,16a)-Eburnamenine-14-carboxylic acid ethyl ester); sipatrigine (2-(4-Methylpiperazin-1-yl)-5-(2,3,5-trichlorophenyl)-pyrimidin-4-amine); amiloride (3,5 -diamino-N-(aminoiminomethyl)-6-chloropyrazinecarbox amide hydrochloride); carbamazepine (5H-dibenzo[b,f]azepine-5-carboxamide); TTX (octahydro-12-(hydroxymethyl)-2-imino-5,9:7,10a-dimethan o-10aH-[1,3]dioxocino[6,5-d]pyrimidine-4,7,10,11,12-pen tol); RS100642 (1-(2,6-dimethyl-phenoxy)-2-ethylaminopropane hydrochloride); mexiletine ((1-(2,6-dimethylphenoxy)-2-aminopropane hydrochloride)); QX-314 (N-(2,6-Dimethylphenylcarbamoylmethyl)triethylammonium bromide); phenytoin (5,5-diphenylimidazolidine-2,4-dione); lamotrigine (6-(2,3-dichlorophenyl)-1,2,4-triazine-3,5-diamine); 4030W92 (2,4-diamino-5-(2,3-dichlorophenyl)-6-fluoromethylpyrimidine); BW1003C87 (5-(2,3,5-trichlorophenyl) pyrimidine-2,4- 1.1 ethanesulphonate); QX-222 (2-[(2,6-dimethylphenyl)amino]-N,N,N-trimethyl-2-oxoetha niminium chloride); ambroxol (trans-4-[[(2-Amino-3,5-dibromophenyl)methyl]amino]cyclo hexanol hydrochloride); R56865 (N-[1-(4-(4-fluorophenoxy)butyl]-4-piperidinyl-N-methyl-2-benzo-thiazolamine); lubeluzole; ajmaline ((17R,21alpha)-ajmalan-17,21-diol); procainamide (4-amno-N-(2-diethylaminoethyl)benzamide hydrochloride); flecainide; riluzoleor; or combinations thereof.

*AMPA receptor modulators*

In some embodiments, an active agent modulates ion channel activity (e.g., in auris hair cells, in sinonasal epithelia) and is an AMPA receptor antagonist. In some embodiments, the agent which antagonizes the AMPA receptors is CNQX (6-cyano-7-nitroquinoxaline-2,3-dione); NBQX (2,3-dihydroxy-6-nitro-7-sulfamoyl-benzo[f]quinoxaline-2,3-dione); DNQX (6,7-dinitroquinoxaline-2,3-dione); kynurenic acid; 2,3-dihydroxy-6-nitro-7-sulfamoylbenzo-[f]quinoxaline; or combinations thereof.

*Metabotropic glutamate receptor modulators*

In some embodiments, an active agent modulates ion channel activity (e.g., in auris hair cells, in sinonasal epithelia) and indirectly controls the opening of ion channels by the activation of biochemical cascades. In some of such embodiments, the agent is a modulator of mGlu receptors.

Examples of agents that are group II mGlu receptor agonists include and are not limited to LY389795 ((-)-2-thia-4-aminobicyclo-hexane-4,6-dicarboxylate); LY379268 ((-)-2-oxa-4-aminobicyclo-hexane-4,6-dicarboxylate); LY354740 ((+)-2-aminobicyclo-hexane-2,6dicarboxylate); DCG-IV ((2S,2'R,3'R)-2-(2',3'-dicarboxycyclopropyl)glycine); 2R,4R-APDC (2R,4R-4-aminopyrrolidine-2,4-dicarboxylate), (S)-3C4HPG ((S)-3-carboxy-4-hydroxyphenylglycine); (S)-4C3HPG ((S)-4-carboxy-3-hydroxyphenylglycine); L-CCG-I ((2S,1'S,2'S)-2-(carboxycyclopropyl)glycine); or the like. Example of agents that are group III mGlu receptor agonists include and are not limited to ACPT-I ((1S,3R,4S)-1-aminocyclopentane-1,3,4-tricarboxylic acid); L-AP4 (L-(+)-2-Amino-4-phosphonobutyric acid); (S)-3,4-DCPG ((S)-3,4-dicarboxyphenylglycine); (RS)-3,4-DCPG ((RS)-3,4-dicarboxyphenylglycine); (RS)-4-phosphonophenylglycine ((RS)PPG); AMN082 (,N'-bis(diphenylmethyl)-1,2-ethanediamine dihydrochloride); DCG-IV ((2S,2'R,3'R)-2-(2',3'-dicarboxycyclopropyl)glycine); or the like. Other mGlu receptor modulators include and are not limited to is 3,5-Dimethyl pyrrole-2,4-dicarboxylic acid 2-propyl ester 4-(1,2,2-trimethyl-propyl) ester (3,5-dimethyl PPP); 3,3'-difluorobenzaldazine (DFB), 3,3'-dimlethoxybenzaldazine (DMeOB), 3,3'-dichlorobenzaldazine (DCB) and other allosteric modulators of $mGluR_5$ disclosed in Mol. Pharmacol. 2003, 64, 731-740; (E)-6-methyl-2-(phenyldiazenyl)pyridin-3-ol (SIB 1757); (E)-2-methyl-6-styrylpyridine (SIB 1893); 2-methyl-6-(phenylethynyl)pyridine (MPEP), 2-methyl-4-((6-methylpyridin-2-yl)ethynyl)thiazole (MTEP); 7-(Hydroxyimino)cyclopropa[b]chromen-1$\alpha$-carboxylate ethyl ester (CPCCOEt), N-cyclohexyl-3-methylbenzo[d]thiazolo[3,2-a]imidazole-2-carboxamide (YM-298198), tricyclo[3.3.3.1]nonanyl quinoxaline-2-carboxamide (NPS 2390); 6-methoxy-N-(4-methoxyphenyl)quinazolin-4-amine (LY 456239), piracetam, Oxiracetam, Aniracetam, Pramiracetam, Phenylpiracetam (Carphedon), Etiracetam, Levetiracetam, Nefiracetam, Nicoracetam, Rolziracetam, Nebracetam, Fasoracetam, Coluracetam, Dimiracetam, Brivaracetam, Seletracetam, Rolipram or the like.

*TRPV1 modulators*

In some embodiments, an active agent modulates ion channel activity (e.g., in auris hair cells, in sinonasal epithelia) and is a TRPV1 agonist or antagonist. In some embodiments, an agonist of one or more of the TRPV receptors is capsaicin, resiniferatoxin, or combinations thereof.

Other ion channel modulators include purinergic receptor modulators, GABA receptor modulators or the like. Ion channel modulators described in U.S. Appl. Nos. 12/506,664, 12/427,663, and 12/494,156 are incorporated herein by reference and are contemplated as being within the scope of embodiments presented herein.

Anti-angiogenesis Agents

Contemplated for use with the formulations disclosed herein are agents which are anti-angiogenesis agents. In some embodiments, the formulations provided herein allow for sustained release of anti-angiogenic in the intrasinusoidal and/or nasal and/or nasopharyngeal regions. In some embodiments, the anti-angiogenesis agent is a modulator of the VEGF1 and/or VEGF2 receptor(s). Examples of anti-angiogenic agents that are suitable for use in the methods described herein include and are not limited to bevacizumab, thalidomide, linomide,TNP-470, matrix metalloprotease inhibitors, VEGFR antagonists, and the like.

**Immunosuppressants**

Contemplated for use with the formulations disclosed herein are agents which are immunosupressants. In some embodiments, the formulations provided herein allow for sustained release of immunosuppressants in an affected area for long term treatment of condition such as, for example, Wegerner's granulomatosis. Further, the intrasinusoidal and/or nasal and/or nasopharyngeal formulations described herein are administered with reduced dosing frequency thereby improving patient compliance and comfort where long term therapy is indicated. Examples of immunosuppressants include and are not limited to Cyclosporine, 6-MP, and Methotrexate. In some embodiments, an immunosuppresant is an agent that acts at glucocorticoid receptors (e.g., any glucocorticoid described herein, including and not limited to Hydrocortisone, Cortisone, Prednisone, Prednisolone, Methylprednisolone, Dexamethasone, Betamethasone, Triamcinolone, Beclometasone, Fludrocortisone acetate, Aldosterone or the like).

RNAi

In some embodiments, where inhibition or down-regulation of a target is desired (e.g. genes encoding one or more calcineurins, IKKs, TACEs, TLRs, or cytokines), RNA interference are utilized. In some embodiments, the agent that inhibits or down-regulates the target is an siRNA molecule. In certain instances, the siRNA molecule inhibits the transcription of a target by RNA interference (RNAi). In some embodiments, a double stranded RNA (dsRNA) molecule with sequences complementary to a target is generated (e.g. by PCR). In some embodiments, a 20-25 bp siRNA molecule with sequences complementary to a target is generated. In some embodiments, the 20-25 bp siRNA molecule has 2-5 bp overhangs on the 3' end of each strand, and a 5' phosphate terminus and a 3' hydroxyl terminus. In some embodiments, the 20-25 bp siRNA molecule has blunt ends. For techniques for generating RNA sequences see Molecular Cloning: A Laboratory Manual, second edition (Sambrook et al., 1989) and Molecular Cloning: A Laboratory Manual, third edition (Sambrook and Russel, 2001), jointly referred to herein as "Sambrook"); Current Protocols in Molecular Biology (F. M. Ausubel et al., eds., 1987, including supplements through 2001); Current Protocols in Nucleic Acid Chemistry John Wiley & Sons, Inc., New York, 2000) which are hereby incorporated by reference for such disclosure.
In some embodiments, the dsRNA or siRNA molecule is incorporated into a sustained-release formulation described herein and is injected into or in the vicinity of the sinonasal and/or otic cavity or structure.
In certain instances, after administration of the dsRNA or siRNA molecule, cells at the site of administration (e.g. the cells of sinonasal passages, auris hair cells) are transformed with the dsRNA or siRNA molecule. In certain instances following transformation, the dsRNA molecule is cleaved into multiple fragments of about 20-25 bp to yield siRNA molecules. In certain instances, the fragments have about 2bp overhangs on the 3' end of each strand.
In certain instances, an siRNA molecule is divided into two strands (the guide strand and the anti-guide strand) by an RNA-induced Silencing Complex (RISC). In certain instances, the guide strand is incorporated into the catalytic component of the RISC (i.e. argonaute). In certain instances, the guide strand binds to a complementary target mRNA sequence. In certain instances, the RISC cleaves the target mRNA. In certain instances, the expression of the target gene is down-regulated.
In some embodiments, a sequence complementary to a target is ligated into a vector. In some embodiments, the sequence is placed between two promoters. In some embodiments, the promoters are orientated in opposite directions. In some embodiments, the vector is contacted with a cell. In certain instances, a cell is transformed with the vector. In certain instances following transformation, sense and anti-sense strands of the sequence are generated. In certain instances, the sense and anti-sense strands hybridize to form a dsRNA molecule which is cleaved into siRNA molecules. In certain instances, the strands hybridize to form an siRNA molecule. In some embodiments, the vector is a plasmid (e.g pSUPER; pSUPER.neo; pSUPER.neo+gfp).
In some embodiments, the vector is incorporated into a sustained release microsphere or microparticle, hydrogel, liposome, or thermoreversible gel.

**Other agents**

In some embodiments, agents that are suitable for use in formulations described herein include agents that modulate activity of epithelial cells lining the sinonasal cavities and/or nasal passages and/or auris hair cells. Examples of agents that modulate the activity of epithelial cells include and are not limited to modulators of the PTEN pathway; modulators of PPAR; modulators of EGFR; growth factors including and not limited to TGF-beta, and fibroblast growth factor; and/or modulators of epithelial cell adhesion.
In some embodiments, agents suitable for use in formulations described herein include agents that modulate synthesis and/or activity of keratin (e.g., actinomycin D, vitamin A, or the like). In some embodiments, agents that are suitable for use in intrasinusoidal formulations described herein include agents that modulate eosinophils and/or inflammatory cytokines. Examples of agents that modulate the activity of eosinophils and/or inflammatory cytokines include and are not limited to leukotriene blockers (e.g., monteleukast, Singulair®), prostaglandin $D_2$ receptor (PGD2) modulators, lipophosphatidic acid receptor (LPA) modulators, 5-lipoxygenase activating protein (FLAP) modulators, CRTH2 (DP2) modulators, or the like. In some embodiments, agents suitable for use in intrasinusoidal formulations described herein include agents that modulate cadherins (e.g., Trichostatin A, ADH1 (Molecular and Cellular Neuroscience, 28, 2005, 253-263), Antibody sc-59778 or the like).
In some embodiments, active agents compatible with the formulations described herein include neurotoxins for the treatment of active nerve disorders. Such neurotoxins include venoms, channel agents and/or nerve agents

including but not limited to Botulinum Toxin Type A (Botox®), erabutoxin, tetrodotoxin, batrachotoxin, maurotoxin, agitoxin, charybdotoxin, margatoxin, slotoxin, scyllatoxin, hefutoxin, calciseptine, taicatoxin, calcicludine, PhTx3 or the like. In some embodiments, active agents compatible with the formulation described herein include vascular and/or vestibular suppressants. Examples of vestibular suppressants include and are not limited to meclizine, amytriptyline, droperidol and other vascular and/or vestibular suppressants described in U.S. Appl. No. 12/486,697, vascular and/or vestibular suppressants described therein are incorporated herein by reference). In some embodiments, active agents compatible with the formulations described herein include agents that modulate re-growth of damaged auris sensory hair cells. In some instances, modulation of the WNT pathway promotes morphogenesis and/or re-growth of damaged auris sensory hair cells. WNT signalling proteins include protein products encoded by genes such as WNT1, WNT2, WNT2B, WNT3, WNT3A, WNT4, WNT5A, WNT5B, WNT6, WNT7A, WNT7B, WNT8A, WNT8B, WNT9A, WNT9B, WNT10A, WNT10B, WNT11, or WNT16. Modulators of the WNT pathway include, and are not limited to, 2-amino-4- [3,4-(methylenedioxy)benzyl-amino] -6-(3 -methoxyphenyl)pyrimidine, the signalling molecule Cerberus, or the like.

Other active agents that are compatible with the formulations described herein include and are not limited to active agents described in U.S. Appl. Nos. 12/427,663, 12/466,310, 12/472,034, 12/486,697, 12/493,611, 12/494,156, 12/500,486, 12/504,553, 12/506,091, 12/506,127, 12/506,573, 12/506,616, and 12/506,664, the disclosure of active agents described therein is incorporated herein by reference.

## Combination therapy

### Antimicrobial agents and Anti-inflammatory agents

Contemplated within the scope of the embodiments presented herein are compositions that comprise an antimicrobial agent in combination with an anti-inflammatory agent. In specific embodiments, a formulation described herein comprises an antimicrobial agent (e.g., any antimicrobial agent described herein) in combination with an anti-inflammatory agent (e.g., any anti-inflammatory agent described herein). In certain embodiments, a formulation described herein comprises an antibiotic (e.g., any antibiotic described herein) in combination with a corticosteroid.

In some embodiments, a composition comprising an antibiotic and a corticosteroid has different release profiles for each of the active agents. In other embodiments, a composition comprising an antibiotic agent and a corticosteroid agent has substantially similar release profiles for each of the active agents.

In certain embodiments, a formulation described herein comprises an antibiotic in combination with dexamethasone. In certain embodiments, a formulation described herein comprises an antibiotic in combination with methylprednisolone or prednisolone. In certain embodiments, a formulation described herein comprises ciprofloxacin in combination with dexamethasone. In certain embodiments, a formulation described herein comprises ciprofloxacin in combination with methylprednisolone or prednisolone or triamcinolone.

In some embodiments, a composition comprising an antibiotic and a corticosteroid contains one or both active agents as multiparticulates (e.g., as micronized active agents). By way of example, in some embodiments, a composition comprising water soluble dexamethasone and multiparticulates of a form of ciprofloxacin with poor water solubility provides extended release of dexamethasone for at least 3 days and extended release of ciprofloxacin for at least 10 days. By way of example, in some embodiments, a composition comprising multiparticulates (e.g., micronized particles) of a form of dexamethasone with poor water solubility, and a water soluble form of ciprofloxacin provides extended release of ciprofloxacin for at least 3 days and extended release of dexamethasone for at least 10 days. By way of example, in some embodiments, a composition comprising multiparticulates (e.g., micron-sized particles, nanoparticles, non-sized particles) of a form of dexamethasone with poor water solubility and mulitparticulates (e.g., micron-sized particles, nanoparticles, non-sized particles) of a form of ciprofloxacin with poor water solubility provides an extended release of each active agent for at least 7 days.

Other active agents suitable for combination therapy include and are not limited to agents described in U.S. Appl. Nos. 12/427,663, 12/466,310, 12/472,034, 12/486,697, 12/493,611, 12/494,156, 12/500,486, 12/504,553, 12/506,091, 12/506,127, 12/506,573, 12/506,616, and 12/506,664, agents described therein are incorporated herein by reference.

## Imaging devices

In some embodiments, any formulation described herein is used in combination with a mechanical or imaging device to monitor or survey the condition being treated. For example, magnetic resonance imaging (MRI) devices are contemplated within the scope of the embodiments described herein, wherein the MRI devices (for example, 3 Tesla MRI devices) are capable of evaluating disease progression, and subsequent treatment with the pharmaceutical formulations disclosed herein. In some embodiments, formulations described herein comprise Gadolinium-based dyes, iodine-based dyes, barium-based dyes, or the like and are used in the treatment of any active disorder described herein and/or

with any mechanical or imaging device or method described herein (e.g., a CAT scan). Such formulations allow for visualization of disease progression and/or formulation penetration (e.g., penetration across round window membrane into the inner ear and/or therapeutic effectiveness of the formulation. In certain embodiments, an imaging agent (e.g., gadolinium hydrate injection) is used in combination with three-dimensional real inversion recovery (3D-real IR) and/ three-dimensional fluid-attenuated inversion recovery (3D-FLAIR) magnetic resonance imaging (MRI), and/or any formulation described herein to evaluate disease severity (e.g., size of nasal polyps), formulation penetration at the site or administration, and/or therapeutic effectiveness of the formulation. In some instances, a formulation described herein facilitates delivery of a sufficient amount of an imaging agent to the site of treatment and allows for visualization of disease progression and/or formulation penetration and/or therepautic effectiveness of the formulation.

In some embodiments, the compositions described herein include a dye to help enhance the visualization of penetration of the formulation targeted sites of administration and/or treatment. In some of such embodiments, dyes that are compatible with the compositions described herein include and are not limited to Evans blue, Methylene blue, Isosulfan blue, Trypan blue, indocyanine green or the like.

**Surgery and Implants**

In some embodiments, the pharmaceutical formulations described herein are used in combination with (e.g., implantation, short-term use, long-term use, or removal of) implants (e.g., cochlear implants). As used herein, implants include medical devices, examples of which include cochlear implants, hearing sparing devices, hearing-improvement devices, short electrodes, tympanostomy tubes, micro-prostheses or piston-like prostheses; needles; stem cell transplants; drug delivery devices; any cell-based therapeutic; drug delivery stent; catheter, ballon rhinoplasty; or the like.

In some embodiments, administration of an pharmaceutical formulation described herein in combination with surgery delays or prevents collateral damage, e.g., irritation, inflammation and/or infection, caused by the external active intervention (e.g., installation of an external device or surgery). In some embodiments, administration of an pharmaceutical formulation described herein in combination with an active intervention reduces or eliminates post-surgical and/or post-implantation complications (e.g., inflammation, cell damage, infection, osteoneogenesis or the like). In some instances, perfusion of a surgical area with a formulation described herein reduces post-surgery or post-implantation recuperation time. In one aspect, formulations described herein, and modes of administration thereof, are applicable to methods of direct perfusion at the site of srugery, during surgery, before surgery or after surgery, or a combination thereof. In specific embodiments, formulations described herein comprising an anti-microbial agent (e.g., an antibiotic such as ciprofloxacin) or an anti-inflammatory agent (e.g., a corticosteroid such as dexamethasone, triamcinolone or the like), or a combination thereof, is administered in combination with surgery (e.g., ear surgery for cholesteatoma or Glue ear).

**Sterilization**

Included within the embodiments disclosed herein are means and processes for sterilization of a pharmaceutical formulation disclosed herein for use in humans. The goal is to provide a safe pharmaceutical product, relatively free of infection causing microorganisms. The U. S. Food and Drug Administration has provided regulatory guidance in the publication "Guidance for Industry: Sterile Drug Products Produced by Aseptic Processing" available at: http://www.fda.gov/cder/guidance/5882fnl.htm, which is incorporated herein by reference in its entirety.

As used herein, sterilization means a process used to destroy or remove microorganisms and/or pyrogens that are present in a product or packaging. Available methods for the inactivation of microorganisms include, but are not limited to, the application of extreme heat, lethal chemicals, or gamma radiation.

Heat sterilization methods include the use of a saturated steam autoclave at a temperature of at least 121 °C, or dry heat sterilization (e.g., heating a dry powder for about 3 - 11 hours at internal powder temperatures of 130-140 °C, or for 1-2 hours at internal temperatures of 150-180 °C). Filtration sterilization is a method used to remove microorganisms from solutions.

In some embodiments, a formulation is subjected to terminal sterilization. In other words, the formulation that is autoclaved comprises the active agent and all the excipients. In other embodiments, all the excipients are subjected to heat sterilization and the active agent is sterilized separately; the active agent and the excipients are then mixed aseptically. In yet other embodiments, the active agent is sterilized separataely (e.g., dry-heat sterilized, irradiated, steam-sterilized) and the other excipients are sterile-filtered; the sterile active agent and the sterile-filtered solution are then mixed aseptically. In further embodiments, a sterile suspension of active agent in a solution comprising a thermosetting polymer is aseptically mixed with a second solution comprising a thermosetting polymer and optionally a second active agent.

In some instances, conventionally used methods of sterilization (e.g., heat treatment (e.g., in an autoclave), gamma irradiation, filtration) lead to irreversible degradation of polymeric components (e.g., thermosetting polymer components) and/or the active agent in the formulation. In some instances, sterilization of a pharmaceutical formulation

by filtration through membranes (e.g., 0.2 μm membranes) is not possible if the formulation comprises thixotropic polymers.

Accordingly, provided herein are methods for sterilization of pharmaceutical formulations that prevent degradation of polymeric components and/or the active agent during the process of sterilization. In some embodiments, the use of an appropriate thermosetting polymer in combination with a specific buffer and/or pH range for the formulation allows for high temperature terminal sterilization of formulations described herein with substantially low degradation of the therapeutic agent and/or the polymeric excipients.

Any appropriate buffer is used depending on the active agent used in the formulation. In some instances, since $pK_a$ of TRIS decreases as temperature increases at approximately -0.03/°C and $pK_a$ of PBS increases as temperature increases at approximately 0.003/°C, autoclaving at 250°F (121°C) results in a significant downward pH shift (i.e. more acidic) in the TRIS buffer whereas a relatively much less upward pH shift in the PBS buffer and therefore much increased hydrolysis and/or degradation of an active agent in TRIS than in PBS. Degradation of an active agent and/or polymeric components is reduced by the use of an appropriate combination of a buffer and concentration of thermosensitive polymer.

In certain embodiments, any sustained release formulation described herein has less than about 100 colony forming units, less than about 60 colony forming units, less than about 50 colony forming units, less than about 40 colony forming units, or less than about 30 colony forming units of microbial agents per gram of formulation. The sterile formulations described herein are substantially free of microbes.

An additional aspect of the sterilization process is the removal of by-products from the killing of microorganisms. The process of depyrogenation removes such pyrogens from the sample. Because the molecular size of endotoxins can vary widely, the presence of endotoxins is expressed in "endotoxin units" (EU). One EU is equivalent to 100 picograms of E. coli LPS. Humans can develop a response to as little as 5 EU/kg of body weight. In certain embodiments, active compositions described herein contain lower endotoxin levels (e.g. < 5 EU/kg of body weight of a subject, < 4 EU/kg of body weight of a subject) when compared to conventionally acceptable endotoxin levels (e.g., 5 EU/kg of body weight of a subject). In certain embodiments, the formulations described herein are substantially free of pyrogens.

**pH and Practical Osmolarity**

In some embodiments, an pharmaceutical formulation disclosed herein is formulated to provide an ionic balance that is compatible biological fluids (e.g., endolymph and/or perilymph in an inner ear environment, spinal fluid in the intrathecal space or the like).

As used herein, "practical osmolarity/osmolality" or "deliverable osmolarity/osmolality" means the osmolarity/osmolality of a formulation as determined by measuring the osmolarity/osmolality of the active agent and all excipients except the thermosensitive polymer agent (e.g., polyoxyethylene-polyooxypropylene copolymers, or the like). The practical osmolarity of a formulation disclosed herein is measured by any suitable method, e.g., a freezing point depression method as described in Viegas et. al., Int. J. Pharm., 1998, 160, 157-162. In some instances, the practical osmolarity of a formulation disclosed herein is measured by vapor pressure osmometry (e.g., vapor pressure depression method) that allows for determination of the osmolarity of a formulation at higher temperatures. In some instances, vapor pressure depression method allows for determination of the osmolarity of a formulation comprising a a thermosensitive polymer at a higher temperature such as for example the gelation temperature of the thermosensitive polymer.

In some embodiments, the osmolarity at a target site of action (e.g., the perilymph in the inner ear, spinal fluid, sinonasal fluids or the like) is about the same as the practical osmolarity (i.e., osmolarity of materials that cross or penetrate the round window membrane in the ear) of a formulation described herein.

The practical osmolality of an pharmaceutical formulation disclosed herein is from about 100 mOsm/kg to about 1000 mOsm/kg, from about 200 mOsm/kg to about 800 mOsm/kg, from about 250 mOsm/kg to about 500 mOsm/kg, or from about 250 mOsm/kg to about 320 mOsm/kg, or from about 250 mOsm/kg to about 350 mOsm/kg or from about 280 mOsm/kg to about 320 mOsm/kg. In some embodiments, a formulation described herein has a practical osmolarity of about 100 mOsm/L to about 1000 mOsm/L, about 200 mOsm/L to about 800 mOsm/L, about 250 mOsm/L to about 500 mOsm/L, about 250 mOsm/L to about 350 mOsm/L, about 250 mOsm/L to about 320 mOsm/L, or about 280 mOsm/L to about 320 mOsm/L. In some embodiments, the practical osmolality is estimated as an additive combination of buffer osmolality and the osmolality of the supernatant of the gelled poloxamer in water.

In specific embodiments, the practical osmolality of a formulation described herein is measured in a cell-based assay. The osmolality experienced by red blood cells isolated from guinea pigs was determined as a function of the hemolysis index. RBCs were placed in poloxamer solutions of varying concentrations. 0.5 mL of 10% guinea pig red blood cells in saline was added into a 2.5 mL solution of poloxamer 407 in buffer. The resulting suspension was serially diluted and the hemolysis index of RBCs was recorded for each solution. The hemolysis index is defined as the ratio of absorbance of a sample at 540 nm to the absorbance of a 0.9% saline solution at 540 nm. A hemolysis index of 1 indicates that the "practical osmolality" experienced by the RBCs is suitable for inner ear administration. The RBCs are intact in media with a suitable practical osmolality (**Figure 28**). The osmolality of the poloxamer solution was also

measured by freezing point depression method or vapor pressure methods. The practical osmolality of the formulation is measured using commercially available osmometers and the value is confirmed by the hemolysis assay.

Table 10 shows a comparison of osmolality as determined by the serial dilution cell-based assay and a direct measurement using freezing point depression or vapor pressure methods. The serial dilution method is predictive of practical osmolality that is compatible with the inner ear environment.

**Table 10**

| Sample[a] | Hemolysis Index[b] | Osmolality per "Direct Measurement"(mOsM per FP/VP) |
|---|---|---|
| RBC in DI Water | 360 | 50/55 |
| RBC in 0.45% Saline | 5 | 170/161 |
| RBC in 0.9% Saline | 1 | 293/293 |
| RBC in 2% Saline | 2 | 611/619 |
| RBC in 10% Saline | 155 | >QL/2674 |
| RBC in P407-in-DI water | 19 | 245/114 |
| RBC in P407-in-50mM TRIS 0.3% NaCl | 1 | 458/310 |
| RBC in P407-in-50mM TRIS 0.45% NaCl | 1 | 523/377 |

FP: freezing-point osmometry; VP: vapor-pressure osmometry
[a] Sample preparation: 0.5 mL of 10% guinea pig red blood cells in saline was added into 2.5 mL of P407 in buffer solution
[b] Hemolysis Index is defined as the 540 nm Absorbance ratio of sample:0.9% saline

In some embodiments, useful formulations also include one or more pH adjusting agents or buffering agents. Suitable pH adjusting agents or buffers include, but are not limited to acetate, bicarbonate, ammonium chloride, citrate, phosphate, pharmaceutically acceptable salts thereof and combinations or mixtures thereof. In certain embodiments of the present disclosure, the amount of buffer included in the gel formulations are an amount such that the pH of the gel formulation does not interfere with the body's natural buffering system and/or the osmolarity of physiological fluids. In some embodiments, the pH of a formulation described herein is between about 3.0, 3.5, 4.0, 4.5, 5.0, 5.5, 6.0, 6.5, or 7.0 and about 7.0, 7.5, 8.0, 8.5, 9.0, 9.5, 10.0, 10.5, 11.0, 11.5, or 12.0. In some embodiments, the pH of a formulation described herein is between about 3.0 and about 12.0. In some embodiments, the pH of a formulation described herein is between about 4.5 and about 10.0. In some embodiments, the pH of a formulation described herein is between about 3.5 and about 8.5. In some embodiments, the pH of a formulation described herein is between about 5.5 and about 8.0. In some embodiments, the pH of a formulation described herein is between about 6.5 and about 8.0. In some embodiments, the pH of a formulation described herein is between about 7.0 and about 7.8. In some embodiments, the pH of a formulation described herein is between about 7.0 and about 7.6. In some embodiments, the pH of a formulation described herein is between about 7.0 and about 7.4. In some embodiments, the pH of a formulation described herein is between about 7.4 and about 7.8.

In some embodiments, the formulations described herein have a pH and/or practical osmolarity as described herein, and have a concentration of active pharmaceutical ingredient between about 1 μM and about 10 μM, between about 1 mM and about 100 mM, between about 0.1 mM and about 100 mM, betwen about 0.1 mM and about 100 nM. In some embodiments, the formulations described herein have a pH and/or practical osmolarity as described herein, and have a concentration of active pharmaceutical ingredient between about 0.01% - about 40%, between about 0.01% - about 20%, between about 0.01% - about 10%, between about 0.01% - about 7.5%, between about 0.01% - 6%, between about 0.01 - 5%, between about 0.1% - about 40%, between about 0.1% - about 30%, between about 0.1% - about 20%, between about 0.1 - about 10%, or between about 0.1 - about 6% of the active ingredient by weight of the formulation. In some embodiments, formulations described herein have a pH and/or practical osmolarity as described herein, and have a concentration of active pharmaceutical agent between about 1% - about 40%, between about 5% - about 40%, between about 10% - about 40%, betwen about 15% - about 40%, between about 10% - about 30%, between about 10% - 20%, betwen about 15% - about 25%, or between about 20% - 30%, of the active ingredient by weight of the formulation. In some embodiments, the formulations described herein have a pH and/or practical osmolarity as described herein, and have a concentration of active pharmaceutical ingredient between about 1 μg/mL and about 500 μg/mL, between about 1 μg/mL and about 250 μg/mL, between about 1 μg and about 100 μg/mL, between about 1

μg/mL and about 50 μg/mL, or between about 1 μg/mL and about 20 μg/mL of the active agent by volume of the formulation.

**Tunable Release Characteristics**

**Particle Size**

Size reduction is used to increase surface area and/or modulate formulation dissolution properties and/or to maintain a consistent average particle size distribution (PSD) (e.g., micrometer-sized particles, nanometer-sized particles or the like) for any formulation described herein. In some embodiments, any formulation described herein comprises multiparticulates, i.e., a plurality of particle sizes (e.g., micronized particles, nano-sized particles, non-sized particles, colloidal particles); i.e, the formulation is a multiparticulate formulation. In some embodiments, any formulation described herein comprises one or more multiparticulate (e.g., micronized) therapeutic agents. In some embodiments, any formulation described herein comprises micronized therapeutic agents. Micronization is a process of reducing the average diameter of particles of a solid material. In some embodiments, the average diameter of particles in a micronized solid is from about 0.5 μm to about 500 μm. In some embodiments, the average diameter of particles in a micronized solid is from about 1 μm to about 200 μm. In some embodiments, the average diameter of particles in a micronized solid is from about 2 μm to about 100 μm. In some embodiments, the average diameter of particles in a micronized solid is from about 3 μm to about 50 μm. In some embodiments, the use of multiparticulates of active agent allows for extended and/or sustained release of the active agent from any formulation described herein compared to a formulation comprising non-multiparticulate or a water-soluble active agent.

In specific embodiments, upon administration of a sustained release pharmaceutical formulation comprising micronized active agent to an individual in need thereof, the micronized active agent particles serve as a depot for further extended release of the active agent even after the gel has eroded. In some of such embodiments, the micronized particles remain adhered to active surfaces. Accordingly, in some embodiments, sustained release pharmaceutical formulations suitable for methods described herein comprise substantially high concentrations of micronized active agent. In some of such embodiments, sustained release pharmaceutical formulations are suspensions comprising micronized active agents.

In some instances, any particle in any formulation described herein is a coated or uncoated particle (e.g., a coated micronized particle, nano-particle) and/or a microsphere and/or a liposomal particle. Particle size reduction techniques include, by way of example, grinding, milling (e.g., air-attrition milling (jet milling), ball milling), coacervation, complex coacervation, high pressure homogenization, spray drying and/or supercritical fluid crystallization. In some instances, particles are sized by mechanical impact (e.g., by hammer mills, ball mill and/or pin mills). In some instances, particles are sized via fluid energy (e.g., by spiral jet mills, loop jet mills, and/or fluidized bed jet mills).

In some embodiments formulations described herein comprise crystalline particles and/or isotropic particles. In some embodiments, formulations described herein comprise amorphous particles and/or anisotropic particles. In some embodiments, formulations described herein comprise therapeutic agent particles wherein the therapeutic agent is a free base, or a salt, or a solvate, or a prodrug of a therapeutic agent, or any combination thereof.

As illustrated in **Figure 2**, compositions comprising multiparticulate (e.g., micronized) active agents provide extended release over a longer period of time compared to compositions comprising non-particulate and/or water soluble active agents. In some instances, the multiparticulate and/or less water-soluble active agent provides a steady supply (e.g., +/- 20%) of active agent via slow degradation and serves as a depot for the active agent; such a depot effect increases residence time of the active agent in the ear. In specific embodiments, selection of an appropriate particle size of the active agent (e.g., micronized active agent) and solubility of the active agent is water, in combination with the amount of thermosensitive polymer component in the composition, provides tunable extended release characteristics that allow for release of an active agent over a period of hours, days, weeks or months.

**Solubility**

The release characteristics of an active agent from a formulation described herein are tuned by modifying the solubility of the active agent in biological and/or aqueous media. One approach to extend release of an active agent is to desolubilize the soluble active agent. Solubility of the drug in biological and/or aqueous fluids is modified by selection of a pharmacologically acceptable salt that is insoluble or has a lower solubility than the drug alone or a different salt of the drug. In certain instances, solubility of the drug in biological and/or aqueous fluids is modified by selection of crystalline salt forms (polymorphs) that are insoluble or have lower solubility than other salt forms or the drug alone.

By way of example, in the case of anionic drugs (e.g., active agents bearing acidic moieties like carboxylic acids, phosphates, sulfates, or the like) a soluble drug is rendered insoluble or less soluble in biological and/or aqueous fluids by exchanging the counterion from a Group I metal ion (e.g., sodium or potassium), to a counterion from group II

of the periodic table (e.g., calcium or magnesium) or any other polyvalent cation (e.g., iron, zinc, barium, cesium or the like). By way of example, an oligonucleotide anionic drug (e.g., alicaforsen) is rendered insoluble or less soluble in biological and/or aqueous media by formation of a calcium salt thereof. By way of example, a protein (e.g., insulin) is rendered insoluble or less soluble in biological and/or aqueous fluids by formation of a zinc salt thereof.

By way of example, for cationic drugs (e.g., active agents containing primary, secondary, or tertiary aliphatic or aromatic amines), a soluble drug is rendered insoluble or less soluble in biological and/or aqueous fluids by formulating at or above the pKa of at least one of the amine moieties. By way of example, for a pKa of ~ 5 for an amine moiety in a drug, a formulation at a pH >5 reduces the solubility of the drug in biological and/or aqueous fluids. By way of example, meclizine is insoluble in water with two amine groups (pKa of ~ 5 and 9), however it is readily solubilized in a poloxamer formulation when the pH of the solution is maintained below a pH of 5.5, and it is insoluble in a poloxamer formulation above a pH of 6. By way of example, when an active agent is a cationic drug (e.g., an agent bearing at least one amine moiety with a pKa~5), a poloxamer gel formulation at a pH of 4.5 has a lower mean dissolution time (MDT) compared to a poloxamer formulation at a pH of 7.4.

In addition, modifying the solubility of the active agent can also have an effect on the properties of the thermosensitive gel. By way of example, amitriptyline is water soluble (greater than 100 mg/mL) and increases the gelation temperature of a poloxamer formulation. Reducing the solubility of amitriptyline (e.g., by formation of a prodrug) allows for tuning of the gelation temperature of a poloxamer formulation.

Further, cationic drugs (e.g., drugs with one or more amine moieties) are rendered insoluble or less soluble in biological and/or aqueous media by exchanging the salt of such a drug from a mineral acid salt (e.g., hydrochloric acid or sulfuric acid salts) to a salt of a small to medium sized organic acid (e.g., a citrate, maleate, nicotinate, or besylate salt or the like). By way of example, dexamethasone acetate is less soluble than dexamethasone hydrochloride in biological and/or aqueous fluids. By way of example, a water soluble active agent has a solubility of ≥ 10 mg/ mL. An active agent that has been rendered less soluble or insoluble in aqueous and/or biological media has a water solubility of less than 10 mg/mL, less than 1mg/mL or less than 0.1mg/mL. The release profile of an active agent and/or any salts thereof is compared using in vitro and in vivo procedures described herein.

A second approach for controlling the dissolution and/or release profile of an active agent is to form a complex of an active agent with a complexation agent that hinders dissolution of the active agent in biological and/or aqueous media. Examples of such complexation agents include and are not limited to cryptands (e.g., [2.2.2]cryptand, diaza-18-crown-6), cyclodextrins, crown ethers (e.g., 12-crown-4, 15-crown-5, 18-crown-6, dibenzo-18-crown-6 or the like), or the like. In further instances, by way of example, anionic active agents, cationic (e.g., amine based) active agents and zwitterionic active agents are rendered insoluble or less soluble in biological and/or aqueous media by complexation with polymers (e.g., hyaluronic acid), insoluble organic compounds (e.g., surfactants such as phospholipids), or polyvalent metal ions (e.g., multimeric complexes with cesium, calcium, magnesium, iron, zinc, or the like). By way of example, complex coacervation of proteins (e.g., insulin) with bovine serum albumin (BSA) or gelatin modifies the dissolution and/or release profile of a protein from a formulation described herein.

By way of example, the amount of a systemically administered (e.g., oral or intravenously administered) bisphosphonate reaching the perilymph in the inner ear and/or otic bone structure is about 0.6 ng/day for rosidronate, and about 0.1 ng/day for zoledronic acid and alters cochlear function. The formulations described herein deliver a more therapeutically effective amount of a bisphosphonate to the perilymph and/or active capsule compared to a bisphosphonate that is administered via a systemic route thus reducing toxicity of bisphosphonates. By way of example, an extended release formulation of zoledronate (e.g., a formulation comprising a complex of zoledronate with calcium ions) releases a therapeutically effective amount of zoledronate reducing any toxicity caused by higher amounts of zoledronate that could alter cochlear function (e.g., by calcium depletion in the delivery site). **Figure 6** illustrates a comparison of the in vitro mean dissolution time (MDT) for a 16% P407 formulation comprising zoledronate versus a 16% P407 formulation comprising a complex of zoledronate with calcium. Complexation with calcium increases the mean dissolution time of zoledronate from 2 hours to 8 hours.

Yet another approach to tune the release profile of an active agent from a formulation described herein is to complex a salt or free base of an active agent with a polyelectrolyte (e.g., poly(sodium styrene sulfonate), polyacrylic acid, polyamines or the like). The ionic interactions between the polyelectrolyte and the salt or free base of the active agent modify the dissolution characteristics of the active agent in biological and/or aqueous fluids. By way of example, solubility of genetic material in biological and/or aqueous media is modified by addition of cationic polymers and/or formation of cationic micelles. The release profile of an active agent and a complex thereof is compared using in vitro and in vivo procedures described herein.

A further approach to extend the release profile of an active agent from a formulation described herein is to use prodrugs of an active agent. An active agent (anionic, cationic, zwitterionic or neutral) is rendered insoluble or less soluble in biological and/or aqueous media by formation of a prodrug that is insoluble or less soluble in biological and/or aqueous media than the drug alone. Such prodrugs are formed by covalent attachment of a moiety (e.g., an ester, or amide of a bulky or water insoluble group such as benzoic acid, amines, fatty acids, cyclic or aromatic acids or alcohols,

polymeric chains, or the like) to the parent drug. The release profile of an active agent and a prodrug thereof is compared using in vitro and in vivo procedures described herein.

A further approach to tuning the dissolution properties and/or release profile of an active agent is to coat particles of the active agent with certain sustained release excipients (e.g., hydroxypropylmethyl cellulose, carboxymethylcellulose or the like). By way of example, an active agent is micronized and the micronized particles are coated with sustained release excipients; the coated active agent particulates are then formulated in any of the compositions described herein.

**Active agent concentration**

The release profile of an active agent is tuned by changing the concentration of an active agent in the formulation. By way of example, at increased concentration of an active agent, a) initial drug levels reached in the inner ear (as measured in perilymph) are high and b) there is an increase in the duration of exposure. **Figure 3** illustrates a dose proportionality effect of the drug when formulated in a poloxamer gel. **Figure 4** illustrates the dose proportionality effect in vitro in a release kinetic assay in which increasing the drug concentration is associated with an increase in the mean dissolution time. An increase in active agent concentration in the formulation prolongs residence time and/or MDT of the active agent in the ear.

In some embodiments, the MDT for an active agent from a formulation described herein is from about 30 hours to about 48 hours. In some embodiments, the MDT for an active agent from a formulation described herein is from about 30 hours to about 96 hours. A linear relationship between the formulations mean dissolution time (MDT) and the P407 (also known as PF-127, Pol-407, Pluronic-127) concentration indicates that the active agent is released due to the erosion of the polymer gel (poloxamer) and not via diffusion. A non-linear relationship indicates release of active agent via a combination of diffusion and/or polymer gel degradation.

The MDT is inversely proportional to the release rate of an active agent from a composition described herein. Experimentally, the released active agent is optionally fitted to the Korsmeyer-Peppas equation:

$$\frac{Q}{Q_\alpha} = kt^n + b$$

where Q is the amount of active agent released at time t, $Q_\alpha$ is the overall released amount of active agent, k is a release constant of the nth order, n is a dimensionless number related to the dissolution mechanism and b is the axis intercept, characterizing the initial burst release mechanism wherein n=1 characterizes an erosion controlled mechanism. The mean dissolution time (MDT) is the sum of different periods of time the drug molecules stay in the matrix before release, divided by the total number of molecules and is optionally calculated by:

$$MDT = \frac{nk^{-1/n}}{n+1}$$

In some embodiments, the MDT for an active agent from a formulation described herein is from about 30 hours to about 1 week. In some embodiments, the MDT for a formulation described herein is from about 1 week to about 6 weeks.

In some embodiments, the mean residence time (MRT) for an active agent in a formulation described herein is from about 20 hours to about 48 hours. In some embodiments, the MRT for an active agent from a formulation described herein is from about 20 hours to about 96 hours. In some embodiments, the MRT for an active agent from a formulation described herein is from about 20 hours to about 1 week. In some embodiments, the MRT for an active agent from a formulation described herein is from about 1 week to about 6 weeks.

In some embodiments, middle or external ear formulations described herein allow for maintenance of therapeutic levels of active agent in dry ear conditions or wet ear conditions. By way of example, a formulation described herein comprising ciprofloxacin, about 40-60% of a thermoreversible polymer, a buffer and an additional solvent such as ethanol provides a sustained release of ciprofloxacin for at least 7 days and the drug levels detected in middle ear fluids are about the same as or higher than the minimum inhibitory concentration (MIC), i.e., such a formulation provides ciprofloxacin concentrations of > 1 $\mu$g/mL in the middle ear fluids (MEF) for at least 7 days. In an other example, a formulation comprising dexamethasone, about 40-60% of a thermoreversible polymer, a buffer and an additional solvent such as ethanol provides a sustained release of dexamethasone for at least 7 days and the drug levels detected in middle ear fluids are > 1-40 mcg/mL for at least 7 days. **Figure 15-19** illustrate the sustained release profiles of otic agents from formulations described herein when the formulations are administered in the middle ear. **Figure 20** illustrates the sustained release characteristics of the formulations described herein when compared with release characteristics of Ciprodex®. **Figure 21** is a comparison of therapeutic efficacy for an otic solution (Ciprodex®) and formulations described herein and illustrates the the minimal hearing shifts that occur upon administration of gel formulations described herein.

**Gel strength**

The gel strength and concentration of the active agent affects release kinetics (e.g., mean dissolution time) of an active agent from the composition. For example, at low poloxamer concentration, elimination rate is accelerated (Mean Dissolution time (MDT) is lower). **Figure 5** illustrates in vitro mean dissolution time of high versus low solubility drug substances and solution versus gel formulations.

In some embodiments, the MDT for poloxamer from a formulation described herein is at least 6 hours. In some embodiments, the MDT for poloxamer from a formulation described herein is at least 10 hours. In some embodiments, the MDT for poloxamer from a formulation described herein is at least 24, 48, 60, 100, 150, 200 or 250 hours. The MDT is determined using techniques described herein in, for example, Example 6. **Figure 7** illustrates the MDT for certain formulations.

In some embodiments, the Mean Residence Time (MRT) of an active agent in the perilymph for any formulation described herein is between about 5, 7, 10, 15, 20, 24, 36, 48, 60, 70 or 80 hours and about 100, 200, 300, 400, 500 or 600 hours. **Figure 8** illustrates the MRT for dexamethasone (Dex), dexamethasone sodium phosphate (DSP), and dexamethasone acetate (DA) from certain formulations following intratympanic injection in guinea pigs. **Figure 9** illustrates the MRT for soluble form or methylprednisolone (MPS) and insoluble form of methylprednisolone (MP) from certain formulations following intratympanic injection in guinea pigs. **Figure 10** illustrates the MRT for 0.6% L-701324 in 17% poloxamer 407 formulation following intratympanic injection in guinea pigs. **Figure 11** illustrates the MRT for 0.5% SP-600125 in 17% poloxamer 407 formulation following intratympanic injection in guinea pigs. **Figure 12** illustrates the MRT for 2% meclizine in 17% poloxamer 407 formulation following intratympanic injection in guinea pigs.

In some embodiments, a composition described herein is a solution of microparticulates or micronized active agent and is substantially free of thermosensitive polymer components. In some of such embodiments, the composition provides essentially immediate release of an active agent. In other embodiments, a suspension of microparticulates or micronized active agent that is substantially free of thermosensitive polymer components provides intermediate sustained release of active agent. In certain other embodiments, a formulation comprising microparticulates or micronized active agent and a thermosensitive polymer provides an extended sustained release of active agent. As used herein, immediate release of an active agent refers to substantially complete release of an active agent from the formulation in less than about 5 hours. As used herein, sustained release refers to extended release of an active agent from a formulation such as, for example, a sustained release of active agent over at least 2, 3, 5, 7, 14, 21, 28 days, or at least 1, 2, 3, 4, 5 or 6 months or 1 year.

In some embodiments, pharmaceutical formulations provided herein provide sustained release of an active agent (e.g., a corticosteroid, an antibiotic) for a period of at least 1 day. In some embodiments, pharmaceutical formulations provided herein provide sustained release of an active agent (e.g., a corticosteroid, an antibiotic) for a period of at least 2 days. In some embodiments, pharmaceutical formulations provided herein provide sustained release of an active agent (e.g., a corticosteroid, an antibiotic) for a period of at least 2 days. In some embodiments, pharmaceutical formulations provided herein provide sustained release of an active agent (e.g., a corticosteroid, an antibiotic) for a period of at least 3 days. In some embodiments, pharmaceutical formulations provided herein provide sustained release of an active agent (e.g., a corticosteroid, an antibiotic) for a period of at least 4 days. In some embodiments, pharmaceutical formulations provided herein provide sustained release of an active agent (e.g., a corticosteroid, an antibiotic) for a period of at least 5 days. In some embodiments, pharmaceutical formulations provided herein provide sustained release of an active agent (e.g., a corticosteroid, an antibiotic) for a period of at least 6 days. In some embodiments, pharmaceutical formulations provided herein provide sustained release of an active agent (e.g., a corticosteroid, an antibiotic) for a period of at least 7 days. In some embodiments, pharmaceutical formulations provided herein provide sustained release of an active agent (e.g., a corticosteroid, an antibiotic) for a period of at least 8 days. In some embodiments, pharmaceutical formulations provided herein provide sustained release of an active agent (e.g., a corticosteroid, an antibiotic) for a period of at least 9 days. In some embodiments, pharmaceutical formulations provided herein provide sustained release of an active agent (e.g., a corticosteroid, an antibiotic) for a period of at least 10 days. In some embodiments, pharmaceutical formulations provided herein provide sustained release of an active agent (e.g., a corticosteroid, an antibiotic) for a period of at least 2 weeks. In some embodiments, pharmaceutical formulations provided herein provide sustained release of an active agent (e.g., a corticosteroid, an antibiotic) for a period of at least 3 weeks. In some embodiments, pharmaceutical formulations provided herein provide sustained release of an active agent (e.g., a corticosteroid, an antibiotic) for a period of at least 4 weeks. In some embodiments, pharmaceutical formulations provided herein provide sustained release of an active agent (e.g., a corticosteroid, an antibiotic) for a period of at least 5 weeks. In some embodiments, pharmaceutical formulations provided herein provide sustained release of an active agent (e.g., a corticosteroid, an antibiotic)

for a period of at least 7 days. In some embodiments, pharmaceutical formulations provided herein provide sustained release of an active agent (e.g., a corticosteroid, an antibiotic) for a period of at least 6 weeks. In some embodiments, pharmaceutical formulations provided herein provide sustained release of an active agent (e.g., a corticosteroid, an antibiotic) for a period of at least 7 weeks. In some embodiments, pharmaceutical formulations provided herein provide sustained release of an active agent (e.g., a corticosteroid, an antibiotic) for a period of at least 8 weeks. In some embodiments, pharmaceutical formulations provided herein provide sustained release of an active agent (e.g., a corticosteroid, an antibiotic) for a period of at least 3 months. In some embodiments, pharmaceutical formulations provided herein provide sustained release of an active agent (e.g., a corticosteroid, an antibiotic) for a period of at least 4 months. In some embodiments, pharmaceutical formulations provided herein provide sustained release of an active agent (e.g., a corticosteroid, an antibiotic) for a period of at least 5 months. In some embodiments, pharmaceutical formulations provided herein provide sustained release of an active agent (e.g., a corticosteroid, an antibiotic) for a period of at least 6 months. In some embodiments, pharmaceutical formulations provided herein provide sustained release of an active agent (e.g., a corticosteroid, an antibiotic) for a period of at least 7 months. In some embodiments, pharmaceutical formulations provided herein provide sustained release of an active agent (e.g., a corticosteroid, an antibiotic) for a period of at least 8 months. In some embodiments, pharmaceutical formulations provided herein provide sustained release of an active agent (e.g., a corticosteroid, an antibiotic) for a period of at least 10 months. In some embodiments, pharmaceutical formulations provided herein provide sustained release of an active agent (e.g., a corticosteroid, an antibiotic) for a period of at least 12 months.

The release profile can also be modified by the formation of cocrystals (norfloxacin is known to form cocrystal , Crystal Growth & Design, Vol. 6, No. 12, 2006 Basavoju et al.,) As an example ciprofloxacin free base forms cocrystals with dexamethasone that will modify the release profile of the cocrystals. The MDT of ciprofloxacin (Cipro)/ dexamethasone (Dex) or dexamethasone phosphate (DSP) suspensions in 16% P407 are manipulated by the formation of cocrystal or inclusion-crystal as seen in the table below

| Sample | MDT Cipro | MDT Dex | Crystal Structure |
|---|---|---|---|
| 2%Cipro/0.67% Dex in 16% P407 | 187 | 83 | Needles |
| 2%Cipro/0.67% Dex in 16% P407 | 93 | 37 | Plates |
| 2%Cipro in 16% P407 | 187 | - | Needles |
| 0.6% Dex in 16% P407 | - | 17 | |
| 2%Cipro/0.67% DSP in 16% P407 | 169 | 11 | Needles |
| 0.67% DSP in 16% P407 | - | Less than 3 | |

The release profile of an active agent from a solution or suspension or gel formulation is tunable as described above. Accordingly, in certain embodiments, a suspension of microparticulates or micronized active agent provides intermediate sustained release or extended sustained release. In certain embodiments, a composition comprising a thermosensitive polymer and microparticulate or micronized active agent provides intermediate sustained release or extended sustained release. In certain embodiments, a solution of an active agent provides immediate release or intermediate sustained release.

**Biodistribution**

In some embodiments, in vivo distribution of drugs from formulations described herein is governed by passive diffusion. In some embodiments, a formulation comprising a thermosensitive gel described herein advantageously allows for substantially uniform distribution of an active agent and reduces variability in drug exposure in vivo. For example, solutions of dexamethasone that do not contain thermosensitive polymers provide uneven distribution (large gradient) of active agents in the cochlea. **Figure 13** illustrates a substantially uniform distribution of dexamethasone in the chochlea upon administration of a formulation comprising a thermosensitive polymer and the uneven distribution of dexamethasone in the cochlea upon administration of a dexamethasone solution not containing a thermosensitive polymer-following intratympanic injection.

**Pharmacokinetics**

The pharmacokinetic profile of active agents released from formulations described herein is dependent on the nature of the vehicle (for example, aqueous solution comprising a thermosensitive polymer versus aqueous solution

that does not contain a thermosensitive polymer). In addition, the pharmacokinetic profile of active agents also depends on the physicochemical properties of the active agent as described above. Thus, a combination of an appropriate thermosensitive polymer vehicle and physicochemical properties of a drug provides an optimized release profile. By way of example, for a 17% Poloxamer 407 formulation, when either dexamethasone or methylprednisolone is present as a water soluble salt, i.e. DSP and MPS, respectively, MDT values are about 3h. However, the MDT values of water insoluble forms of dexamethasone and methylprednisolone (e.g., DEX, DA and MP) range from 40 to 71 h. By way of example, a DSP aqueous solution has a MDT of 0.3h whereas a micronized DEX suspension in water has a MDT value of 44h.

In some embodiments, the solubility of the drug modulates the pharmacokinetics regardless of the vehicle used in the formulation. By way of example, intratympanic administration of DSP in either an aqueous or hydrogel vehicle in guinea pigs resulted in limited inner ear exposure (AUC values ranging from 28 to 57 μg.h/ml) and rapid elimination from inner ear compartment (MRT of 4-7 h). However, administration of a less soluble form of the drug, i.e., DEX or DA in either aqueous or hydrogel vehicle led to higher dexamethasone exposure in the perilymph (AUC of 84-359 μg.h/ml) and prolonged residence time (MRT 17-82 h).

By way of example, the inner ear profile of methylprednisolone is tunable via the use of soluble (MPS) and water insoluble (MP) forms. Methylprednisolone levels in the perilymph peaked rapidly following intratympanic administration of the MPS hydrogel in guinea pigs at 6.5 μg/ml and decreased to a fraction of the peak levels (0.8-1.0 %) within 3 days. In contrast, administration of a formulation comprising the less soluble MP resulted in higher peak levels (19.2 μg/ml) that decreased slowly over 10 days.

Thus, in certain embodiments, the nature and the composition of the vehicle and the degree of aqueous solubility of the drug present in the formulation affects pharmacokinetic parameters such as the mean residence time and/or exposure in the target area.

In certain instances, once drug exposure (e.g., concentration in perilymph, sinonasal fluid, spinal fluid or the like) of a drug reaches steady state, the concentration of the drug stays at or about the therapeutic dose for an extended period of time (e.g., one day, 2 days, 3 days, 4 days, 5 days, 6 days, or 1 week, 3 weeks, 6 weeks, 2 months). In some embodiments, the steady state concentration of active agent released from a sustained release formulation described herein is about 5 to about 20 times the steady state concentration of an active agent released from a formulation that is not a sustained release formulation. In some embodiments, the steady state concentration of active agent released from a sustained release formulation described herein is about 20 to about 50 times the steady state concentration of an active agent released from a formulation that is not a sustained release formulation.

In specific embodiments, any formulation described herein provides extended release of an active agent for at least 7 days, at least 10 days, at least 2 weeks, at least 4 weeks, at least 6 weeks, at least 8 weeks, at least 12 weeks, or at least 16 weeks..

## Pharmaceutical Formulations

Provided herein are pharmaceutical formulations that include at least one active agent and a pharmaceutically acceptable diluent(s), excipient(s), or carrier(s).

## Thermosensitive Gels

Polymers composed of polyoxypropylene and polyoxyethylene form thermosensitive gels when incorporated into aqueous solutions. These polymers have the ability to change from the liquid state to the gel state at temperatures close to body temperture, therefore allowing useful formulations that are applied to the targeted structure(s). The liquid state-to-gel state phase transition (gelation temperature) is dependent on the polymer concentration, buffer concentration and the ingredients in the solution. In some embodiments, a thermosensitive gel suitable for compositions described herein is an aqueous gel comprising of a polymer of polyoxypropylene and polyoxyethylene.

Poloxamer is a synthetic block polymer of ethylene oxide and propylene oxide. Poloxamer 407 (PF-127, P407) is a theroreversible polymer composed of polyoxyethylene-polyoxypropylene copolymers. Other poloxamers include 124, 188 (F-68 grade), 237 (F-87 grade), and 338 (F-108 grade). Aqueous solutions of poloxamers are stable in the presence of acids, alkalis, and metal ions. PF-127 (or P407) is a commercially available polyoxyethylene-polyoxypropylene triblock copolymer, with an average molar mass of 13,000. The polymer can be further purified by suitable methods that will enhance gelation properties of the polymer. It contains approximately 70% ethylene oxide, which accounts for its hydrophilicity. It is one of the series of poloxamer ABA block copolymers, whose members share the chemical formula shown below.

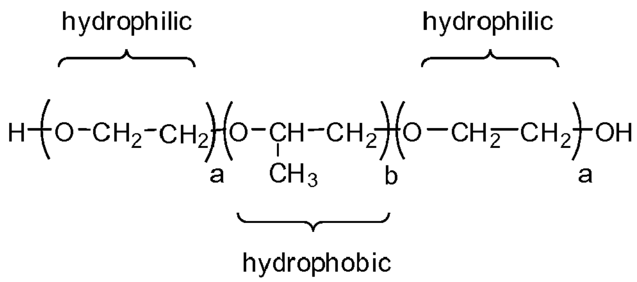


Poloxamers are available in several types, and with varying molecular weights ranging from about 2000 to about 15000. The α-hydro-ω-hydroxypoly(oxyethylene)$_a$ poly(oxypropylene)$_b$ poly(oxyethylene)$_a$ block copolymers comprise varying ratios of a and b as shown below:

| poloxamer | a | b |
|---|---|---|
| 124 | 12 | 20 |
| 188 | 80 | 27 |
| 237 | 64 | 37 |
| 338 | 141 | 44 |
| 407 | 101 | 56 |

In certain embodiments, a thermosensitive gel formulation described herein comprises a poloxamer. In specific embodiments, a thermosensitive gel formulation described herein comprises P407. When placed in contact with the body, such a gel preparation will form a semi-solid structure and a sustained release depot. Furthermore, poloxamers (e.g., P407) have good solubilizing capacity, low toxicity, and are biocompatible.

In an alternative embodiment, the thermosensitive gel comprises a PEG-PLGA-PEG triblock copolymer (Jeong etal, Nature (1997), 388:860-2; Jeong etal, J. Control. Release (2000), 63:155-63; Jeong etal, Adv. Drug Delivery Rev. (2002), 54:37-51). The polymer exhibits sol-gel behavior over a concentration of about 5% w/w to about 40% w/w. Depending on the properties desired, the lactide/glycolide molar ratio in the PLGA copolymer ranges from about 1:1 to about 20:1. The resulting coploymers are soluble in water and form a free-flowing liquid at room temperature, but form a gel at body temperature.

ReGel® is a tradename of MacroMed Incorporated for a class of low molecular weight, biodegradable block copolymers having reverse thermal gelation properties as described in U.S. Pat. Nos. 6,004,573, 6,117949, 6,201,072, and 6,287,588. It also includes biodegradable polymeric drug carriers disclosed in pending U.S. patent application Ser. Nos. 09/906,041, 09/559,799 and 10/919,603. The biodegradable drug carrier comprises ABA-type or BAB-type triblock copolymers or mixtures thereof, wherein the A-blocks are relatively hydrophobic and comprise biodegradable polyesters or poly(orthoester)s, and the B-blocks are relatively hydrophilic and comprise polyethylene glycol (PEG), said copolymers having a hydrophobic content of between 50.1 to 83% by weight and a hydrophilic content of between 17 to 49.9% by weight, and an overall block copolymer molecular weight of between 2000 and 8000 Daltons.

In some embodiments, other thermosensitive polymers are useful depending upon the particular active agent, other pharmaceutical agent or excipients/additives used, and as such are considered to fall within the scope of the present disclosure. For example, other commercially-available glycerin-based gels, glycerin-derived compounds, conjugated, or crosslinked gels, matrices, hydrogels, and polymers, as well as gelatins and their derivatives, alginates, and alginate-based gels, and even various native and synthetic hydrogel and hydrogel-derived compounds are all expected to be useful in the pharmaceutical formulations described herein. In some embodiments, bioacceptable gels include, but are not limited to, alginate hydrogels SAF®-Gel (ConvaTec, Princeton, N.J.), Duoderm® Hydroactive Gel (ConvaTec), Nu-gel ®(Johnson & Johnson Medical, Arlington, Tex.); Carrasyn®(V) Acemannan Hydrogel (Carrington Laboratories, Inc., Irving, Tex.); glycerin gels Elta® Hydrogel (Swiss-American Products, Inc., Dallas, Tex.), K-Y® Sterile (Johnson & Johnson), gelatin hydrogels, chitosan, silicon-base gels (e.g., Medgel®) or the like. Other thermosensitive and/or bioacceptable gels suitable for compositions described herein include acrylic acid-based polymers (e.g., Carbopol®), cellulose based polymers (e.g., hydroxypropylmethyl cellulose, carboxymethyl cellulose, or the like), alkyl aryl polyether alcohol-based polymer (e.g., Tyloxapol®), or the like.

**Purification poly(oxyethylene)/poly(oxypropylene) triblock polymers**

In some embodiments, any active composition described herein comprises purified thermosensitive polymer. In some embodiments, any active composition described herein comprises fractionated a purified thermosensitive polymer composed of polyoxyethylene-polyoxypropylene copolymers. In some of such embodiments, the thermosensitive polymer is a poloxamer.

The purification of poloxamers is based on the removal of low molecular weight components (e.g., oligomers, unreacted material and/or other unwanted impurities that are produced during manufacturing or storage) and/or large molecular weight components (components from unwanted polymer-polymer reactions). The resulting purified product has a narrower PDI with approximately the same molecular weight as the original material. In some embodiments, a purified poloxamer has better gelling characteristics (e.g., a lower Tgel for the same % poloxamer concentration while providing a higher viscosity in the gel state).

As used herein, a purified thermosensitive polymer has low polydispersity (i.e., a narrow distribution of molecular weights amongst the individual polymer chains therein). For example, commercially available poloxamers contain certain impurities such as poly(oxyethylene) homopolymer and poly(oxyethylene)/poly(oxypropylene) diblock polymers due to the nature of the manner in which they are produced. The relative amounts of these byproducts increase as the molecular weights of the component blocks increase. In some instances, in commercially available poloxamer 407, byproducts may constitute from about 15 to about 50% by weight of the polymer depending upon the manufacturer, thereby resulting in high polydispersity. Example 15 illustrates a procedure for fractionation of P407 that reduces polydispersity in commercially available P407.

In some embodiments, super critical fluid extraction technique is used to fractionate polyoxyalkylene block copolymers. *See*, U.S. Pat. No. 5,567,859, the disclosure for fractionation of polymers described therein is incorporated herein by reference. In this technique, lower molecular weight fractions in commercially purchased polymer are removed in a stream of $CO_2$ maintained at a pressure of 2200 pounds per square inch (psi) and a temperature of 40 °C, thereby providing purified polymer having low polydispersity.

**.** In some embodiments, gel permeation chromoatography allows for isolation of fractions of polymers. *See*, European Patent Application WO 92/16484; the use of gel permeation chromatography to isolate a fraction of poloxamer having low polydispersity and saturation described therein is incorporated herein by reference.

In some embodiments, one or more of the blocks is purified prior to manufacture of the copolymer. By way of example, purifying either the polyoxypropylene center block during synthesis of the copolymer, or the copolymer product itself (*See*, U.S. Pat. Nos. 5,523,492, and 5,696,298, incorporated herein by reference for such disclosure) allows for manufacture of purified poloxamers.

In some embodiments, fractionation of polyoxyalkylene block copolymers is acheived by batchwise removal of low molecular weight species using a salt extraction and liquid phase separation technique (*See*, U.S. Pat. No. 5,800,711, which process of purification of polymers described therein is incorporated herein by reference). Such fractionation produces polyoxyalkylene block copolymers (e.g., poloxamer 407, poloxamaer 188 or the like) having improved physical characteristics including increased gel strength, decreased polydispersity, higher average molecular weight, decreased gelling concentration and/or extended gel dissolution profiles compared to commercially available poloxamers (e.g., P407 NF grade from BASF). Other processes for purification and/or fractionation of polymers are described in, for example, US 6,977,045 and US 6,761,824 which processes are incorporated herein by reference.

In some instances, low molecular weight contaminants of polymers (e.g., poloxamers) cause deleterious side effects in vivo; the use of purified poloxamers in pharmaceutical formulations described herein reduces such in vivo side effects.

Accordingly, also contemplated within the scope of embodiments presented herein are formulations comprising purified poly(oxyethylene)/poly(oxypropylene) triblock polymers that are substantially free of the poly(oxyethylene) homopolymers and/or poly(oxypropylene)/poly(oxyethylene) diblock byproducts, thereby narrowing the molecular weight distribution of block copolymers, (i.e., providing low polydispersity). In some embodiments, such purified poly(oxyethylene)/poly(oxypropylene) triblock polymers (e.g., fractionated poloxamers) allow for formulation of active compositions that comprise lower concentrations of the poly(oxyethylene)/poly(oxypropylene) triblock polymers compared to active compositions that comprise non-fractionated poly(oxyethylene)/poly(oxypropylene) triblock polymers.

Advantageously, such compositions comprising lower concentrations of fractionated poly(oxyethylene)/poly(oxypropylene) triblock polymers (e.g., poloxamers) retain gelation properties (e.g., gelation between about 15 °C and about 42 °C) and sustained release characteristics (e.g., sustained release of dexamethasone over at least 3 days, 5 days or 7 days) despite having a lower concentration of the poly(oxyethylene)/poly(oxypropylene) triblock polymer (e.g., poloxamer).

Accordingly, by way of example, a formulation comprising micronized dexamethasone and lower concentrations of fractionated P407 (e.g., between about 5% to about 14% P407) has gelation properties and/or sustained release characteristics that are substantially the same or better than the gelation properties and/or sustained release characteristics of a formulation comprising micronized dexamethasone and non-fractionated P407 (e.g., between about 14.5% to about 25% of P407 NF from BASF).

**Gelation temperature modifying agents**

In some embodiments, pharmaceutical formulations described herein comprise gelation temperature modifying agents. A "gelation temperature modifying agent" or a "gel temperature modifying agent" is an additive added to any formulation described herein, and changes the gelation temperature of the formulation such that the gel temperature of the formulation is maintained between about 14 °C and about 42 °C. A gel temperature modifying agent increases or decreases the gelation temperature of any formulation described herein such that the formulation maintains a gelation temperature of between about 14 °C and about 42 °C.

In some embodiments, a gel temperature modifying agent is a gel temperature increasing agent. For example, where a formulation comprising a thermosensitive polymer has a gelation temperature below 14 °C, addition of a gel temperature increasing agent (e.g., P188, P388, cyclodextrin, carboxymethyl cellulose, hyaluronic acid, Carbopol®, Tween 20, Tween 40, Tween 60, Tween 80, Tween 81, Tween 85, n methyl pyrrolidone, short chain fatty acid salts (e.g., sodium oleate, sodium caprate, sodium caprylate or the like) increases the gelation temperature of the formulation to above 14 °C, to between about 14 °C and about 42 °C.

In some embodiments, a gel temperature modifying agent is a gel temperature decreasing agent. For example, where a formulation comprising a thermosensitive polymer has a gelation temperature above 42 °C, addition of a gel temperature decreasing agent (e.g., P188, P388, cyclodextrin, carboxymethyl cellulose, hyaluronic acid, Carbopol®, Tween 20, Tween 40, Tween 60, Tween 80, Tween 81, Tween 85, n methyl pyrrolidone, fatty acid salts (e.g., sodium oleate, sodium caprate, sodium caprylate or the like) decreases the gelation temperature of the formulation to below 42 °C, to between about 14 °C and about 42 °C.

In some embodiments, a gel temperature modifying agent is a pH sensitive polymer (e.g., chitosan). In some embodiments, a gel temperature modifiying agent is a thermosensitive polymer. In some embodiments, a gel temperature modifying agent is an ion-sensitive polymer (e.g., alginates gel in the presence of calcium ions). In some embodiments, a gel temperature modifying agent is an acrylic acid-based polymer (e.g., Carbopol®). In some embodiments, a gel temperature modifiying agent is a cellulose based polymer (e.g., hydroxypropylmethyl cellulose, carboxymethyl cellulose, or the like). In some embodiments, a gel temperature modifying agent is an alkyl aryl polyether alcohol-based polymer (e.g., Tyloxapol®).

In some embodiments, a gel temperature modifiying agent is a poloxamer. By way of example, addition of not more than about 5% poloxamer 188 to a formulation comprising about 16% P407 increases the gelation temperature of a 16% P407 formulation by about 5 °C.

**Gelation temperature**

In one embodiment, a pharmaceutical formulation described herein is a liquid at about room temperature. In certain embodiments, the pharmaceutical formulation is characterized by a phase transition between about room temperature and about body temperature (including an individual with a serious fever, e.g., up to about 42 °C). In some embodiments, the phase transition occurs between at least about 1 °C below body temperature and body temperature, between at least about 2 °C below body temperature and body temperature, between at least about 3 °C below body temperture and body temperature, between at least about 4 °C below body temperature and body temperature, between at least about 6 °C below body temperature and body temperature, between at least about 8 °C below body temperature and body temperature, between at least about 10 °C below body temperature and body temperature, between at least about 15 °C below body temperature and body temperature, or between at least about 20 °C below body temperature and body temperature.

In some embodiments, a formulation described herein has a gelation temperature of between about 5 °C, 10 °C, 14 °C, 15 °C, 16 °C, 17 °C, 18 °C, 19 °C, or 20 °C, and about 25 °C, 28 °C, 30 °C , 33 °C, 35 °C , 37 °C , 40 °C or 42 °C. In some embodiments, a formulation described herein has a gelation temperature of between about 5 °C and about 42 °C. In some embodiments, a formulation described herein has a gelation temperature of between about 10 °C and about 42 °C. In some embodiments, a formulation described herein has a gelation temperature of between about 14 °C and about 42 °C. In some embodiments, a formulation described herein has a gelation temperature of between about 14 °C and about 40 °C. In some embodiments, a formulation described herein has a gelation temperature of between about 14 °C and about 37 °C. In some embodiments, a formulation described herein has a gelation temperature of between about 14 °C and about 35 °C. In some embodiments, a formulation described herein has a gelation temperature of between about 16 °C and about 35 °C. In some embodiments, a formulation described herein has a gelation temperature of between about 18 °C and about 35 °C. In some embodiments, a formulation described herein has a gelation temperature of between about 20 °C and about 42 °C. In some embodiments, a formulation described herein has a gelation temperature

of between about 20 °C and about 37 °C. In some embodiments, a formulation described herein has a gelation temperature of between about 20 °C and about 35 °C. In some embodiments, a formulation described herein has a gelation temperature of between about 20 °C and about 30 °C. In some embodiments, a formulation described herein has a gelation temperature of between about 20 °C and about 28 °C. In some embodiments, a formulation described herein has a gelation temperature of between about 20 °C and about 25 °C.

Since the polymer systems of thermosensitive gels dissolve more completely at reduced temperatures, methods of solubilization include adding the required amount of polymer to the amount of water to be used at reduced tempertures. Generally after wetting the polymer by shaking, the mixture is capped and placed in a cold chamber or in a thermostatic container at about 0-10 °C in order to dissolve the polymer. In some embodiments, the dissolution is carried out a temperature between about 10 °C and about 20 °C. The mixture is stirred or shaken to bring about a more rapid dissolution of the thermosensitive polymer. In some instances the active agent and/or other pharmaceutically active agent is suspended if it is insoluble in water. The pH/osmolarity of the formulation is modulated by the addition of appropriate buffering agents.

**Viscosity**

In some embodiments, a formulation described herein contains a thermosensitive polymer sufficient to provide a viscosity of between about 10,000 and about 1,000,000 centipoise. In some embodiments, a formulation described herein contains a thermosensitive polymer sufficient to provide a viscosity of between about 50,000 and about 1,000,000 centipoise. In some embodiments, a formulation described herein contains a thermosensitive polymer sufficient to provide a viscosity of between about 150,000 and about 1,000,000 centipoise. In some embodiments, a formulation described herein contains a thermosetting polymer sufficient to provide a viscosity of between about 50,000 and about 600,000 centipoise. In some embodiments, a formulation described herein contains a thermosensitive polymer sufficient to provide a viscosity of between about 100,000 and about 500,000 centipoise. In some embodiments, a formulation described herein contains a thermosensitive polymer sufficient to provide a viscosity of between about 150,000 and about 400,000 centipoise. By way of example, a thermosensitive polymer concentration of about 15.5% in a composition described herein provides an apparent viscosity of about 270,000 cP. By way of example, a thermosensitive polymer concentration of about 16% in a composition described herein provides an apparent viscosity of about 360,000 cP. By way of example, a thermosensitive polymer concentration of about 17% in a composition described herein provides an apparent viscosity viscosity of about 480,000 cP.

In some embodiments, the formulations described herein are low viscosity formulations at body temperature. In some embodiments, a low viscosity formulation described herein provides an apparent viscosity of from about 100 cP to about 10,000 cP.

In some embodiments, a formulation described herein contains a viscosity enhancing polymer sufficient to provide a viscosity of between about 1,000 and about 1,000,000 centipoise at body temperature. In some embodiments, a formulation described herein contains a viscosity enhancing polymer sufficient to provide a viscosity of between about 1,000 and about 500,000 centipoise at body temperature. In some embodiments, a formulation described herein contains a viscosity enhancing polymer sufficient to provide a viscosity of between about 1,000 and about 250,000 centipoise at body temperature. In some embodiments, a formulation described herein contains a viscosity enhancing polymer sufficient to provide a viscosity of between about 1,000 and about 100,000 centipoise at body temperature.

In one embodiment, administration of any formulation described herein at about room temperature (e.g., between about 18 °C to about 28 °C) reduces or inhibits vertigo associated with intratympanic administration of cold (e.g., temperature below about 18 °C) otic formulations.

In some embodiments, use of a higher concentration of active agent results in formulations having higher viscosity compared to formulations have lower concentration of active agents. As shown in Example 15, increase in concentration of drug in the formulation, and use of purified poloxamer, allows for use of lower concentrations of thermosensitive polymer by weight of the formulation.

The viscosity is measured at a shear rate of 0.31 $s^{-1}$ using a cone/plate viscometer ( Brookfield DVII + Pro viscometer with a CP50 spindle at 0.08 rpm as a reference).

In some embodiments, a formulation described herein comprises between about 0.1%, 0.5%, 1%, 2%, 3%, 4%, 5%, 6%, 7%, 8%, 9%, 10%, 15%, 20%, 25%, 30%, 35%, 40%, 45%, 50%, or 55% and about 0.5%, 1%, 5%, 10%, 15%, 20% 25%, 30%, 35%, 40%, 45%, 50%, 55%, 60%, 65%, 70%, 75% 80% or 89% of a viscosity enhancing polymer. In some embodiments, a formulation described herein comprises between about 0.1% and about 50% of a viscosity enhancing polymer by weight of the composition. In some embodiments, a formulation described herein comprises between about 0.5% and about 30% of a viscosity enhancing polymer by weight of the composition. In some embodiments, a formulation described herein comprises between about 0.1% and about 20% of a viscosity enhancing polymer by weight of the composition. In some embodiments, a formulation described herein comprises between about 0.1% and about 10% of a viscosity enhancing polymer by weight of the composition. In some embodiments, a formulation described

herein comprises between about 0.1% and about 1% of a viscosity enhancing polymer by weight of the composition. In some embodiments, a formulation described herein comprises between about 0.1% and about 0.5% of a viscosity enhancing polymer by weight of the composition. In some embodiments, a formulation described herein comprises between about 1% and about 30% of a viscosity enhancing polymer by weight of the composition. In some embodiments, a formulation described herein comprises between about 1% and about 10% of a viscosity enhancing polymer by weight of the composition. In some embodiments, a formulation described herein comprises between about 10% and about 80% of a viscosity enhancing polymer by weight of the composition. In some embodiments, a formulation described herein comprises between about 10% and about 50% of a viscosity enhancing polymer by weight of the composition. In some embodiments, a formulation described herein comprises between about 10% and about 30% of a viscosity enhancing polymer by weight of the composition. In some embodiments, a formulation described herein comprises between about 20% and about 75% of a viscosity enhancing polymer by weight of the composition. In some embodiments, a formulation described herein comprises between about 20% and about 65% of a viscosity enhancing polymer by weight of the composition. In some embodiments, a formulation described herein comprises between about 20% and about 50% of a viscosity enhancing polymer by weight of the composition. In some embodiments, a formulation described herein comprises between about 25% and about 75% of a viscosity enhancing polymer by weight of the composition. In some embodiments, a formulation described herein comprises between about 15% and about 75% of a viscosity enhancing polymer by weight of the composition. In some embodiments, a formulation described herein comprises between about 30% and about 75% of a viscosity enhancing polymer by weight of the composition. In some embodiments, a formulation described herein comprises between about 35% and about 75% of a viscosity enhancing polymer by weight of the composition. In some embodiments, a formulation described herein comprises between about 40% and about 75% of a viscosity enhancing polymer by weight of the composition. In some embodiments, a formulation described herein comprises between about 45% and about 75% of a viscosity enhancing polymer by weight of the composition. In some embodiments, a formulation described herein comprises between about 45% and about 65% of a viscosity enhancing polymer by weight of the composition. In some embodiments, a formulation described herein comprises between about 40% and about 60% of a viscosity enhancing polymer by weight of the composition. In some of such embodiments, a viscosity enhancing polymer is a hydrogel, a thermoreversible polymer, an acrylic acid based polymer, a pH sensitive polymer, a polymer sensitive to concentration of ions (e.g., alginate gels in the presence of Calcium ions) and the like.

In some embodiments, a formulation described herein comprises between about 10%, 15%, 20%, 25%, 30%, 35%, 40%, 45%, 50%, or 55% and about 25%, 20%, 25%, 30%, 35%, 40%, 45%, 50%, 55%, 60%, 65%, 70%, 75% or 89% of a thermoreversible polymer. In some of such embodiments, a thermoreversible polymer is a poloxamer. In some embodiments, the resulting formulation is a thermoreversible gel, but it need not be thermoreversible; that is, depending on the amount of thermoreversible polymer in the composition, the resulting gel may be thermoreversible or not thermoreversible. The classification "thermoreversible polymer" refers to polymers that are capable of forming thermoreversible gels in the range 15-42 degrees Celsius. In some of such embodiments, the poloxamer is Pluronic-127 (PF-127, Pol-407). By way of example, a buffered poloxamer 407 solution comprising between about 15-25% of poloxamer exhibits thermoreversible gelation properties and degrades in an aqueous environment. By way of example, a buffered poloxamer 407 solution comprising between about 35% and about 80% of poloxamer by weight of the composition and an additional solvent such as ethanol exhibits substantially reduced thermoreversible gelation properties and is substantially stable in an aqueous environment. In some of such embodiments, a formulation comprising between about 35% and about 80% of poloxamer by weight of the composition, an alcohol (e.g. ethanol) and water exhibits high visocity (e.g., 5000-8000 cP) at about room temperature (e.g., about 25 °C) or about body temperature (e.g., about 37 °C - 42 °C, including individuals with a fever).

In some embodiments, a formulation described herein comprises between about 10% and about 80% of of PF-127 by weight of the composition. In some embodiments, a formulation described herein comprises between about 10% and about 75% of PF-127 by weight of the composition. In some embodiments, a formulation described herein comprises between about 15% and about 75% of PF-127 by weight of the composition. In some embodiments, a formulation described herein comprises between about 20% and about 75% of PF-127 by weight of the composition. In some embodiments, a formulation described herein comprises between about 25% and about 75% of a thermoreversible polymer of PF-127 by weight of the composition. In some embodiments, a formulation described herein comprises between about 30% and about 75% of a thermoreversible polymer of PF-127 by weight of the composition. In some embodiments, a formulation described herein comprises between about 35% and about 75% of PF-127 by weight of the composition. In some embodiments, a formulation described herein comprises between about 40% and about 75% of PF-127 by weight of the composition. In some embodiments, a formulation described herein comprises between about 45% and about 75% of PF-127 by weight of the composition. In some embodiments, a formulation described herein comprises between about 45% and about 65% of PF-127 by weight of the composition. In some embodiments, a formulation described herein comprises between about 40% and about 60% of PF-127 by weight of the composition.

## Buffers

In some embodiments, formulations described herein comprise buffers. In one embodiment is a buffer such as acetate or citrate buffer at slightly acidic pH. In one embodiment the buffer is a sodium acetate buffer having a pH of about 4.5 to about 6.5. In one embodiment the buffer is a sodium citrate buffer having a pH of about 5.0 to about 8.0, or about 5.5 to about 7.0.

In an alternative embodiment, the buffer used is tris(hydroxymethyl)aminomethane, bicarbonate, carbonate or phosphate at slightly basic pH. In one embodiment, the buffer is a sodium bicarbonate buffer having a pH of about 6.5 to about 8.5, or about 7.0 to about 8.0. In another embodiment the buffer is a sodium phosphate dibasic buffer having a pH of about 6.0 to about 9.0.

In some embodiments, the concentration of the buffer component is adjusted to bring the practial osmolarity of any formulation described herein within a biocompatible range.

## Solvents

In some embodiments, the release profile of a thickened formulation is modified by selection of an appropriate solvent or combination of solvents. In some embodiments in a formulation described herein, the solvent is water. In some embodiments, a formulation described herein comprises a mixture of solvents (e.g., a mixture of water and an additional solvent such as an alcohol, or the like). In some embodiments, a formulation described herein comprises additional solvents including and not limited to ethanol, propylene glycol, PEG 400, DMSO, N-methyl pyrrolidone or any other auris-suitable solvent. In some embodiments, the additional solvent is a water-miscible solvent. In some embodiments, following administration of a formulation comprising a mixture of solvents, the additional solvent diffuses out into the aqueous and/or biological fluids thereby thickening the composition. In some embodiments, an additional solvent comprises between about 5% to about 50%, between about 10% to about 40%, between about 10% to about 30%, or between about 10% to about 20% of the solvent present in a formulation described herein. By way of example, a formulation described herein in comprises water (including water present in the buffer solution) as the solvent and ethanol as an additional solvent. **Figure 15** shows a comparison of in vitro release profiles of otic agents in middle ear fluids from compositions comprising water and a mixture of water and ethanol as solvent. **Figure 16** shows a comparison of in vivo release profiles of otic agents in middle ear fluids from compositions comprising water and a mixture of water and ethanol as solvent.

In some embodiments, in a formulation described herein, the solvent is water. In some embodiments, a formulation described herein comprises a mixture of solvents (e.g., a mixture of water and an alcohol, or the like). In some embodiments, in a formulation described herein the solvent is a mixture of ethanol and water.

## Additional excipients

In some embodiments, a formulation described herein further comprises additional biocompatible excipients. Example of additional excipients include agents for imaging and/or visualization, penetration enhancers, including and not limited to alkyl saccharides (e.g., dodecyl maltoside, or the like), hyaluronic acid, (including and not limited to Hyalastine®, Hyalectin®, Hyaloftil®), and/or partial esters and/or salts thereof (e.g., barium salt of hyaluronic acid, or any other salt of hyaluronic acid described in WO/1998/017285, salts described therein are incorported herein by reference), hyaluronidase (e.g., PH-20 (Halzoyme)) or any other excipient that modulates release profile and/or stabilty and/or permeability and/or drug uptake and/or bioavailability and/or toxicity and/or immunogenicity and/or gelation characteristics of any formulation described herein. Additional excipients are described in U.S. Appl. Nos. 12/427,663, 12/466,310, 12/472,034, 12/486,697, 12/493,611, 12/494,156, 12/500,486, 12/504,553, 12/506,091, 12/506,127, 12/506,573, 12/506,616, and 12/506,664, the disclosure of excipients described therein is incorporated herein by reference.

## Dosing Methods

In some embodiments, formulations described herein are perfused in auditory and/or sinonasal structures. In some embodiments, formulations described herein are administered via needle or cannula or catheter in intrasinusoidal cavities or in the vicinity of sinuosoidal structures (e.g., nasal polyps, swollen turbinates), in intrathecal space, in synovial spaces, in the ear (e.g., via intratympanic injection, or at or near the round window membrane of the ear) or the like. In some embodiments, formulations described herein are administered as drops, paint, foam, in situ sponge or the like.

## Frequency of Administration

In some embodiments, a compositon disclosed herein is administered to an individual in need thereof once. In

some embodiments, a compositon disclosed herein is administered to an individual in need thereof more than once.

The number of times a composition is administered to an individual in need thereof depends on the discretion of a medical professional, the disorder, the severity of the disorder, and the individuals's response to the formulation. In some embodiments, a formulation described herein is administered as prophylactically, therapeutically or as a chronic treatment over an extended perior of time.

In the case wherein the patient's status does improve, upon the doctor's discretion the administration of the active agent compounds may be given continuously; alternatively, the dose of drug being administered may be temporarily reduced or temporarily suspended for a certain length of time (*i.e.*, a "drug holiday"). The length of the drug holiday varies between 2 days and 1 year, including by way of example only, 2 days, 3 days, 4 days, 5 days, 6 days, 7 days, 10 days, 12 days, 15 days, 20 days, 28 days, 35 days, 50 days, 70 days, 100 days, 120 days, 150 days, 180 days, 200 days, 250 days, 280 days, 300 days, 320 days, 350 days, and 365 days. The dose reduction during a drug holiday may be from 10%-100%, including by way of example only 10%, 15%, 20%, 25%, 30%, 35%, 40%, 45%, 50%, 55%, 60%, 65%, 70%, 75%, 80%, 85%, 90%, 95%, and 100%.

Once improvement of the patient's active conditions has occurred, a maintenance active agent dose is administered if necessary. Subsequently, the dosage or the frequency of administration, or both, is optionally reduced, as a function of the symptoms, to a level at which the improved disease, disorder or condition is retained. In certain embodiments, patients require intermittent treatment on a long-term basis upon any recurrence of symptoms.

**Kits/Articles of Manufacture**

In some embodiments, pharmaceutical formulations described herein are manufactured as ready to use single component solutions that are administered to an individual in need thereof. In other embodiments, pharmaceutical formulations described herein are manufactured as multi-component kits comprising dry-heat sterilized multiparticulate (e.g., micronized, nanoparticles, non-sized particles) active agent powder, a medium for reconstitution of the dry powder (e.g., sterile water or buffer or saline) and/or a solution comprising the thermosensitive polymer and a buffer. The dry powder is reconstituted with the sterile medium and/or the solution comprising the thermosensitive polymer and buffer just prior to administration of the pharmaceutical formulation to an individual in need thereof.

**EXAMPLES**

Example 1 - Preparation of a Thermosensitive Gel Dexamethasone Composition comprising micronized dexamethasone powder

Formulation A

| Ingredient | Quantity (mg/g of formulation) |
|---|---|
| dexamethasone | 20.0 |
| BHT | 0.002 |
| Poloxamer 407 | 160.0 |
| PBS buffer (0.1 M) | 9.0 |

Formulation B

| Ingredient | Quantity (mg/g of formulation) |
|---|---|
| dexamethasone | 20.0 |
| BHT | 0.002 |
| Purified Poloxamer 407 | 120.0 |
| PBS buffer (0.1 M) | 9.0 |

A 10-g batch of gel formulation containing 2.0% micronized dexamethasone is prepared. 13.8 mg of sodium phosphate dibasic dihydrate USP (Fisher Scientific.) + 3.1 mg of sodium phosphate monobasic monohydrate USP

(Fisher Scientific.) + 74 mg of sodium chloride USP (Fisher Scientific.) is dissolved with 8.2g of sterile filtered DI water and the pH is adjusted to 7.4 with 1 M NaOH. The buffer solution is chilled down and a suitable amount of poloxamer 407 (BASF Corp., containing approximately 100 ppm of BHT) or purified poloxamer (*See* Example 15 below) is sprinkled into the chilled PBS solution while mixing, the solution is mixed until all the poloxamer is dissolved. The poloxamer is sterile filtered using a 33mm PVDF 0.22μm sterile syringe filter (Millipore Corp.) and delivered to 2 mL sterile glass vials (Wheaton) in an aseptic environment, the vials are closed with sterile butyl rubber stoppers (Kimble) and crimped sealed with 13 mm Al seals (Kimble). 20 mg of micronized dexamethsone is placed in separate clean depyrogenated vials, the vials are closed with sterile butyl rubber stoppers (Kimble) and crimped sealed with 13 mm Al seals (Kimble), vials are dry heat sterilized (Fisher Scientific Isotemp oven) for 7 hours at 140°C. Before administration for the experiments described herein, 1 mL of the cold poloxamer solution is delivered to a vial containing 20 mg of sterile micronized dexamethasone using a 21G needle (Becton Dickinson) attached to a 1 mL sterile syringe (Becton Dickinson), suspension mixed well by shaking to ensure homogeneity of the suspension. The suspension is then withdrawn with the 21G syinge and the needle is switched to a 27 G needle for administration.

Examples 2-5 Formulations comprising amoxicillin, moxifloxacin, triamcinolone, and prednisolone respectively are prepared using the above procedure.

Example 6 - Preparation of a Thermosensitive Gel Zoledronate Composition

16% poloxamer 407 NF in 50mM TRIS buffer: Weigh 0.4518 g of sodium chloride (Fisher scientific) + 0.6034 g of tromethamine (Fisher scientific) dissolve with 82 g of DI water, then add 850 μL of 5 N HCl to adjust pH to 7.5, osmolality of solution is 277 mOsm/kg. Weigh 67.3 g of above buffer cool down buffer then sprinkle 12.8 g of poloxamer 407 NF (Spectrum chemicals) while mixing. Mix until a clear translucid solution is obtained. The solution is filter-sterilized using a 0.2μm sterilizing filter.

A 3.4 mM formulation of Zoledronic acid in 16% P407 in 50 mM TRIS buffer was prepared by dissolving 17.6 mg of zoledronic acid monohydrate (Betapharma) into 17g of a 16% poloxamer 407 in 50mM TRIS buffer, and the pH was adjusted to 7.3 with 5N NaOH.

A 3.4 mM formulation of Zoledronic acid complexed with Calcium in 16% P407 in 50 mM TRIS buffer was prepared by dissolving 17.6 mg of zoledronic acid monohydrate (Betapharma) into 17g of a 16% poloxamer 407 in 50mM TRIS buffer, and the pH was adjusted to 7.3 with 5N NaOH. Then 2 mg of calcium chloride dehydrate was added to 2 mL of the above solution, and the mixture was stirred until it was homogeneous.

Dissolution was performed at 37°C in snapwells (6.5 mm diameter polycarbonate membrane with a pore size of 0.4 μm). 0.2 mL of the formulation was placed into snapwell and left to harden, then 0.5 mL of 0.9% saline is placed into reservoir and shaken using a Labline orbit shaker at 70 rpm. Samples were taken every hour (0.1 mL withdrawn and replaced with warm buffer). Samples were analyzed for zoledronic acid concentration by UV at 215 nm using an Evolution 160 UV/Vis spectrophotometer (Thermo Scientific). Quantitation was performed against an external calibration standard.

| Sample | MDT (hr) |
|---|---|
| Zoledronic acid in 16% P407 | 2 |
| Zoledronic acid- Calcium in 16% P407 | 8 |

Example 7 - Preparation of a Thermosensitive Gel JNK inhibitor Composition

A formulation comprising 0.5% w/w SP600125 in 16% Poloxamer 407 was made by dispersing 5.3 mg of SP600125 (LC Labs) in 994.7 mg of a 16% P407 in 50mM Tris buffer. Solubility in the gel was measured to be ~ 190μg/mL. The reported water solubility of SP600125 is 11 μg/mL with a LogD (7.4) of 3.2 and a mp of 183°C.

Dissolution testing was performed as above. MDT for a 0.5% SP600125 in 16% poloxamer 407 gel was calculated to be 60 hours.

Example 8 - Preparation of a Thermosensitive Gel Vascular Suppressant Compositions comprising poloxamer 407 alone or in combination with poloxamer 188

2% Amitriptyline HCl in 16% Poloxamer 407: 102 mg of amitriptyline HCl (MP biomedicals) was QS to 5 g with a 16% poloxamer 407 in 50 mM TRIS buffer, pH of 6.8-; the mixture was stirred until amitriptyline was dissolved. Tgel measurements were performed using a Brookfield viscometer RVDV-II+P with a CP-51 spindle rotated at 0.08 rpm

(shear rate of 0.31 $s^{-1}$) equipped with a temperature control unit (temperature ramped from 15-37°C at 1.6 °C/min). Tgel was measured at 30.7°C

**Table 1:** Meclizine formulations in 16% poloxamer 407.

| % Meclizine di-HCl | mg Meclizine di-HCl | QS to with 16% P407 in TRIS | μL of 5 N NaOH | Final pH |
|---|---|---|---|---|
| 0.5 | 26 | 5.0 | 12.5 | 5.5 |
| 1 | 50 | 5.0 | 25 | 5.1 |
| 2 | 102 | 5.0 | 60 | 7.5 |

2% Meclizine di-HCl in 16% poloxamer 407/2% poloxamer 188 in TRIS buffer (final pH of 4.7): 100.5 g of meclizine di-HCl (MP biomedicals) was QS to 5.023 g with a 16% poloxamer 407/ 2% poloxamer 188 solution in 50 mM TRIS buffer. Twenty microliters of a 5 N NaOH was added to adjust pH to 4.7 mixed until clear, filtered through a 0.22μm PES syringe filter. Gelation temperature of this formulation was 26°C.

2% Meclizine di-HCl in 16% poloxamer 407/1% poloxamer 188 in TRIS buffer (final pH of 6.8). 102.5 g of meclizine di-HCl (MP biomedicals) was QS to 5.023 g with a 16% poloxamer 407/ 1% poloxamer 188 solution in 50 mM TRIS buffer. Take 1 mL of the above solution and add 7 μL of a 5 N NaOH was added to adjust pH to 6.8, the resulting suspension was mixed to ensure homogeneity. Gelation temperature of this formulation was 24°C.

**Table 2:** Mean dissolution time in 10mM PBS buffer pH 7.4

| Sample | MDT (hours) |
|---|---|
| 2% Amitriptyline in 16% P407 | 3 |
| 0.5% Meclizine in 16% Poloxamer 407 | 5 |
| 1% Meclizine in 16% Poloxamer 407 | 9 |
| 2% Meclizine in 16% Poloxamer 407 | 70 |

**Table 3:** Mean dissolution time in 0.9% sodium chloride (switched from 10mM PBS to eliminate the effect of buffer on release profile).

| Sample | MDT (hours) |
|---|---|
| 2% Meclizine in 16% Poloxamer 407/2% Poloxamer 188 (pH 4.7) | 3.5 |
| 2% Meclizine in 16% Poloxamer 407/1% Poloxamer 188 (pH 6.8) | >100 |

Example 9 - Preparation of Thermosensitive Gel Compositions comprising micronized ciprofloxacin hydrate powder or ciprofloxacin powder and micronized dexamethasone powder

The procedure in Example 1 is used to prepare the following formulations, formulations comprising a gel temperature modifying agent (Formulations A and C) and one comprising P407 alone (Formulation B).

Formulation A

| Ingredient | Quantity (mg/g of formulation) |
|---|---|
| Ciprofloxacin hydrate, micronized | 15.0 |
| dexamethasone | 15.0 |
| BHT | 0.002 |
| Poloxamer 407 | 180.0 |
| Poloxamer 188 | 20.0 |
| PBS buffer (0.1 M) | 9.0 |

Formulation B

| Ingredient | Quantity (mg/g of formulation) |
|---|---|
| ciprofloxacin | 15.0 |
| dexamethasone | 15.0 |
| BHT | 0.002 |
| Poloxamer 407 | 180.0 |
| PBS buffer (0.1 M) | 9.0 |

Formulation C

| Ingredient | Quantity (mg/g of formulation) |
|---|---|
| Ciprofloxacin | 15.0 |
| dexamethasone | 15.0 |
| BHT | 0.002 |
| Poloxamer 407 | 120.0 |
| Carboxymethylcellulose | 60.0 |
| PBS buffer (0.1 M) | 9.0 |

The gel temperature of Formulations A, B and C are compared. Formulation A is expected to gel at about body temperature, Formlation B is expected to gel at higher than body temperature, Formulation C is expected to gel at about body temperature. Thus P188 is expected to be a gel temperature lowering agent in Formulation A, and

Carboxymethylcellulose is expected to be a gel temperature increasing agent in Formulation C.

Example 10 Effect of pH on degradation products for autoclaved 17% poloxamer 407NF/ 2% active agent in PBS buffer

A stock solution of a 17% poloxamer 407/ 2% active agent is prepared by dissolving 351.4 mg of sodium chloride (Fisher Scientific), 302.1 mg of sodium phosphate dibasic anhydrous (Fisher Scientific), 122.1 mg of sodium phosphate monobasic anhydrous (Fisher Scientific) and an appropriate amount of an active agent with 79.3 g of sterile filtered DI water. The solution is cooled down in a ice chilled water bath and then 17.05 g of poloxamer 407NF (SPECTRUM CHEMICALS) is sprinkled into the cold solution while mixing. The mixture is further mixed until the poloxamer is completely dissolved. The pH for this solution is measured.

**17% poloxamer 407/ 2% active agent in PBS pH of 5.3.** Take an aliquot (approximately 30mL) of the above solution and adjust the pH to 5.3 by the addition of 1 M HCl.

**17% poloxamer 407/ 2% active agent in PBS pH of 8.0.** Take an aliquot (approximately 30mL) of the above stock solution and adjust the pH to 8.0 by the addition of 1 M NaOH.

A PBS buffer (pH 7.3) is prepared by dissolving 805.5 mg of sodium chloride (Fisher Scientific), 606 mg of sodium phosphate dibasic anhydrous (Fisher Scientific), 247 mg of sodium phosphate monobasic anhydrous (Fisher Scientific), then QS to 200g with sterile filtered DI water.

A 2% solution of an active agent in PBS pH 7.3 is prepared by dissolving an appropriate amount of the active agent in the PBS buffer and QS to 10 g with PBS buffer.

One mL samples are individually placed in 3mL screw cap glass vials (with rubber lining) and closed tightly. The vials are placed in a Market Forge-sterilmatic autoclave (settings, slow liquids) and sterilized at 250°F for 15 minutes. After the autoclave the samples are left to cool down to room temperature and then placed in refrigerator. The samples are homogenized by mixing the vials while cold.

Appearance (e.g., discoloration and/or precipitation) is observed and recorded. HPLC analysis is performed using an Agilent 1200 equipped with a Luna C18(2) 3μm, 100Å, 250x4.6 mm column) using a 30-80 acetonitrile gradient (1-10min) of (water - acetonitrile mixture containing 0.05%TFA), for a total run of 15 minutes. Samples are diluted by taking 30μL of sample and dissolved with 1.5mL of a 1:1 acetonitrile water mixture. Purity of the active agent in the autoclaved samples is recorded.

Formulations comprising gentamicin, ciprofloxacin and micronized dexamethasone, prepared according to the

procedure above, are tested using the above procedure to determine the effect of pH on degradation during the autoclaving step.

Example 11 Effect of buffer type on the degradation products for formulations containing poloxamer 407NF after heat sterilization (autoclaving).

A TRIS buffer is made by dissolving 377.8 mg of sodium chloride (Fisher Scientific), and 602.9 mg of Tromethamine (Sigma Chemical Co.) then QS to 100g with sterile filtered DI water, pH is adjusted to 7.4 with 1M HCl.

**Stock solution containing 25% Poloxamer 407 solution in TRIS buffer:**

Weigh 45 g of TRIS buffer, chill in an ice chilled bath then sprinkle into the buffer, while mixing, 15 g of poloxamer 407 NF (Spectrum Chemicals). The mixture is further mixed until all the poloxamer is completely dissolved.

A series of formulations is prepared with the above stock solution. An appropriate amount of active agent (or salt or prodrug thereof) and/or active agent as micronized/coated/liposomal particles (or salt or prodrug thereof) is used for all experiments.

**Stock solution (pH 7.3) containing 25% Poloxamer 407 solution in PBS buffer:**

PBS buffer described above is used. Dissolve 704mg of sodium chloride (Fisher Scientific), 601.2 mg of sodium phosphate dibasic anhydrous (Fisher Scientific), 242.7 mg of sodium phosphate monobasic anhydrous (Fisher Scientific) with 140.4 g of sterile filtered DI water. The solution is cooled down in an ice chilled water bath and then 50g of poloxamer 407NF (SPECTRUM CHEMICALS) is sprinkled into the cold solution while mixing. The mixture is further mixed until the poloxamer is completely dissolved.

A series of formulations is prepared with the above stock solution. An appropriate amount of active agent (or salt or prodrug thereof) and/or active agent as micronized/coated/liposomal particles (or salt or prodrug thereof) is used for all experiments.

Tables 4 and 5 list samples prepared using the procedures described above. An appropriate amount of active agent is added to each sample to provide a final concentration of 2% active agent in the sample.

**Table 4.** Preparation of samples containing TRIS buffer

| Sample | pH | 25% Stock Solution (g) | TRIS Buffer (g) |
|---|---|---|---|
| 20%P407/2% active agent/TRIS | 7.45 | 8.01 | 1.82 |
| 18%P407/2% active agent/TRIS | 7.45 | 7.22 | 2.61 |
| 16%P407/2% active agent/TRIS | 7.45 | 6.47 | 3.42 |
| 18%P407/2% active agent/TRIS | 7.4 | 7.18 | 2.64 |
| 4% active agent/TRIS | 7.5 | - | 9.7 |
| 2% active agent /TRIS | 7.43 | - | 5 |
| 1% active agent /TRIS | 7.35 | - | 5 |
| 2% active agent /TRIS (suspension) | 7.4 | - | 4.9 |

**Table 5.** Preparation of samples containing PBS buffer (pH of 7.3)

| Sample | 25% Stock Solution in PBS (g) | PBS Buffer (g) |
|---|---|---|
| 20%P407/2% active agent/PBS | 8.03 | 1.82 |
| 18%P407/2% active agent/PBS | 7.1 | 2.63 |
| 16%P407/2% active agent/PBS | 6.45 | 3.44 |
| 18%P407/2% active agent/PBS | - | 2.63 |
| 2% active agent /PBS | - | 4.9 |

One mL samples are individually placed in 3mL screw cap glass vials (with rubber lining) and closed tightly. The vials are placed in a Market Forge-sterilmatic autoclave (setting, slow liquids) and sterilized at 250°F for 25 minutes. After the autoclaving the samples are left to cool down to room temperature. The vials are placed in the refrigerator and mixed while cold to homogenize the samples.

HPLC analysis is performed using an Agilent 1200 equipped with a Luna C18(2) 3μm, 100Å, 250x4.6 mm column) using a 30-80 acetonitrile gradient (1-10min) of (water - acetonitrile mixture containing 0.05%TFA), for a total run of 15 minutes. Samples are diluted by taking 30μL of sample and dissolving with 1.5mL of a 1:1 acetonitrile water mixture. Purity of the active agent in the autoclaved samples is recorded. The stability of formulations in TRIS and PBS buffers is compared.

Viscosity measurements are performed using a Brookfield viscometer RVDV-II+P with a CPE-51 spindle rotated at 0.08 rpm (shear rate of 0.31 $s^{-1}$), equipped with a water jacketed temperature control unit (temperature ramped from 15-34°C at 1.6 °C/min). Tgel is defined as the inflection point of the curve where the increase in viscosity occurs due to the sol-gel transition. Only formulations that show no change after autoclaving are analyzed.

Formulations comprising gentamicin, ciprofloxacin and dexamethasone, are tested using the above procedure to determine the degradation products and viscosity of a formulation containing 2% active agent and 17% poloxamer 407NF after heat sterilization (autoclaving). Stability of formulations containing micronized active agent is compared to non-micronized drug formulation counterparts.

Example 12: Modulation of gel temperature

The effect of Poloxamer 188 and Dexamethasone sodium phospahate (DSP) on the gelation temperature and viscosity of Poloxamer 407 formulations was evaluated with the purpose of manipulating the the gelation temperature.

A 25% Poloxamer 407 stock solution in PBS buffer and the PBS solution from Example 11 were used. Poloxamer 188NF from BASF was used.

**Table 6** Preparation of samples containing poloxamer 407/poloxamer 188

| Sample | 25%P407 Stock Solution (g) | Poloxamer 188 (mg) | PBS Buffer (g) |
|---|---|---|---|
| 16%P407/10%P188 | 3.207 | 501 | 1.3036 |
| 17%P407/10%P188 | 3.4089 | 500 | 1.1056 |
| 18%P407/10%P188 | 3.6156 | 502 | 0.9072 |
| 19%P407/10%P188 | 3.8183 | 500 | 0.7050 |
| 20%P407/10%P188 | 4.008 | 501 | 0.5032 |
| 20%P407/5%P188 | 4.01 | 256 | 0.770 |

Mean dissolution time (MDT) for the 20% poloxamer 407/ 10% poloxamer 188 was measured to be 2.2hr and for the 20% poloxamer 407/ 5% poloxamer 188 showed to be 2.6 hr. Table 7 illustrates the change is gel temperature upon incorporation of a mixture of polymers in a composition

**Table 7. Viscosity and Tgel of formulations containing poloxamer 407/poloxamer 188**

| Sample | Tgel (°C) | Max Viscosity (Pas) Up to 37°C | MDT (hr) |
|---|---|---|---|
| 16%P407/10%P188 | 37.0 | 0.1 | - |
| 17%P407/10%P188 | 35.4 | 357 | - |
| 18%P407/10%P188 | 33.5 | 661 | - |
| 19%P407/10%P188 | 31.2 | 678 | - |
| 20%P407/10%P188 | 28.9 | >712 | 2.2 |
| 20%P407/5%P188 | 27.6 | >712 | 2.6 |

An equation was fitted to the data obtained and can be utilized to estimate the gelation temperature of F127/F68 mixtures (for 17-20% F127 and 0-10% F68).

$$T_{gel} = -1.8\ (\%F127) + 1.3\ (\%F68) + 53$$

An equation was fitted to the data obtained and can be utilized to estimate the Mean Dissolution Time (hr) based on the gelation temperature of F127/F68 mixtures (for 17-25% F127 and 0-10% F68), using results obtained in example 6 and 8.

$$MDT = -0.2\ (T_{gel}) + 8$$

Gelation temperature modifiers and the effect on PK in guinea pigs after intratympanic administration:

Table 8 describes the following formulations that were prepared:

**Table 8**

| Formulation (Modifier) | % Dexamethasone | % Modifier | % P407 in 50mM TRIS buffer |
|---|---|---|---|
| 007-97 T80 (Tween 80) | 1.5 | 1 | 16 |
| 007-97NMP (NMP) | 1.5 | 1 | 16 |
| 007-98 (Neat-no modifier) | 1.5 | - | 16 |
| 008-13-OL (Na Oleate) | 1.5 | 1 | 16 |
| 008-13-T20 (Tween 80) | 1.5 | 1 | 16 |

Samples were prepared using the following general method.

A saline-TRis buffer in dionized water was made, followed by the addition of the modifier (or without). The osmolality of this mixture was adjusted if necessary to be in the 250-300 mOsM/kg. The solution was then chilled and poloxamer 407 was sprinkled in while mixing until a clear solution was obtained. This solution was sterile filtered and was deliverd to a sterile dexamethasone containing dexamethasone enough to reach a concentration of 1.5% w/v dexamethasone. Tgel and max viscosity were measured as described herein.

Guinea pigs were administered 50μl via intratympanic delivery and PK in the perilymph was measured as described herein. Table 9 describes certain measured values.

**Table 9**

| Formulation (Modifier) | Tgel (°C) | Max Viscosity (cP) | **Cmax μg/mL** | **AUC μgh/mL** | **MRT h** |
|---|---|---|---|---|---|
| 007-97 T80 (Tween 80) | 25.3 | 422007 | 61.8 | 9638 | 55 |
| 007-97NMP (NMP) | 27.6 | 363107 | 7.7 | 1195 | 56 |
| 007-98 (Neat-no modifier) | 26.4 | 348868 | 8.7 | 1363 | 56 |
| 008-13-OL (sodium Oleate) | 21.7 | 596765 | 1.0 | 497 | 448 |
| 008-13-T20 (Tween 20) | 26.2 | 368285 | 4.3 | 6865 | 59 |

Example 13: In vitro comparison of relase profile.

Dissolution is performed at 37°C in snapwells (6.5 mm diameter polycarbonate membrane with a pore size of 0.4 μm), 0.2 mL of a gel formulation described herein is placed into snapwell and left to harden, then 0.5 mL buffer is placed into reservoir and shaken using a Labline orbit shaker at 70 rpm. Samples are taken every hour (0.1 mL withdrawn and replace with warm buffer). Samples are analyzed for active agent concentration by UV at 245nm against an external calibration standard curve. Pluronic concentration is analyzed at 624 nm using the cobalt thiocyanate method. Relative rank-order of mean dissolution time (MDT) as a function of %P407 is determined. A linear relationship between the formulations mean dissolution time (MDT) and the P407 concentration indicates that the active agent is released due to the erosion of the polymer gel (poloxamer) and not via diffusion. A non-linear relationship indicates release of active agent via a combination of diffusion and/or polymer gel degradation.

The MDT is inversely proportional to the release rate of an active agent from a composition described herein.

Experimentally, the released active agent is optionally fitted to the Korsmeyer-Peppas equation:

$$\frac{Q}{Q_\alpha} = kt^n + b$$

where Q is the amount of active agent released at time t, $Q\alpha$ is the overall released amount of active agent, k is a release constant of the nth order, n is a dimensionless number related to the dissolution mechanism and b is the axis intercept, characterizing the initial burst release mechanism wherein n=1 characterizes an erosion controlled mechanism. The mean dissolution time (MDT) is the sum of different periods of time the drug molecules stay in the matrix before release, divided by the total number of molecules and is optionally calculated by:

$$MDT = \frac{nk^{-1/n}}{n+1}$$

Alternatively, samples are analyzed using the method described by Li Xin-Yu paper [Acta Pharmaceutica Sinica 2008,43(2):208-203] and Rank-order of mean dissolution time (MDT) as a function of %P407 is determined.

Example 14 Effect of poloxamer concentration and active agent concentration on release kinetics

A series of compositions comprising varying concentrations of a gelling agent and micronized dexamethasone was prepared using procedures described above. The mean dissolution time (MDT) for each composition in Table 3 was determined using procedures described above.

**Table 6** Preparation of poloxamer/active agent compositions

| Sample | pH | MDT |
|---|---|---|
| 15.5%P407/1.5% dexamethasone /PBS | 7.4 | 46h |
| 16%P407/1.5% dexamethasone /PBS | 7.4 | 40h |
| 17%P407/1.5% dexamethasone /PBS | 7.4 | 39h |
| 15.5%P407/4.5% dexamethasone /PBS | 7.4 | > 7 days |
| 16%P407/4.5% dexamethasone /PBS | 7.4 | > 7 days |
| 17%P407/4.5% dexamethasone /PBS | 7.4 | > 7 days |

The effect of gel strength and active agent concentration on release kinetics of an active agent from the formulation was determined by measurement of the MDT for poloxamer, and measurement of MDT for active agent. The half life of the active agent and mean residence time (MRT) of the active agent was also determined for each formulation by measurement of concentration of the active agent in the perilymph using Korsemeyer-Peppas equation as described above.

Example 15 - Purification of Poloxamer

Method A: Poloxamer 407 (BASF Corporation, lot WPEB612B) is dissolved in of 75/25 water/iso-propanol v/v solution. The solution is equilibrated to 27 °C. Sodium chloride is added with vigorous mixing and the solution is centrifuged to allow two clear, colorless phases to form. The lower phase is drained and the solution is again diluted to near its initial weight/volume by the addition of water/iso -propanol 75/25 v/v solution followed by equilibration to 27 °C and addition of sodium chloride. The solution is centrifuged to allow two clear, colorless phases to form. The lower phase is drained a second time and the solution returned to near its original weight by the addition of water/iso-propanol solution and sodium chloride as described earlier. The resulting solution is centrifuged, the lower phase is drained and discarded. The upper phase from the third extraction is dried then extracted with chloroform. The chloroform layer is then evaporated in vacuo. The residue is dried under vacuum.

Method B: Poloxamer 407 from BASF Corporation, Mount Olive, N.J., is dissolved in deionized water. The

solution is maintained close to freezing, then ammonium sulfate is added. The solution is equilibrated at 2° C. and after two distinct phases are formed, the lower phase is discarded, and the upper phase is collected and weighed. Deionized water is added and the solution is equilibrated to 2°C. followed by addition of ammonium sulfate with stirring. After the salt is dissolved, the solution is maintained at approximately 2° C. until two phases formed. The upper phase is isolated and diluted with deionized water. The solution is chilled to about 2° C. and ammonium sulfate is added. The phases are allowed to separate as above. The upper phase is isolated and extracted with dichloromethane. Two phases are allowed to form overnight. The organic (lower) phase is isolated and dried over sodium sulfate. The dichloromethane phase is filtered through a PTFE filter (0.45 μm pore size) to remove the undissolved salts. The dichloromethane is removed in vacuo and the residue is dried overnight in an oven.

**Sample preparation**

The following ciprofloxacin/ dexamethasone samples were prepared as follows:
Cold 16% P407 in 50mM TRIS buffers-saline (pH 7.4 and osmolality of 280 mOsM) was placed in a open container, then ciprofloxacin free base or ciprofloxacin free base hydrate (3.5 moles) was sprinkled in while mixing. Ciprofloxacin suspension was mixed for not les than 10 mintes, then micronized dexamethasone was added slowly to the mixture while mixing. The homogenous suspension was then transferred to 3 mL vials and filled at different volumens (v1=4g, v2, 3g and v3 2 g). The glass vial were sealed with West stoppers (fluorotec coated) and Aluminum seals, followed by autoclaving at 250°F for 30 minutes.

The following dexamethasone suspensions were prepared by dispersing micronized dexamethasone in either 2% P407 or 10% P407 at a concentration of 28% dexamethasone. One mL of the homogenous suspension was then transferred to 20 mL vials. The glass vial were sealed with West stoppers (fluorotec coated) and Aluminum seals, followed by autoclaving at 250°F for 30 minutes.

Compositions of samples manufactured.

| Sample | % Ciprofloxacin | % Cipro-Hydrate | % Dexamethasone | In % P407 |
|---|---|---|---|---|
| 017-39B | 1.5 | - | 0.5 | 16% P407 |
| 017-39C | 1.5 | - | - | 16% P407 |
| 017-41A | - | 6 | 2 | 16% P407 |
| 017-41B | - | 3 | 1 | 16% P407 |
| 017-41C | - | - | 6 | 16% P407 |
| 017-43C | - | - | 28 | 2%P407 |
| 017-43D | - | - | 28 | 10%P407 |

**Impurity Profile Before and After Autoclaving**

| Sample | % impurity C | % 17-Ketone Dex | % Dehydro-Dex | Tgel (°C) |
|---|---|---|---|---|
| 017-39B presterile | 0.02 | BLQ | BLQ | 24.0 |
| 017-39B v1 sterile | 0.16 | 1.13 | 0.63 | 24.1 |
| 017-39B v2 sterile | 0.16 | 1.12 | 0.65 | 24.1 |
| 017-39B v3 sterile | 0.17 | 1.14 | 0.64 | 24.1 |
| 017-39C presterile | 0.02 | BLQ | BLQ | 24.2 |
| 017-39C v1 | 0.09 | BLQ | BLQ | 24.2 |

| | | | | |
|---|---|---|---|---|
| sterile | | | | |
| 017-39C v2 sterile | 0.07 | BLQ | BLQ | 24.3 |
| 017-39C v3 sterile | 0.07 | BLQ | BLQ | 24.2 |
| 017-41A presterile | 0.02 | BLQ | BLQ | NA |
| 017-41A v2 sterile | 0.09 | 0.55 | 0.29 | NA |
| 017-41B presterile | 0.02 | BLQ | BLQ | NA |
| 017-41B v2 sterile | 0.13 | 1.09 | 0.62 | NA |
| 017-41C presterile | - | BLQ | BLQ | NA |
| 017-41C v2 sterile | - | 0.14 | 0.36 | NA |
| 017-43C presterile | - | BLQ | BLQ | NA |
| 017-43C v2 sterile | - | 0.02 | BLQ | NA |
| 017-43D presterile | - | BLQ | BLQ | NA |
| 017-43D v2 sterile | - | 0.04 | BLQ | NA |

An appreciable particle size increase is seen after autoclaving when ciprofloxacin free base is used, primarly seen as an increase in the size of the ciprofloxacin needles. When ciprofloxacin free base hydrate is used above a concentration of 3%, minimal growth or recrystallization is observed after autoclaving.

Higher viscosity of suspensions made with 2% Ciprofloxacin/0.7% Dex are seen as compared to the viscosities observed with a 6% ciprofloxacin hydrate/ 2% dexamethasone. Minimal degradation or change is observed when dexamethasone is autoclaved at high concentrations with up to 10% P407.

Example 16 - Dry heat sterilization of dexamethasone

Ten milligrams of micronized dexamethsone powder (Spectrum lot XD0385) were filled into 2 mL glass vials and sealed with a 13mm butyl str rubber stopper (Kimble) and placed in the oven at different temperatures for 7-11 hours.

HPLC analysis was performed using an Agilent 1200 equipped with a Luna C18(2) 3μm, 100Å, 250x4.6 mm column) using a 30-95 of solvent B (solvent A 35% methanol:35% water:30% acetate buffer, solvent B 70% methanol: 30% acetate buffer pH 4) gradient (1-6min), then isocratic (95% solvent B) for 11 minutes, for a total run of 22 minutes. Samples were dissolved in ethanol and analyzed. Dry-heat sterilization of micronized dexamethasone at a temperature of up to 138°C did not affect particle size distribution of the micronized dexamethasone. HPLC analysis indicated 99% purity of the dry-heat sterilized micronized dexamethasone.

The dry heat sterilized dexamethasone is optionally mixed aseptically with a sterile-filtered poloxamer solution prior to administration.

Example 17 - Preparation of a Thermosensitive Gel comprising Dexamethasone and moxifloxacin

| Ingredient | Quantity (mg/g of formulation) |
|---|---|
| moxifloxacin | 15.0 |
| dexamethasone | 15.0 |
| BHT | 0.002 |
| Fractionated Poloxamer 407 | 120.0 |
| PBS buffer (0.1 M) | 9.0 |

A formulation comprising micronized dexamethasone and moxifloxacin is prepared according to Example 1 above. Fractionated poloxamer is prepared according to Example 15 described herein.

Example 18 - Preparation of a Composition comprising micronized dexamethasone powder and ciprofloxacin powder

2% dexamethasone, 2% ciprofloxacin HCl in 16 % poloxamer 407.

115.5 mg of ciprofloxacin HCl (LKT laboratories) + 100.2 mg of micronized dexamethasone (Pfizer) was suspended to a weight of 5 g with a 16% poloxamer 407 in TRIS buffer, pH was adjusted to 7.5 with 60μL of 5 N NaOH.

2% dexamethasone, 2% ciprofloxacin HCl in 50% poloxamer 407/ 25% ethanol/25% water.

Weigh 2.5g of pluronic F127 (Sigma Chemical Co) + 1.25g of ethanol (200 proof, Acros). The mixture was dissolved by applying heat (40-60°C), then 1.25 g of water was added while mixing.
115.6 mg of ciprofloxacin HCl (LKT laboratories) + 111.7 mg of micronized dexamethasone (Pfizer) was suspended in the poloxamer/ethanol/water solution.

The 50% poloxamer 407/ 25% ethanol/25% water is sterile filtered through a 0.22μm PES syringe filter. The P407/EtOH/water mixture which has an initial viscosity of about 3000-8000 cP and thickens upon administration.

Dissolution was performed at 37°C in snapwells (6.5 mm diameter polycarbonate membrane with a pore size of 0.4 μm), 0.2 mL of formulation was placed into snapwell and left to harden, then 0.5 mL of 0.9% saline was placed into reservoir and shaken using a Labline orbit shaker at 70 rpm. Samples were taken every hour (0.1 mL withdrawn and replace with warm buffer). Samples analyzed for dexamethasone and ciprofloxacin concentration by UV at 245 and 270 nm, respectively using using a Evolution 160 UV/Vis spectrophotometer (Thermo Scientific). Quantitation performed against an external calibration standard.

| Sample | MDT (hr) Dexamethasone | MDT (hr) Ciprofloxacin |
|---|---|---|
| DEX-Cipro in 16% P407 | 137 | 200 |
| DEX-Cipro in 50% P407/EtOH | 33 | 23 |

Examples 19-21 Formulations comprising combinations of amoxicillin+triamcinolone, moxifloxacin + prednisolone, and zoledronate + dexamethasone are prepared using the above procedure.

Example 22 Manufacturing and properties of a ciprofloxacin hydrogel formulations **3%Ciprofloxacin hydrogel**

Weigh 1.1399 g of NaCl (fisher lot 080788) + 1.5022 g of tromethamine (fisher lot 081507) + 205 g of Millipore DI water. Dissolve and adjust pH with ~ 1.8 mL of a 5 N HCl solution to a pH of 7.75 with a final osmolality of 273 mOsm/kg. Weigh 58.8 g of the above buffer, chill down, then sprinkle while mixing 11.291 g of poloxamer 407 NF (BASF lot WPNF580C), mix until fully dissolved.
Weigh 64.8g of a the 16% P407 (above solution) into a 100mL glass bottle containing a 35 mm stir bar then sprinkle 2.2915 g of ciprofloxacin hydrate (Neuland lot CHI071000). Mix for not less than 2 hours at a setting of 11 (IKA stir plate) minutes while cooling then fill 31 two mL vials with approximately 2 g of suspension, stopper them with 13 mm West stoppers and seal them with Al seal, autoclave for 30 minutes @ 250°C.

**Release profile**

Dissolution was performed at 37°C in snapwells (6.5 mm diameter polycarbonate membrane with a pore size of 0.4 μm), 0.2 mL of gel was placed into snapwell and left to harden, 0.5 mL of 0.9% saline was placed into reservoir and shaken using a Labline orbit shaker at 70 rpm. Samples were taken every hour (All the saline withdrawn and replace with warm 0.9% saline with an osmolality of 290 mOsm). Samples were analyzed for Ciprofloxacin by HPLC.

**Rheological properties**

Tgel measurements were performed using a Brookfield viscometer RVDV-II+P with a CP-51 spindle rotated at 0.08 rpm (shear rate of 0.31 $s^{-1}$) equipped with a temperature control unit (temperature ramped from 15-37°C at 1.6 °C/min).
Viscosity was measured at 20°C using a Brookfield viscometer RVDV-II+P with a CP-40 spindle with a shear rate ramp from 7.5 to 375 $s^{-1}$. Data was fitted to the Casson model to calculate the plastic viscosity and yield stress of the drug product.

| | Pre autoclaved | Autoclaved |
|---|---|---|
| apparent Osmolality (mOsM) | 300 | 300 |
| pH | 7.67 | 7.66 |
| Tgel (°C) | 26.4 | 26.6 |
| Max viscosity (Pas) | 424 | 399 |
| Viscosity (cP) (Casson) | 44.6 | 41.5 |
| Yield stress (D/$cm^2$) | 0.5 | 2.1 |
| MDT ciprofloxacin (h) | 83 | 87 |
| Assay (%) Ciprofloxacin | 97 | 100 |
| Appearance | White -offwhite | White -offwhite |

Ciprofloxacin chromatographic purity is shown in the table below.

| | Area % | |
|---|---|---|
| RRT | Pre autoclaved | Autoclaved |
| impurity E (0.41) | 0.02 | 0.03 |
| impurity C (0.69) | 0.05 | 0.09 |
| 0.87 | BLQ | 0.02 |
| ciprofloxacin | 99.88 | 99.82 |
| impurity D (1.27) | 0.02 | 0.03 |

(continued)

| | Area % | |
|---|---|---|
| RRT | Pre autoclaved | Autoclaved |
| 2.30 | 0.02 | 0.02 |

**12% ciprofloxacin hydrogel**

Weigh 126.1 g of the above buffer (3% ciprofloxacin hydrogel), chill down, then sprinkle while mixing 24.0 g of poloxamer 407 NF (BASF lot WPNF580C), mix until fully dissolved. Weigh 34.68g of the 16% P407 (above solution) into a 100mL glass bottle containing a 35 mm stir bar then sprinkle 5.38 g of ciprofloxacin hydrate (Neuland lot CHI071000). Mix for not less than 2 hours at a setting of 11 (IKA stir plate) minutes while cooling then fill 7 two mL vials with approximately 2 g of suspension, one 3 mL vial with 3g and one 10mL vial with 8 g of the suspension, then stopper them with West stoppers and seal them with Al seals, autoclave them for 30 minutes @ 250°C.

To prepare 0.6, 2 and 6% ciprofloxacin hydrogels the following procedure was used: 16% P407 was delivered to glass vials and autoclaved, then a specific amount of autoclaved 12% ciprofloxacin hydrate was aseptically added and thoroughly mixed, see table below for details.

| % ciprofloxacin in hydrogel | 16% P407/ vial (g) | 12% ciprofloxacin hydrogel (g) used |
|---|---|---|
| 0.6 | 18.4 | 1 |
| 2.0 | 16.4 | 3.4 |
| 6.0 | 10.2 | 10.6 |

In vitro release profile of ciprofloxacin hydrogel formulations is shown below.

| % Ciprofloxacin | MDT |
|---|---|
| 0.6 | 18 |
| 2 | 59 |
| 6 | 174 |
| 12 | 354 |
| slope | 29.376 |
| $R^2$ | 0.9999 |

The viscosity of ciprofloxacin suspensions in 16% poloxamer 407 were measured using a Brookfield viscometer RVDV-II+P with a CP-40 spindle with a ramp speed from 1-50 rpm (shear rate from 7.5 to 375 $s^{-1}$) or a CP-50 spindle with a ramp speed from 1-50 rpm (shear rate from 3.8 to 192 $s^{-1}$), equipped with a temperature control unit (temperature set at 20°C).

Ejection forces are directly proportional to the viscosity of the suspension as expressed by the Poiseuille's equation.

| % API | Plastic viscosity (cP) | Yield Stress (D/cm$^2$) | Viscosity @ a shear rate of 38s$^{-1}$ |
|---|---|---|---|
| 12 | 130 | 17.2 | 238 |
| 6 | 49.1 | 2.4 | 92.6 |
| 3 | 41 | 2 | 79 |
| 0 | 40 | 0 | 36 |

Example 23 - Application of an Enhanced Viscosity Pharmaceutical Formulation onto the Round Window Membrane

A formulation according to Example 1 is prepared and loaded into 5 ml siliconized glass syringes attached to a 27-gauge luer lock disposable needle. Lidocaine is topically applied to the tympanic membrane, and a small incision made to allow visualization into the middle ear cavity. The needle tip is guided into place over the round window membrane, and the formulation applied directly onto the round-window membrane.

Example 24 - In vivo testing of Intratympanic Injection of formulation in a guinea pig.

Female guinea pigs (Charles River) weighing 200-300g, of approximately 6-8 weeks of age are used (N = 4 per group). Prior to any procedure, animals are anesthetized using a combination of xylazine (10 mg/kg), ketamine (40 mg/kg) and acepromazine (0.75 mg/kg) for up to an hour via the intramuscular route. If needed, an intraoperative booster is administered intraperitoneally representing one-tenth of the original dose. *Intratympanic injection* - Each animal is positioned so that the head is tilted at an angle to favor injection towards the round window niche. Briefly, under visualization with an operating microscope, 50 μl of formulations comprising 0 to 50% active agent and varying concentrations of P407 are admininstered to the animals. The formulations are injected using a 27G or 30G needle through the tympanic membrane into the superior posterior quadrant behind which the round window niche is located. During the procedure and until recovery, animals are placed on a temperature controlled (40 °C) heating pad until consciousness is regained at which time they are returned to the vivarium. *Perilymph sampling procedure* - The skin behind the ear of anesthetized guinea pigs is shaved and disinfected with povidone-iodine. An incision is then made behind the ear, and muscles are carefully retracted from over the bulla. A hole is drilled though the bulla using a dental burr so that the middle ear is exposed and accessible. The cochlea and the round window membrane are visualized under a stereo surgical microscope. A unique microhole is hand drilled through the bony shell of the cochlea (active capsule) adjacent to the round window. Perilymph (5 μl) is then collected using a microcapillary inserted into the cochlear scala tympani. *Plasma and CSF collection methods* - Blood is collected by cardiac puncture into heparin coated tubes. To collect the cerebrospinal fluid (CSF), a small skin incision is made just posterior to the cranial vertex. The skin is then retracted, and the trapezius muscle scraped off the occipital bone. A small hole is then drilled through the bone. The dura is cut with a sharp scalpel and a micropipette inserted to collect blood-free CSF (50 μl).

Analytical method

Determination of active agent concentrations is performed using high pressure liquid chromatography (HPLC) combined with mass spectrometry detection (MS). The limit of detection of the method is 1.0 ng/ml. Samples (perilymph, plasma and CSF) are extracted by liquid-liquid extraction using dichloromethane:hexane:MTBE (1:1:1 v/v/v). The organic portion is then dried and the extracts reconstituted with a water:methanol solution (1:1, v/v). The samples are analyzed by reversed phase HPLC (1100 series, Agilent) using an Atlantis dC18 column maintained at 40 °C. The mobile phase is nebulized using heating nitrogen in a Z-spray source/interface and the ionized compounds detected using MS/MS (Tandem quadrupole mass spectrometer, Quattro Ultima, Waters). Peak heights of an active agent are determined using MassLynx software (Waters). The calibration curves are obtained by fitting the peak height ratios of analyte / internal standard and the standard concentrations to a suitable equation using MassLynx. Sample active agent concentrations are then interpolated using the equations derived from the calibration curves.

Data Analysis

Pharmacokinetic parameters are calculated using conventional noncompartmental pharmacokinetic methods. The apparent clearance (CL app) is calculated as the ratio between the administered intratympanic dose and the exposure (AUC). Thus the injection volume and the concentration of an active agent and poloxamer in a formulation are tested to determine optimal parameters for preclinical and clinical studies.

Example 25 - In vivo testing of Intratympanic Injection of formulation in sheep

Female sheep (Buckham Sheep Farm, Kalamazoo, MI) weighing 50-65kg, of approximately 2-4 years of age are used (N=1, 2 ears per group). Prior to any procedure, animals are anesthesized using a combination of xylazine (0.22 mg/kg), glycopyrrolate (0.01 mg/kg) and ketamine (15 mg/kg) administered IM in addition to isoflurane by inhalation. *Intratympanic injection* - Each intubated animal is immobilized and placed laterally in reverse trendelenburg position, with the rostrum slightly elevated to ensure access to the round window. Following ear cleaning (using Otocalm and warm saline), and under otoscopic visualization, 600 μl of formulations comprising 0 to 50% active agent and P407 are admininstered to the animals. The formulation are injected using a 25G or 27G needle through the tympanic membrane

into the posterior inferior quadrant towards the round window niche. After dosing, the animal is left on an incline with its head up for approximately 30min to allow the dosing solution to settle into the tympanic cavity. Procedure is then repeated for the opposite ear. *Perilymph sampling procedure* - The animal is intubated and placed in lateral recumbency. A post-auricular skin incision is made and the post-auricular vein located and ligated. Cautery is performed to expose the bulla and temporal bone. The middle ear is accessed using a nitrogen powered drill and a round-tipped bur. The middle ear ossicles are pushed to the side, with care taken to avoid damaging the round window membrane. Using a 0.5-1 mm round-tipped burr, a hole is hand drilled into the basal turn of the cochlea until the bone is thin enough to pierce with a modified sewing needle. Perilymph (50μl) is then collected using Hamilton syringe connected to a 28-32G needle inserted into the cochlear scala tympani. *Plasma and CSF collection methods* - Blood is collected from the jugular vein into heparin coated tubes. To collect the cerebrospinal fluid (CSF), a small skin incision is made over the cisterna magna and a 22G needle inserted to sample blood-free CSF (500 μl).

The samples are analysed as described above. The gel elimination time course for each formulation is determined. A faster gel elimination time course of a formulation indicates lower mean dissolution time (MDT). Thus the injection volume and the concentration of an active agent and poloxamer in a formulation are tested to determine optimal parameters for preclinical and clinical studies.

Example 26 - In vivo extended release kinetics in the ear

A cohort of 21 guinea pigs (Charles River, females weighing 200-300g) is intratympanically injected with 50 μL 15 - 17% Pluronic F-127 formulation buffered at 280 mOsm/kg and containing 1.5% to 35% active agent by weight of the formulation. Animals are dosed on day 1. The release profile for the formulations is determined based on analysis of the perilymph and/or middle ear fluids.

Example 27 - Evaluation of Otic agent Formulations in an Otitis Media Animal Model *Induction of Otitis Media*

Healthy adult chinchillas weight 400 to 600 g with normal middle ears, ascertained by otoscopy and tympanometry are used for these studies. Eustachian tube obstruction is performed 24 hours before inoculation to prevent the inoculum from flowing out of the eustachian tube. One milliliter of type 3 *S.pneumoniae* strain at 4-h-log phase (containing approximately 40 colony forming units (CFU)) is placed directly into both middle ear hypotympanic bullae of the chinhillas. Control mice are inoculated with one milliliter sterile PBS.

*Treatment*

*S. pneumoniae* inoculated and control mice are sorted into two groups (n = 10 in each group). An otic agent formulation containing amoxicillin is applied to the walls of the tympanic cavity of one group of animals. Control formulation containing no amoxicillin is applied to the second group. The amoxicillin and control formulations are reapplied three days after the initial application. The animals are sacrificed after the seventh day of treatment.

Analysis of Results

Auris media ear fluid (MEF) is sampled at 1, 2, 6, 12, 24, 48 and 72 hours after pneumoccal inocualtion. Quantitative MEF cultures are performed on sheep blood agar, with the quantitation threshold set at 50 CFU/ml. Inflammatory cells are quantitated with a hemocytometer, and differential cell enumeration performed with Wright's staining.

Example 28 - Evaluation of Otic agent Formulations in an Otitis Externa Animal Model

Otitis externa is induced in 20 Sprague-Dawley rats using a plastic pipette to aggravate the tissue of the ear canal. All of the rats develop OE within one day. The formulation of Example 2 is administered to the ears of half of the rats using a needle and syringe, while the remaining rats receive the same formulation without the otic agent. The ear canal tissue is observed for redness and swelling that characterizes the condition. Light microscopy is used to analyze biopsy samples from the rats.

Example 29 - Clinical Trial of Otic agent Formulations in combination with Tympanostomy for Treatment of Otitis Media with Effusion

The purpose of this study is to determine if a composition comprising a combination of Ciprofloxacin and Dexamethasone administered in combination with a tympanostomy is safe and effective in preventing and/or treating middle ear infections in patients with ear tubes.

Study Type: Interventional

Study Design: This will be a non-inferiority open label study to compare the current standard of care versus the use of extended release intratympanic compositions in combination with tympanostomy. The current standard of care requires the use of otic drops for 5-7 days post-surgery. The study is designed to test whether administration of a sustained release composition at the time of surgery obviates the need for out-patient treatment. The test hypothesis is that administration of a single injection of an extended release composition at the time of surgery is not inferior to administration of otic drops after surgery.

Inclusion Criteria:

6 months to 12 years old, Acute Otitis Media with effusion in one or both ears
Patient may not have had otic surgery other than tube placement in the last year
Patient may not have any disease or condition that would negatively affect the conduct of the study
Patient may not require any other systemic antimicrobial therapy during the study.
Analgesic use (other than acetaminophen) is not allowed
Patient may not be pre-disposed to neurosensory hearing loss

Exclusion Criteria: Age

Study Protocol: Twenty patients will be divided into two groups. The first group of patients will receive an injection of an extended release composition comprising micronized ciprofloxacin and micronized dexamethasone during the surgical procedure. Each patient will undergo a tympanostomy for placement of a tube. During the surgical procedure, the surgeon will clean the ear of all effusion and while the myngotomoy incision is open, the surgeon injects a test composition into the middle ear space. The tube is inserted after injection of the extended release composition into the middle ear space. The test composition is either prepared in the operating room by suspending dry micronized powder of ciprofloxacin and dexamethasone with other excipients, or the test composition is a prepared suspension ready for injection.

The second group of patients will be given ear drops comprising non-micronized water soluble form of ciprofloxacin and non-micronized water soluble form of dexamethasone as immediate release components to be administered for 5-7 days after the surgery.

Patients are monitored with weekly follow up visits for one month. Any differences in treatment outcomes between the two groups are recorded.

Primary Outcome Measures: Time to cessation of otorrhea as recorded by the parent or guardian via a patient.
Secondary Outcome Measures: Clinical cure rate; Microbiological outcome; Treatment failures; Recurrence of disease.

The treatment outcome for each group of patients is compared to determine whether administration of the extended release composition comprising ciprofloxacin and dexamethasone in combination with tympanostomy is more effective than administration of ear drops comprising ciprofloxacin and dexamethasone after surgery for reduction of otorrhea, infections, or inflammation associated with tympanostomy.

Example 30 - Treatment of sinusitis in an animal model and evaluation of in vivo sustained release

An animal model described by Chiu et al. in American Journal of Rhinology, 2007, 21, 5-9, is used in this study.

The maxillary sinus ostium of white rabbits is obstructed with a pledget through an antrostomy created in the anterior face of the maxilla. The sinus is inoculated with Pseudomonas aeruginosa. After 7 days, the antrostomy is reopened, the ostial obstruction is removed, and a single lumen catheter is placed. Normal saline is irrigated through the catheter for 7 days in one group of rabbits (placebo group), while a control group receives no irrigation. A third test group receives a single dose of a test intrasinusoidal formulation. On day seven of the study, the rabbits are euthanized, analyzed under light microscopy, and bacterial counts of the nasal lavage are determined. Purulence, mucosal and underlying bony inflammation in both the control and the saline irrigation groups confirms presence of sinusitis. A reduction in bacterial counts in the nasal lavage, purulence and inflammation in the treatment group indicates an effective therapeutic outcome.

Sustained release of an active agent is determined in the nasal lavage or the sinus lavage using a suitable

technique (e.g., UV spectrometry, HPLC, mass spectormety) for detection of active agent the lavage. Epithelial scraping from sinonasal passages is used to determine tissue exposure of the active agent.

Example 31: Clinical trial for evaluation of an intrasinusoidal formulation in combination with surgery

This is a study to determine safety and efficacy of a combination of an intrasinusoidal formulation and balloon rhinoplasty in reducing recurrence of sinusitis in pediatric patients with a long history of sinusitis and failed medication.

Eligibility: 2 Years to 17 Years, both genders; Planned surgical intervention (i.e. endoscopic sinus surgery, adenoidectomy, sinus irrigation for obtaining a culture) recommended by PI, consented to by patient's legal guardian); Longstanding sinusitis: >3 mo symptoms OR 6 episodes/yr AND failed 2 courses antibiotics followed by positive CT scan

Exclusion Criteria: Extensive previous sinonasal surgery in target ostia; cystic fibrosis; extensive sinonasal osteoneogenesis; sinonasal tumors or obstructive lesions; history of facial trauma that distorts sinus anatomy and precludes access to the sinus ostium; ciliary dysfunction

Study design: Balloon dilation of the sinuses is performed using commercially available devices which include sinus guiding catheters, sinus guidewires, sinus exchange and irrigation catheters, sinus balloon inflation devices and sinus balloon catheters. Balloon dilation will be performed using endoscopic equipment with video documentation capability. A single dose of an intrasinusoidal formulation from Example 9 is administered via the catheter into the intrasinusoidal cavitiy. Patients are monitored for one year.

Primary Outcome Measures:

Sinus-related Adverse Events during balloon dilation through 12 months; Improvement in sinus symptom scores.

Secondary Outcome Measures:

Effectiveness of medication through 1 year; effectiveness of surgery and intrasinusoidal composition as measured by post-op interventions; days out of school; recurrence rate

Example 32: Effect of intrasinusoidal compositions in treatment of nasal polyposis

The aim of this study is to determine whether administration of an intrasinusoidal formulation of Example 9 reduces the size of nasal polyps, or reduces thickness of nasal polyps, and relieves symptoms in people with chronic rhinosinusitis (CRS).

Eligibility: Subjects must meet the criteria for CRS, namely they must have (1) at least two major criteria (facial pain/pressure or headache, nasal congestion, anterior or posterior nasal drainage, hyposmia/anosmia) for at least 3 consecutive months; (2) an abnormal sinus CT scan in at least two sinus areas documented within 3 months of entry or endoscopic evidence of disease. Subjects must have bilateral polypoid disease demonstrated either by CT or endoscopy with evidence of nasal polyps or polypoid mucosa on examination in at least two of the following areas: right maxillary sinus, left maxillary sinus, right anterior ethmoid sinus, left anterior ethmoid sinus plus a minimal polyp/polypoid score of 4 on the baseline rhinoscopic examination. Nasal polyps are defined as discreet polyps visible in the middle meatus area.

Exclusion criteria: Subjects who have received antibiotics within 3 weeks of the screening visit; Subjects with uncontrolled moderate to severe asthma (defined as FEV1 < 80% with asthma control Test <19 for the week prior to entry), recent exacerbation, or use of systemic steroids burst within 6 weeks of study enrollment. Subjects who are receiving a maintenance dose of corticosteroid.

Study design: Patients are administered a single dose of an intrasinusoidal composition of Example 9 via a catheter directly into the nasal polyp, or in the vicinity of the nasal polyp. Patients are monitored for one year.

Primary Outcome Measures:

Quantification of polypoid mucosal thickening in the anterior ethmoid and maxillary sinuses on sinus CT scan. Recurrene of symptoms and/or polyps.

Example 32 - Evaluation of Ion Channel Modulator Administration in Meniere's Patients

Study Objective

The primary objective of this study will be to assess the safety and efficacy of dexamethasone in ameliorating

Meniere's Disease in human subjects.

*Study Design*

This will be a Prospective, Randomized, Double-blind, Placebo-controlled, Multicenter, Phase 1B Study comparing dexamethasone administration to placebo in the treatment of Meniere's disease in patients with unilateral disease. Approximately 100 subjects will be enrolled in this study. Each group will receive either a single dose of a sustained release an ion channel modulator formulation comprising dexamethasone or placebo treatment.

Subjects who do not complete the study will not be replaced. Patients receiving the study drug will be administered a gel formulation of Example 1 directly onto the subjects' round window membrane and monitored for 3 months. Each patient will receive a vestibular and hearing evaluation before the treatment and every two weeks after administration of the study drug.

*Primary Outcome Measures:*

The primary objective of this study is to evaluate the safety and tolerability of two ascending doses of the dexamethasone relative to placebo. Safety assessments will be performed for 3 months post single intratympanic injection of the dexamethasone or placebo.

*Secondary Outcome Measures:*

The secondary objective of this study is to evaluate the clinical activity of two doses of dexamethasone relative to placebo. Change in baseline for vertigo frequency will be evaluated. The impact of tinnitus on activities of daily living will be measured. Hearing loss in the affected ear will be measured by audiometric examination. Quality of life will be measured by patient reported questionnaire. Severity of vertigo episodes will be measured by the patient reported vertigo score.

The present invention further relates to the following numbered embodiments:

1. A pharmaceutical formulation comprising an active agent, a thermosensitive polymer comprising polyoxyethylene and polyoxypropylene copolymers, and

a) having a syringable viscosity at time of administration suitable for administration via a 25 - 31 gauge needle;
b) having a gelation temperature between about 14 °C and about 42 °C ;
c) providing in vivo sustained release of a therapeutically effective amount of the active agent for a period of at least 3 days; and
(d) having less than 50 cfu of microbial agents per gram of the formulation;

provided that

(i) the formulation comprises less than 14.5% of the thermosensitive polymer by weight of the formulation and further comprises one or more gelation temperature increasing agents; or
(ii) the formulation comprises more than 25% of the thermosensitive polymer by weight of the formulation and further comprises one or more gelation temperature decreasing agents; or
(iii) the formulation comprises between about 5% and about 20% of the thermosensitive polymer by weight of the formulation, wherein the thermosensitive polymer has been purified, and optionally further comprises one or more gelation temperature increasing or gelation temperature decreasing agents; or
(iv) the formulation comprises between about 14.5% and about 25% of the thermosensitive polymer by weight of the formulation and further comprises one or more gelation temperature increasing or gelation temperature decreasing agents.

2. The formulation of embodiment 1, wherein the formulation provides an in vivo sustained release of a therapeutically effective amount of the active agent for a period of at least 5 days.
3. The formulation of embodiment 1, wherein the formulation provides an in vivo sustained release of a therapeutically effective amount of the active agent for a period of at least 7 days.
4. The formulation of embodiment 1, wherein the formulation is administered at or in the vicinity of the round window membrane of the ear.
5. The formulation of embodiment 1, wherein the in vivo sustained release occurs in the inner ear.
6. The formulation of embodiment 1, wherein the formulation is administered into or in the vicinity of one or more

sinonasal cavities.

7. The formulation of embodiment 1, wherein the in vivo sustained release occurs in a sinusoidal cavity or in the vicinity of a sinonasal cavity.

8. The formulation of embodiment 1, wherein the thermosensitive polymer is P407.

9. The formulation of embodiment 1, wherein the formulation is substantially free of additional preservatives.

10. The formulation of embodiment 1, wherein the formulation is substantially free of pyrogens.

11. The formulation of embodiment 1, wherein the formulation comprises less than about 5 endotoxin units (EU) per kg of body weight of a subject.

12. The formulation of embodiment 1, wherein the formulation is substantially free of additional tonicity agents.

13. The formulation of embodiment 1, wherein the formulation comprises a suspension of one or more multiparticulate active agents.

14. The formulation of embodiment 13, wherein the multiparticulate active agent is a micronized active agent sterilized by dry-heat, irradiation or steam sterilization.

15. The formulation of embodiment 1, wherein the formulation has any individual product related impurity of no more than 1% by weight of the formulation.

16. The formulation of embodiment 1, wherein the formulation has total product related impurities of no more than 2% by weight of the formulation.

17. The formulation of embodiment 1, wherein the active agent is a corticosteroid, or a salt or prodrug or solvate thereof.

18. The formulation of embodiment 17, wherein the corticosteroid is 21-acetoxypregnenolone, alclometasone, algestone, amcinonide, beclomethasone, betamethasone, budesonide, chloroprednisone, clobetasol, clobetasone, clocortolone, cloprednol, corticosterone, cortisone, cortivazol, deflazacort, desonide, desoximetasone, dexamethasone, diflorasone, diflucortolone, difluprednate, enoxolone, fluazacort, flucloronide, flumethasone, flunisolide, fluocinolone acetonide, fluocinonide, fluocortin butyl, fluocortolone, fluorometholone, fluperolone acetate, fluprednidene acetate, fluprednisolone, flurandrenolide, fluticasone propionate, formocortal, halcinonide, halobetasol propionate, halometasone, halopredone acetate, hydrocortamate, hydrocortisone, loteprednol etabonate, mazipredone, medrysone, meprednisone, methylprednisolone, mometasone furoate, paramethasone, prednicarbate, prednisolone, prednisolone 25-diethylamino-acetate, prednisolone sodium phosphate, prednisone, prednival, prednylidene, rimexolone, tixocortol, triamcinolone, triamcinolone acetonide, triamcinolone benetonide, or triamcinolone hexacetonide, or salt or prodrug thereof.

19. The formulation of embodiment 17, wherein the corticosteroid is dexamethasone, prednisolone, methylprednisolone, triamcinolone, or a salt or prodrug or solvate thereof, or a combination thereof.

20. The formulation of embodiment 17, wherein the corticosteroid is dexamethasone, or a salt or prodrug or solvate thereof.

21. The formulation of embodiment 20, wherein the dexamethasone is dexamethasone sodium phosphate or dexamethasone acetate.

22. The formulation of embodiment 20, wherein the dexamethasone, or salt or prodrug or solvate thereof, is present in an amount from about 0.05% to about 40% by weight of the formulation.

23. The formulation of embodiment 20, wherein the dexamethasone, or salt or prodrug or solvate thereof, is present in an amount from about 0.1% to about 30% by weight of the formulation.

24. The formulation of embodiment 20, wherein the dexamethasone, or salt or prodrug or solvate thereof, is present in an amount from about 0.5% to about 15% by weight of the formulation.

25. The formulation of any one of embodiments 13-20, wherein the formulation provides an in vivo sustained release of a therapeutically effective amount of dexamethasone for a period of at least 7 days.

26. The formulation of embodiment 1, wherein the active agent is an antimicrobial agent.

27. The formulation of embodiment 26, wherein the antimicrobial agent is an antibiotic.

28. The formulation of embodiment 27, wherein the antibiotic is amikacin, gentamicin, kanamycin, neomycin, netilmicin, streptomycin, tobramycin, paromycin, geldanamycin, herbimycin, loracarbef, ertapenem, doripenem, imipenem, meropenem, cefaclor, cefamandole, cefotoxin, cefprozil, cefuroxime, cefixime, cefdinir, cefditoren, cefpodoxime, ceftazidime, ceftibuten, ceftizoxime, ceftriaxone, cefepime, ceftobirprole, vancomycin, azithromycin, clarithromycin, dirithromycin, erythromycin, roxithromycin, troleandomycin, telithromycin, spectinomycin, aztreonam, amoxicillin, ampicillin, azociling, carbenicillin, cloxacillin, dicloxacillin, flucloxacillin, mezlocillin, meticillin, nafcillin, oxacillin, peperacillin, ticarcillin, bacitracin, colistin, polymyxin B, ciprofloxacin, clavulanic acid, enoxacin, gatifloxacin, levofloxacin, lomefloxacin, moxifloxacin, nonfloxacin, ofloxacin, trovafloxacin, grepafloxacin, sparfloxacin, AL-15469A, AL-38905, OP-145, afenide, prontosil, sulfacetamide, sulfamethiazole, sulfanilimide, sulfasalazine, sulfisoxazole, trimethoprim, cotrimoxazole, demeclocycline, doxycycline, minocycline, oxytetracycline, tetraycline, linezolid, arsogebanubem chloramphenicol, clindamycin, lincomycin, ethambutol, fosfomycin, fusidic acid, furazolidone, isoniazid, linezolid, metronidazole, mupirocin, nitrofurantoin, platensimycin, pyrazinamide, quinupristin, dalfopristin, rifampicin, thamphenicol, tinidazole, amoxicillin+clavulanic acid, Maximin H5, Dermcidin, Cecropins, andropin, moricin, ceratotoxin, melittin, Magainin, dermaseptin, bombinin, brevinin-1,esculentins and buforin II, CAP18, LL37 , abaecin, apidaecins, prophenin, indolicidin, brevinins, protegrin, tachyplesins, defensins, drosomycin, alamethicin, pexiganan or MSI-78, MSI-843, MSI-594, polyphemusin, colicin, pyocin, klebicin, subtilin, epidermin, herbicolacin, brevicin, halocin , agrocin, alveicin, carnocin, curvaticin, divercin ,enterocin, enterolysin, erwiniocin, glycinecin, lactococin, lacticin, leucoccin, mesentericin, pediocin, plantaricin, sakacin, sulfolobicin, vibriocin, warnerinand, nisin, or a salt or cocrystal, or prodrug or solvate thereof, or a combination thereof.

29. The formulation of embodiment 27, wherein the antibiotic agent is ciprofloxacin, amoxicillin, amoxicillin+clavulanic acid, moxifloxacin or ofloxacin.

30. The formulation of embodiment 27, wherein the antibiotic agent is ciprofloxacin or ciprofloxacin hydrate.

31. The formulation of embodiment 30, wherein the ciprofloxacin or ciprofloxacin hydrate is present in an amount between about 0.1 to about 20% by weight of the formulation

32. The formulation of embodiment 31, wherein the formulation provides an in vivo sustained release of a therapeutically effective amount of ciprofloxacin for a period of at least 7 days.

33. The formulation of embodiment 1, wherein the gel temperature increasing agent or gel temperature decreasing agent is selected from P188, P338, cyclodextrin, Tween 20, Tween 40, Tween 65, Tween 80, Tween 85, sodium oleate, sodium caprate, sodium caprylate and PEG.

34. A kit comprising (a) sterilized multiparticulate active agent powder and (b) a solution comprising a thermosensitive polymer, wherein (a) and (b), when combined, form a formulation according to embodiment 1.

While preferred embodiments of the present invention have been shown and described herein, such embodiments are provided by way of example only. Various alternatives to the embodiments described herein are optionally employed in practicing the inventions. It is intended that the following claims define the scope of the invention and that methods and structures within the scope of these claims and their equivalents be covered thereby.

## Claims

**1.** A pharmaceutical formulation comprising an active agent, and between about 5% to about 17% of a thermosensitive polymer comprising polyoxyethylene and polyoxypropylene copolymers, and
having a gelation temperature between about 14 °C and about 42 °C;
wherein the active agent is a WNT modulator that modulates re-growth of damaged auris sensory hair cells.

**2.** The pharmaceutical formulation of claim 1, wherein the pharmaceutical formulation is formulated to provide an in vivo sustained release of a therapeutically effective amount of the active agent for a period of at least 5 days.

**3.** The pharmaceutical formulation of claim 1, wherein the pharmaceutical formulation is formulated to provide an in vivo sustained release of a therapeutically effective amount of the active agent for a period of at least 7 days.

**4.** The pharmaceutical formulation of claim 1, wherein the pharmaceutical formulation is formulated for administration at or in the vicinity of the round window membrane of the ear.

**5.** The pharmaceutical formulation of claim 2, wherein the pharmaceutical formulation is formulated to provide in vivo sustained release of the active agent in the inner ear.

**6.** The pharmaceutical formulation of claim 1, wherein the thermosensitive polymer is P407.

**7.** The pharmaceutical formulation of claim 1, wherein the pharmaceutical formulation is substantially free of additional preservatives.

**8.** The pharmaceutical formulation of claim 1, wherein the pharmaceutical formulation is substantially free of pyrogens.

**9.** The pharmaceutical formulation of claim 1, wherein the pharmaceutical formulation comprises less than about 5 endotoxin units (EU) per kg of body weight of a subject.

**10.** The pharmaceutical formulation of claim 1, wherein the pharmaceutical formulation comprises a suspension of one or more multiparticulate active agents.

**11.** The pharmaceutical formulation of claim 10, wherein a multiparticulate active agent of the one or more multiparticulate active agents is a micronized active agent.

**12.** The pharmaceutical formulation of claim 1, wherein the pharmaceutical formulation has any individual product related impurity of no more than 1% by weight of the formulation.

**13.** The pharmaceutical formulation of claim 1, wherein the pharmaceutical formulation has total product related impurities of no more than 2% by weight of the formulation.

**14.** The pharmaceutical formulation of claim 1, wherein the pharmaceutical formulation comprises between about 15% to about 17% of the thermosensitive polymer.

**15.** The pharmaceutical formulation of claim 1, wherein the WNT modulator is selected from the group consisting of 2-amino-[3,4-(methylenedioxy)benzyl-amino]-6-(3-methoxyphenyl)pyrimidine and the signaling molecule Cerberus.

**Figure 1**

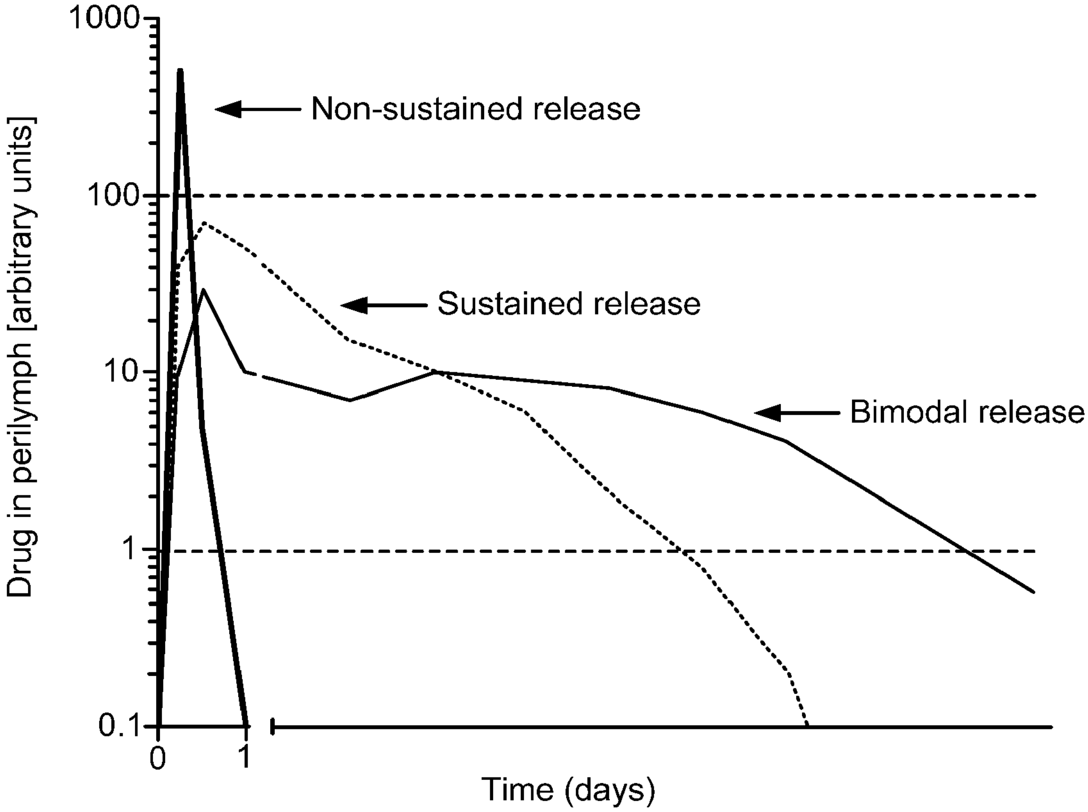
1000
100
10
1
0.1
Drug in perilymph [arbitrary units]
Non-sustained release
Sustained release
Bimodal release
0
1
Time (days)

**Figure 2**

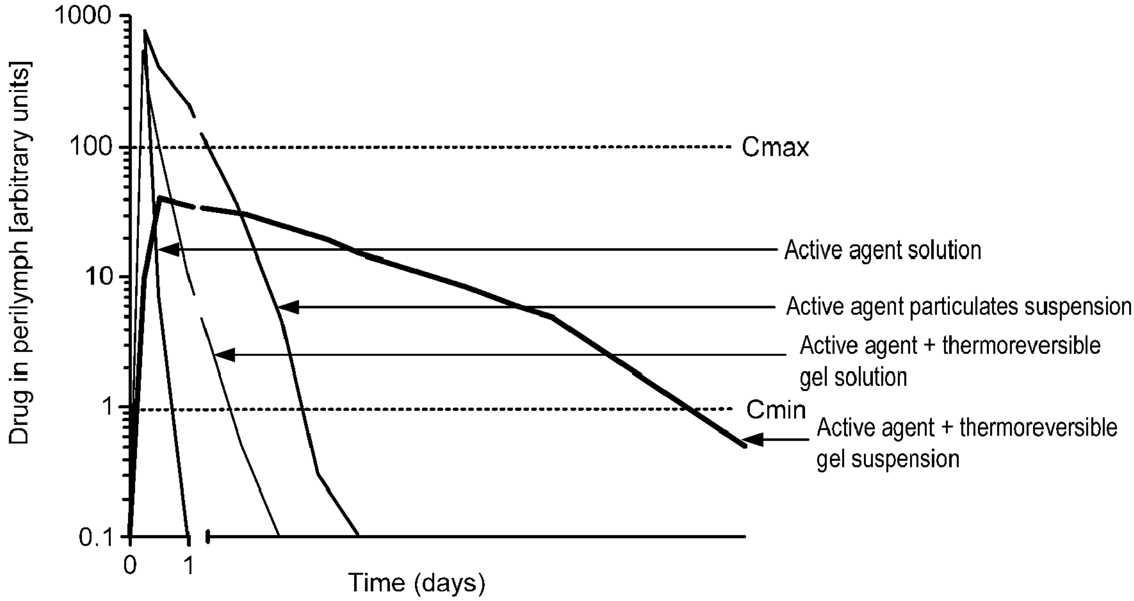
1000
100
10
1
0.1
Drug in perilymph [arbitrary units]
Cmax
Cmin
Active agent solution
Active agent particulates suspension
Active agent + thermoreversible gel solution
Active agent + thermoreversible gel suspension
0
1
Time (days)

**Figure 3**

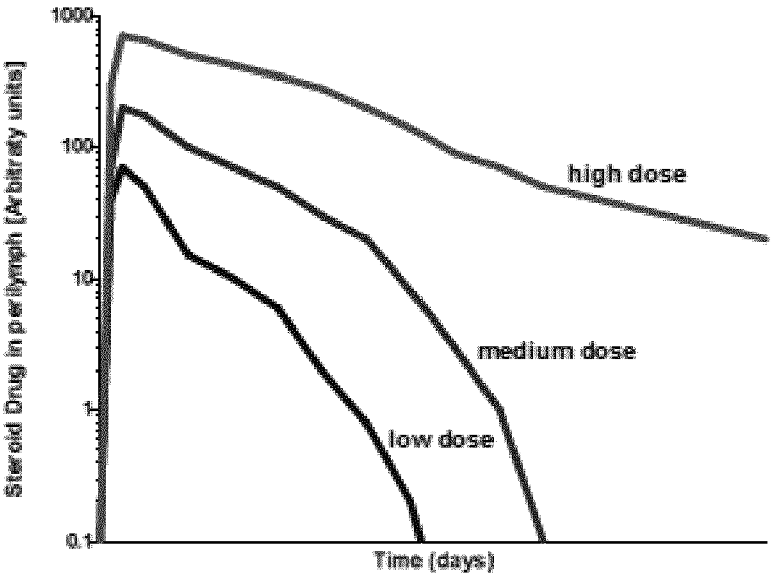
1000
100
10
1
0.1
Steroid Drug in perilymph [Arbitraty units]
high dose
medium dose
low dose
Time (days)

Figure 4

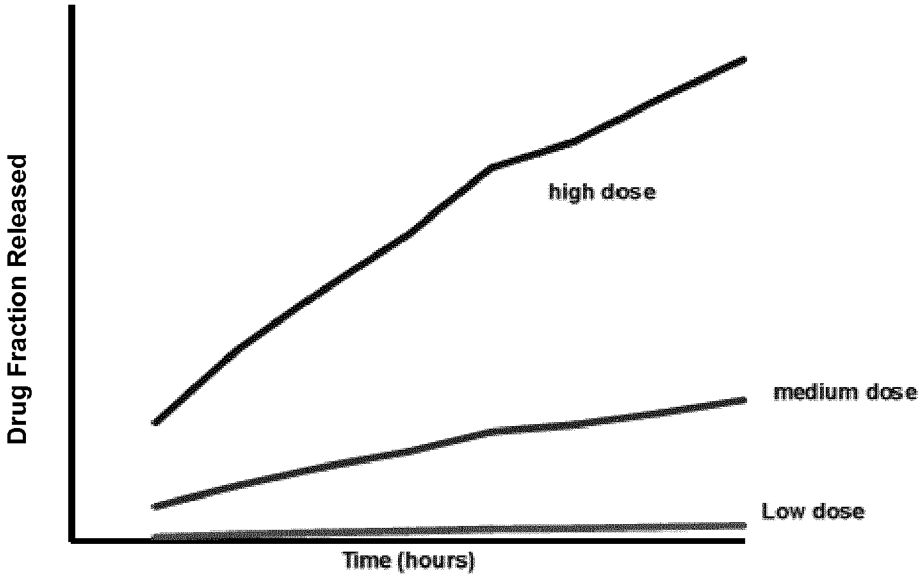
Drug Fraction Released
high dose
medium dose
Low dose
Time (hours)

**Figure 5**

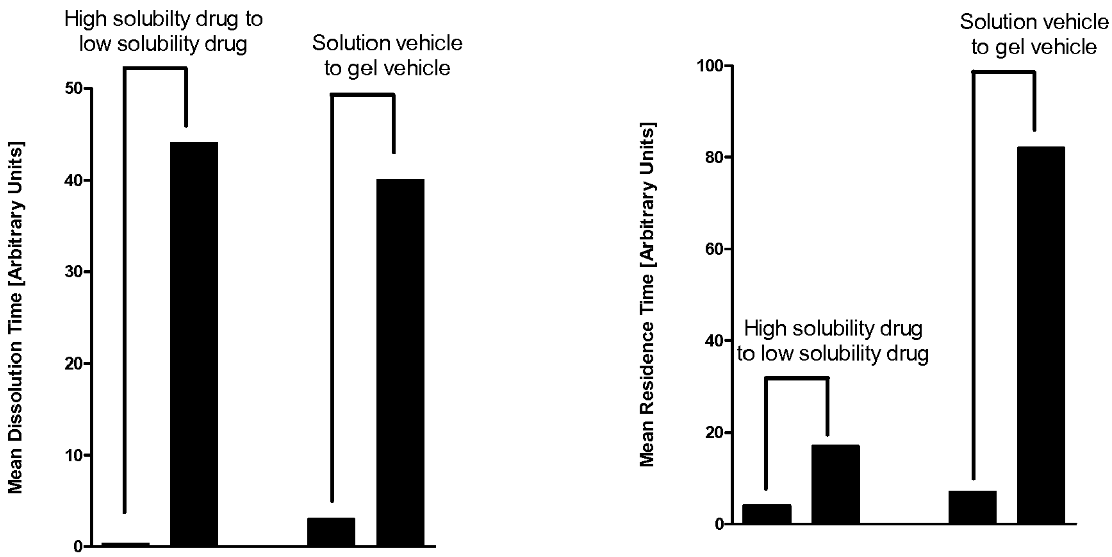
High solubilty drug to low solubility drug
Solution vehicle to gel vehicle
Mean Dissolution Time [Arbitrary Units]
0
10
20
30
40
50
Solution vehicle to gel vehicle
High solubility drug to low solubility drug
Mean Residence Time [Arbitrary Units]
0
20
40
60
80
100

Figure 6

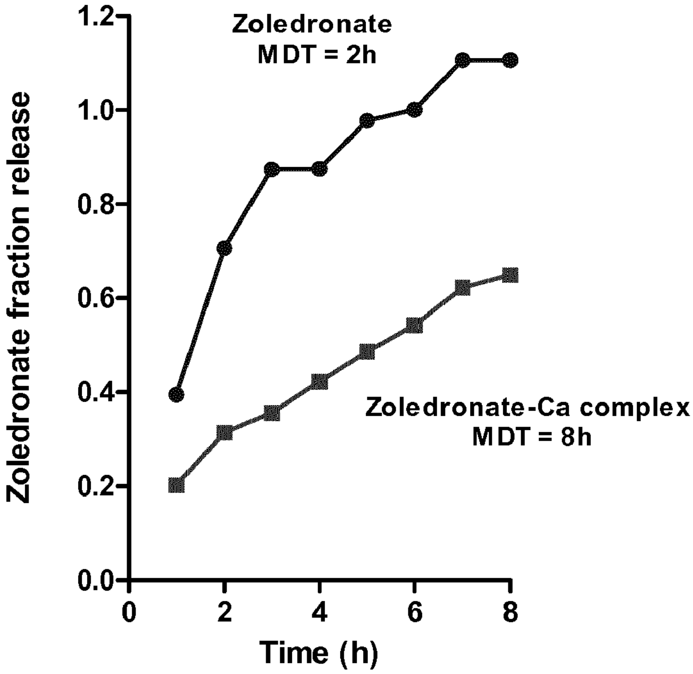
Zoledronate
MDT = 2h
Zoledronate-Ca complex
MDT = 8h
Zoledronate fraction release
1.2
1.0
0.8
0.6
0.4
0.2
0.0
0
2
4
6
8
Time (h)

# Figure 7

| Drug | Drug Class | Drug % | P407 % | Log D pH 7.4 | Solub. mg/ml | MDT h |
|---|---|---|---|---|---|---|
| Dexamethasone | Glucocorticoid | 1.5 | 17 | 1.9 | 0.070 | 40 |
| DSP | Glucocorticoid | 2 | 17 | -3.7 | 200 | 3 |
| DA | Glucocorticoid | 1.5 | 17 | 2.2 | 0.020 | 71 |
| Methylprednisolone | Glucocorticoid | 1.5 | 17 | 2.0 | 0.150 | 45 |
| MPS | Glucocorticoid | 2 | 17 | -0.3 | 250 | 3 |

| Drug | Drug Class | Drug % | P407 % | Log D pH 7.4 | Solub. mg/ml | MDT h |
|---|---|---|---|---|---|---|
| Dextromethorphan | NMDA antagonist | 2 | 16 | 2.4 | 0.2 | 15 |
| Eliprodil | NMDA antagonist | 0.5 | 16 | 4.1 | 0.015 | 48 |
| L-701324 | NMDA antagonist | 0.6 | 16 | 4.5 | 0.005 | 256 |

| Drug | Drug % | P407 % | Log D pH 7.4 | Solub. mg/ml | MDT h |
|---|---|---|---|---|---|
| SP-600125 | 0.5 | 16 | 3.2 | 0.010 | 55 |

| Drug | Drug Class | Drug % | P407 % | Log D pH 7.4 | Solub. mg/ml | MDT h |
|---|---|---|---|---|---|---|
| Amitriptyline | Tricyclic | 2 | 16 | 3.2 | 10 | 3 |
| Droperidol | Antidopaminergic | 2 | 16 | 3.1 | 0.005 | 200 |
| Meclizine | Antihistamine | 2 | 16 | 4.8 | 0.001 | 70 |

Figure 8

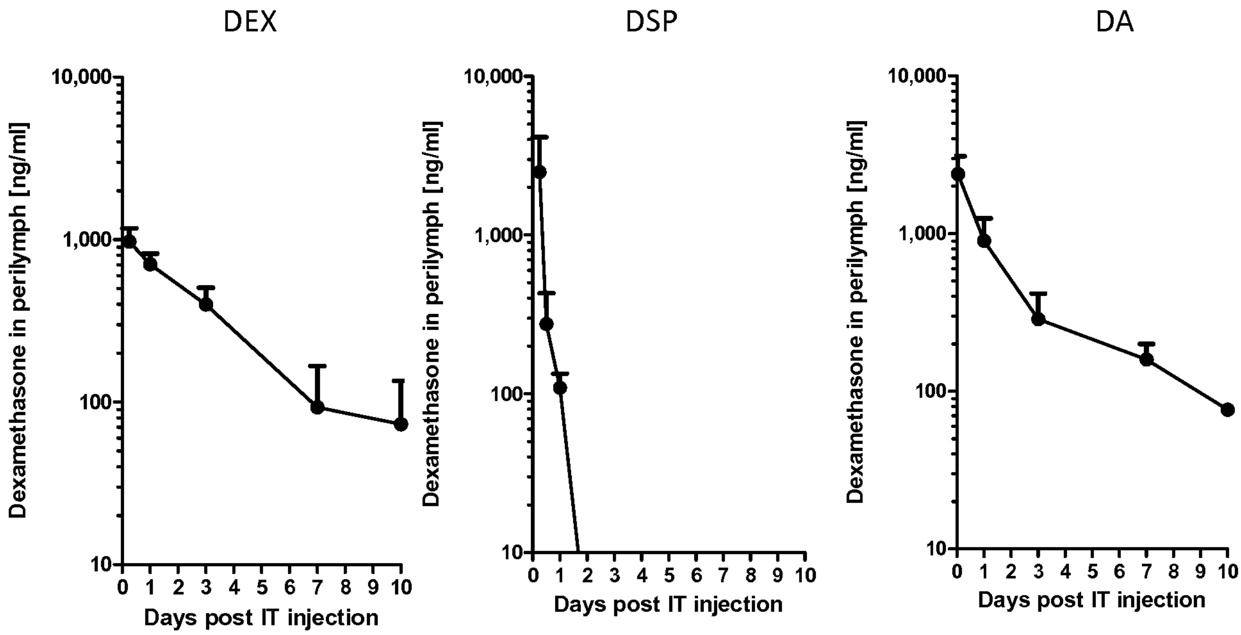


| | AUC µg.h/ml | MRT h | T1/2 h |
|---|---|---|---|
| DEX | 84 | 82 | 64 |
| DSP | 28 | 7 | 5 |
| DA | 104 | 66 | 48 |

**Figure 9**

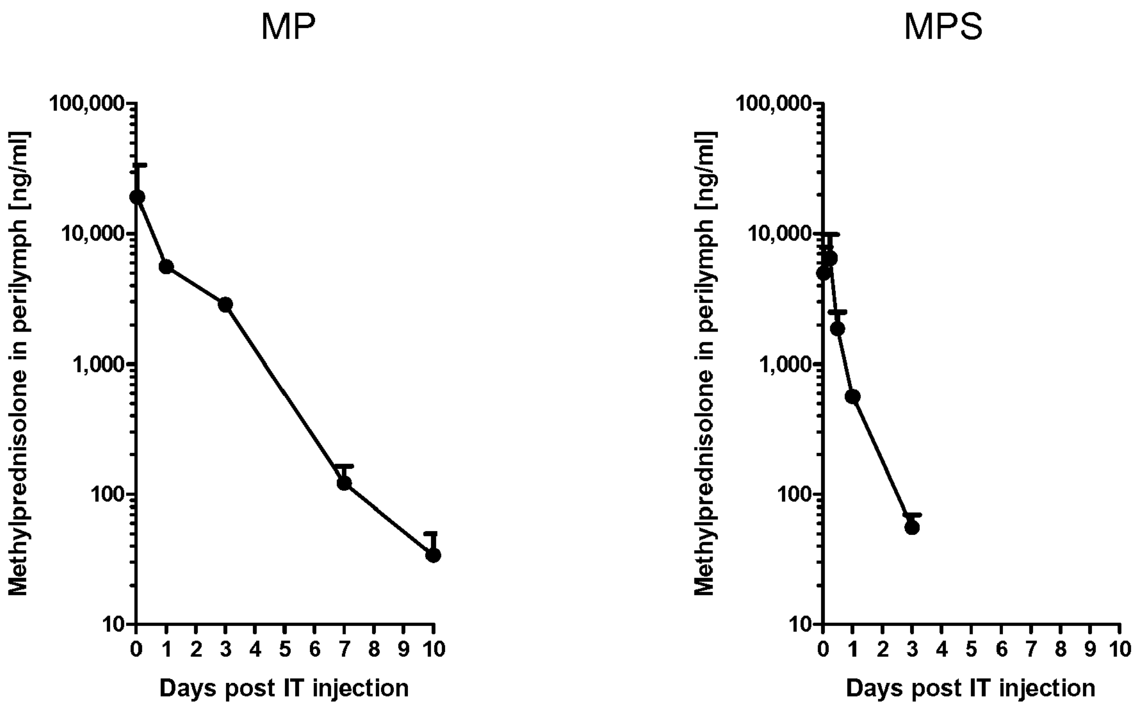


| | AUC µg.h/ml | MRT h | T1/2 h |
|---|---|---|---|
| MP | 659 | 34 | 26 |
| MPS | 89 | 11 | 11 |

Figure 10

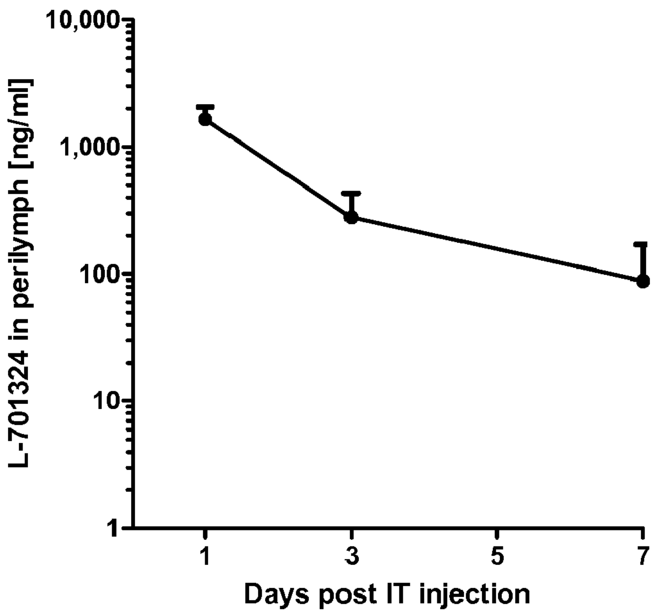


| | AUC<br>µg.h/ml | MRT<br>h | T1/2<br>h | Efficacy<br>ng/ml |
|---|---|---|---|---|
| **L-701324** | 108.0 | 44 | 40 | 1-10 |

Figure 11

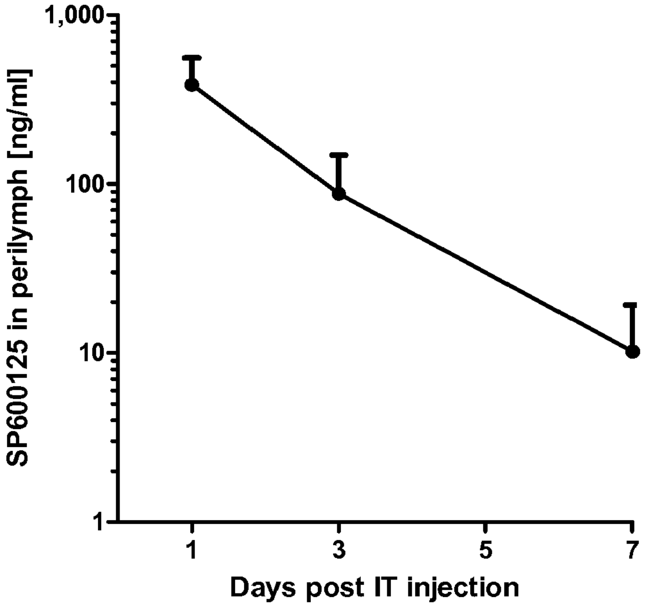


| | AUC | MRT | T1/2 | Efficacy |
|---|---|---|---|---|
| | μg.h/ml | h | h | ng/ml |
| **SP600125** | 25.8 | 38 | 32 | 10-50 |

Figure 12

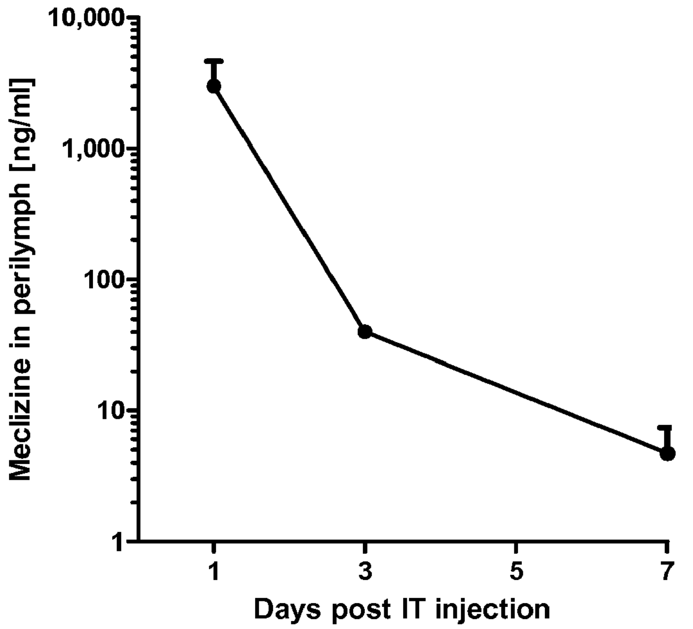


| | AUC<br>µg.h/ml | MRT<br>h | T1/2<br>h | Efficacy<br>ng/ml |
|---|---|---|---|---|
| Meclizine | 146.4 | 19 | 18 | 50-100 |

**Figure 13**

Cochlear Distribution of Dex from Gel

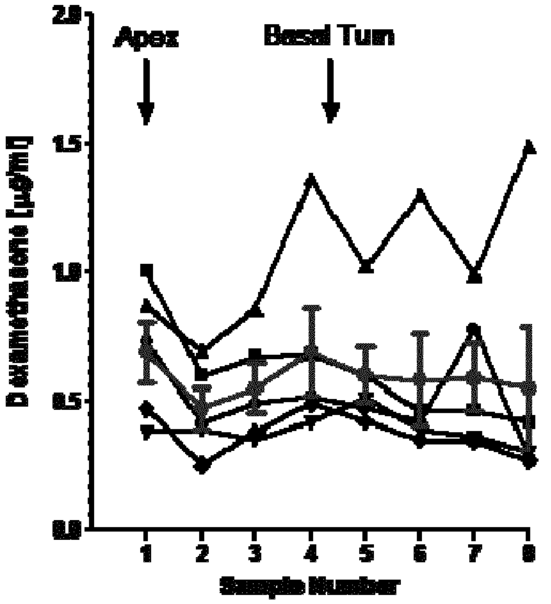


Cochlear Distribution of Dex from Solution

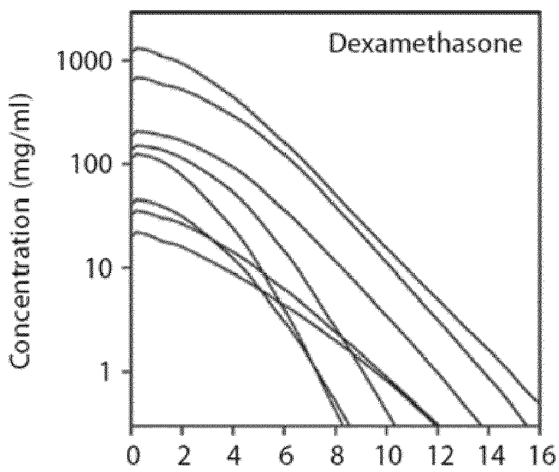


Salt and Plontke, Audiology & Neurotology (2009)

Figure 14

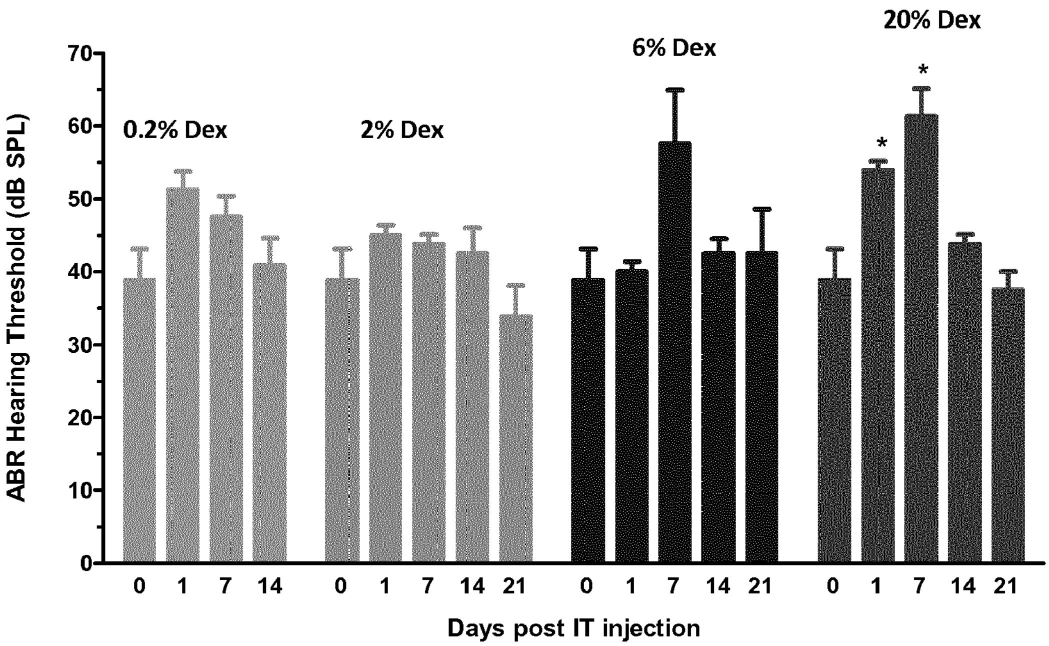
20% Dex
6% Dex
0.2% Dex
2% Dex
*
*
ABR Hearing Threshold (dB SPL)
70
60
50
40
30
20
10
0
0
1
7
14
0
1
7
14
21
0
1
7
14
21
0
1
7
14
21
Days post IT injection

Figure 15

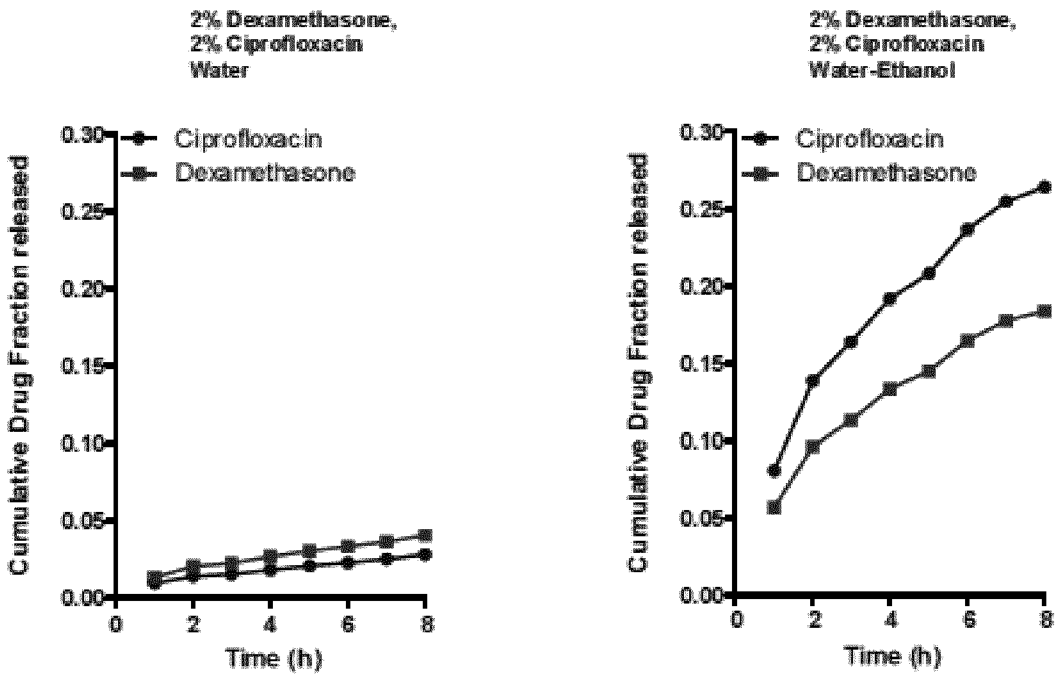


| | MDT (h) | |
|---|---|---|
| | Cipro | DEX |
| Pol407 + water | 200 | 137 |
| Pol407 + water-EtOH | 23 | 33 |

Figure 16

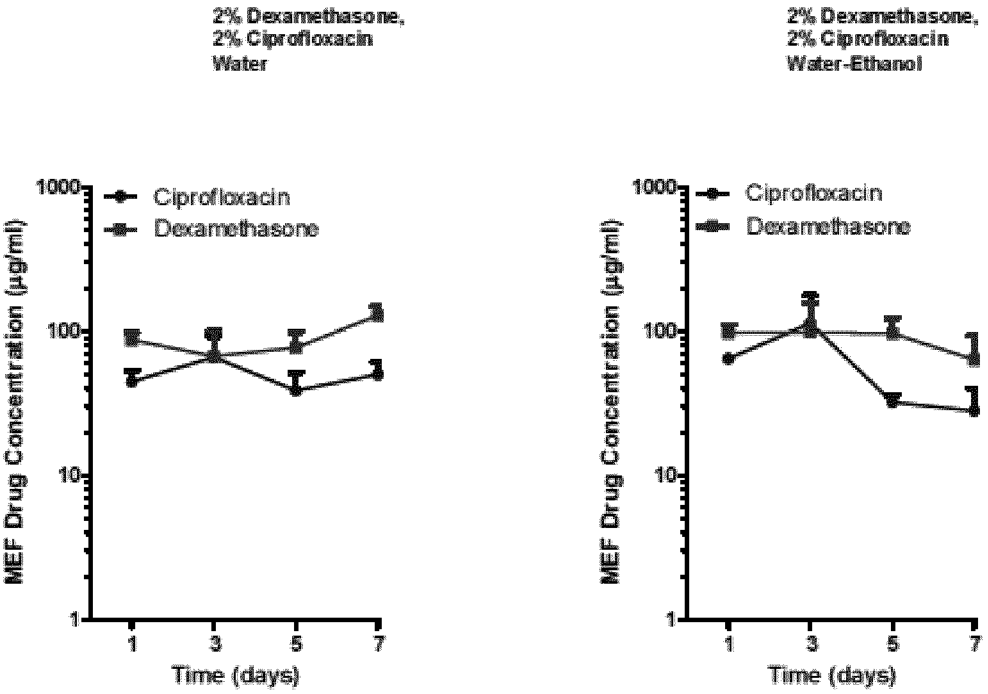
2% Dexamethasone,
2% Ciprofloxacin
Water
2% Dexamethasone,
2% Ciprofloxacin
Water-Ethanol
Ciprofloxacin
Dexamethasone
MEF Drug Concentration (µg/ml)
1000
100
10
1
1
3
5
7
Time (days)

# Figure 17

## (A) 15-18% P407 + water

Ciprofloxacin

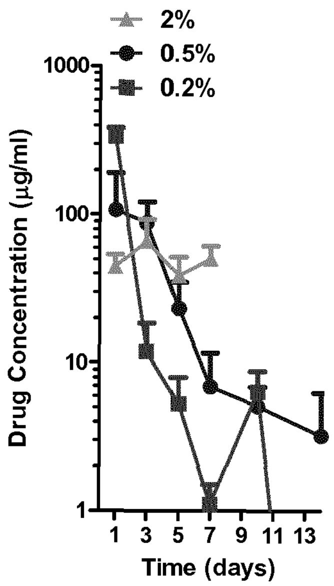


Dexamethansone

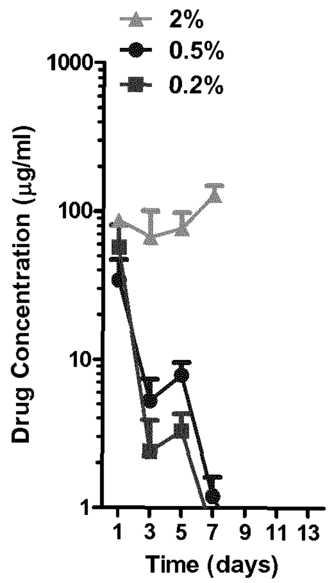


## (B) 50% P407 + water-ethanol

Ciprofloxacin

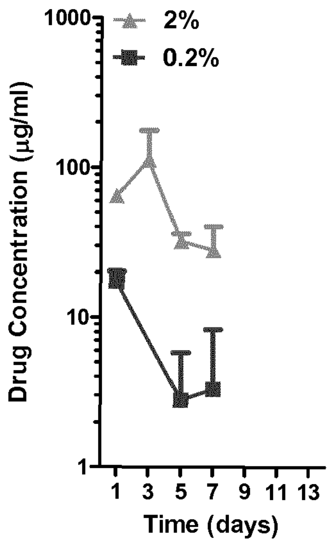


Dexamethasone

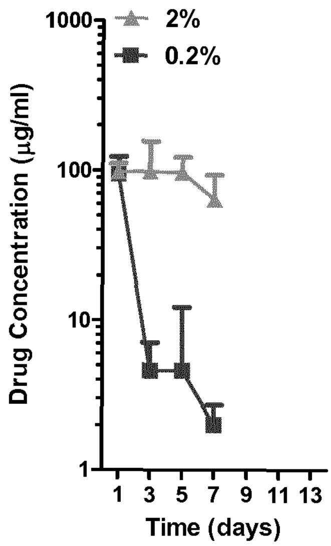

Figure 18

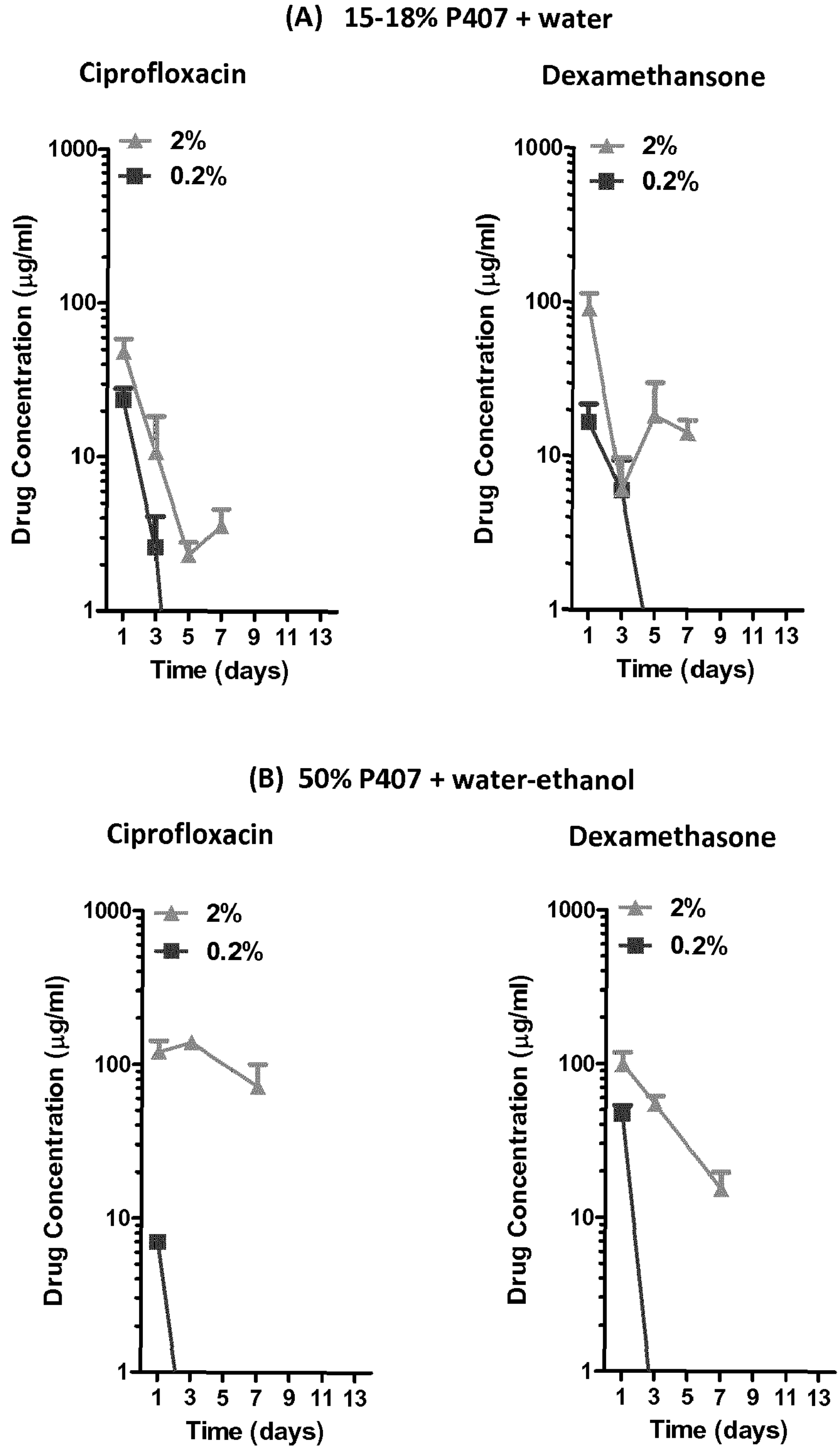
(A) 15-18% P407 + water
Ciprofloxacin
Dexamethansone
2%
0.2%
Drug Concentration (μg/ml)
1000
100
10
1
1 3 5 7 9 11 13
Time (days)
(B) 50% P407 + water-ethanol
Ciprofloxacin
Dexamethasone

Figure 19

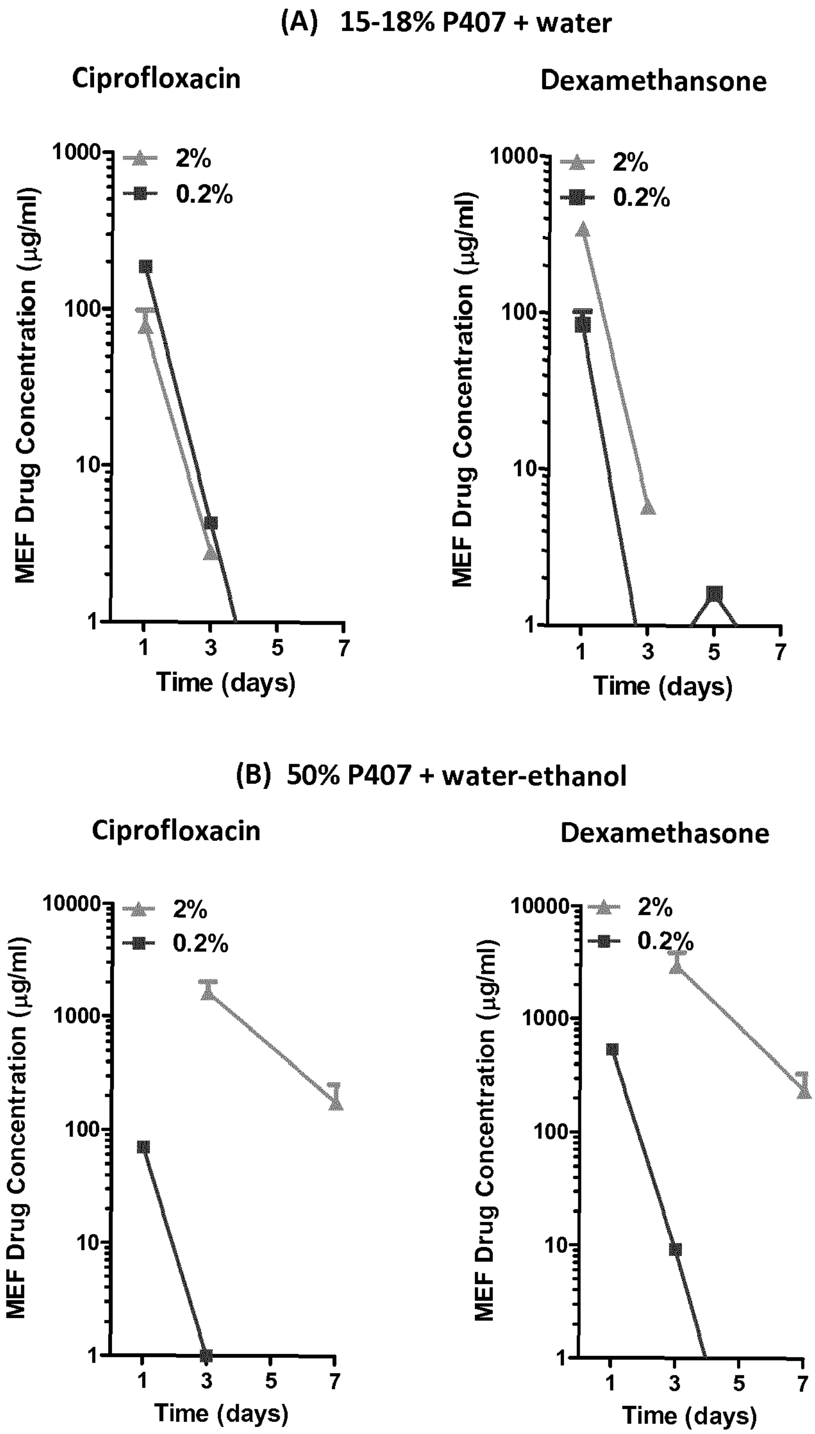
(A) 15-18% P407 + water
Ciprofloxacin
Dexamethansone
MEF Drug Concentration (µg/ml)
1000
100
10
1
2%
0.2%
1
3
5
7
Time (days)
(B) 50% P407 + water-ethanol
Ciprofloxacin
Dexamethasone
MEF Drug Concentration (µg/ml)
10000
1000
100
10
1
2%
0.2%
Time (days)

# Figure 20

| | CIPROFLOXACIN (DRY EAR) Dose µg | Cmax µg/ml | $AUC_{0-7}$ µg.h/ml | Exposure Ratio AUC/Dose | T1/2 h | CIPROFLOXACIN (WET EAR) Dose µg | Cmax µg/ml | $AUC_{0-7}$ µg.h/ml | Exposure Ratio AUC/Dose | T1/2 h |
|---|---|---|---|---|---|---|---|---|---|---|
| **Ciprodex Otic** | | | | | | | | | | |
| 0.3% Cipro HCl, 0.1% DEX | 150 | 3.6 | 340 | 2.3 | 163 | | | | | |
| 0.3% Cipro HCl, 0.1% DEX | 450 | 3.5 | 370 | 0.8 | 74 | | | | | |
| **15-18% Pol + water** | | | | | | | | | | |
| 0.2% Cipro HCl, 0.2% DEX | 100 | 337 | 17000 | 170.0 | 25 | 100 | 24 | 1260 | 12.6 | 19 |
| 0.5% Cipro HCl, 0.5% DEX | 250 | 107 | 10700 | 42.8 | 66 | | | | | |
| 2% Cipro HCl, 2% DEX | 1000 | 68 | 8400 | 8.4 | 576 | 1000 | 138 | 7300 | 7.3 | 20 |
| **50% Pol + water + ethanol** | | | | | | | | | | |
| 0.2% Cipro, 0.2% DEX | 100 | 18 | 1100 | 11.0 | 70 | 100 | 7 | 350 | 3.5 | 29 |
| 2% Cipro, 2% DEX | 1000 | 113 | 10800 | 10.8 | 139 | 1000 | 139 | 19300 | 19.3 | 226 |

| | DEXAMETHASONE (DRY EAR) Dose µg | Cmax µg/ml | $AUC_{0-7}$ µg.h/ml | Exposure Ratio AUC/Dose | T1/2 h | DEXAMETHASONE (WET EAR) Dose µg | Cmax µg/ml | $AUC_{0-7}$ µg.h/ml | Exposure Ratio AUC/Dose | T1/2 h |
|---|---|---|---|---|---|---|---|---|---|---|
| **Ciprodex Otic** | | | | | | | | | | |
| 0.3% Cipro HCl, 0.1% DEX | 150 | 2.4 | 170 | 1.1 | 38 | | | | | |
| 0.3% Cipro HCl, 0.1% DEX | 450 | 2.5 | 250 | 0.6 | 475 | | | | | |
| **15-18% Pol + water** | | | | | | | | | | |
| 0.2% Cipro HCl, 0.2% DEX | 100 | 57 | 3050 | 30.5 | 38 | 100 | 17 | 1100 | 11.0 | 30 |
| 0.5% Cipro HCl, 0.5% DEX | 250 | 34 | 3100 | 12.4 | 47 | | | | | |
| 2% Cipro HCl, 2% DEX | 1000 | 128 | 14200 | 14.2 | 685 | 1000 | 203 | 11250 | 11.3 | 35 |
| **50% Pol + water + ethanol** | | | | | | | | | | |
| 0.2% Cipro HCl, 0.2% DEX | 100 | 95 | 5030 | 50.3 | 30 | 100 | 47 | 2300 | 23.0 | 25 |
| 2% Cipro HCl, 2% DEX | 1000 | 98 | 15400 | 15.4 | 272 | 1000 | 100 | 9450 | 9.5 | 63 |

Figure 21

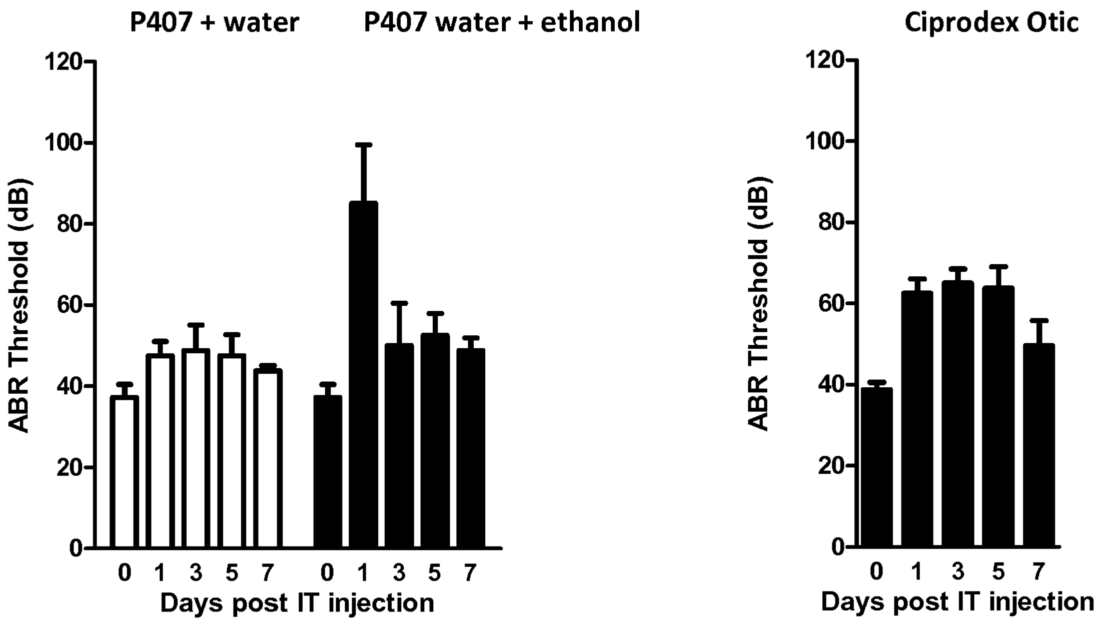
P407 + water
P407 water + ethanol
Ciprodex Otic
ABR Threshold (dB)
120
100
80
60
40
20
0
0 1 3 5 7 0 1 3 5 7
Days post IT injection
ABR Threshold (dB)
120
100
80
60
40
20
0
0 1 3 5 7
Days post IT injection

**Figure 22**

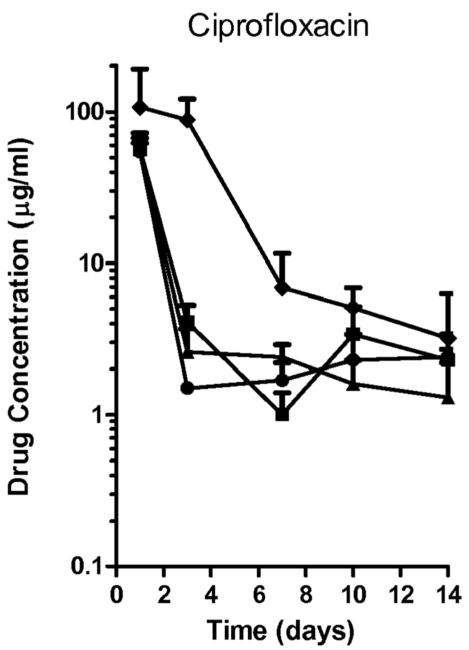
Ciprofloxacin
Drug Concentration (μg/ml)
100
10
1
0.1
0 2 4 6 8 10 12 14
Time (days)

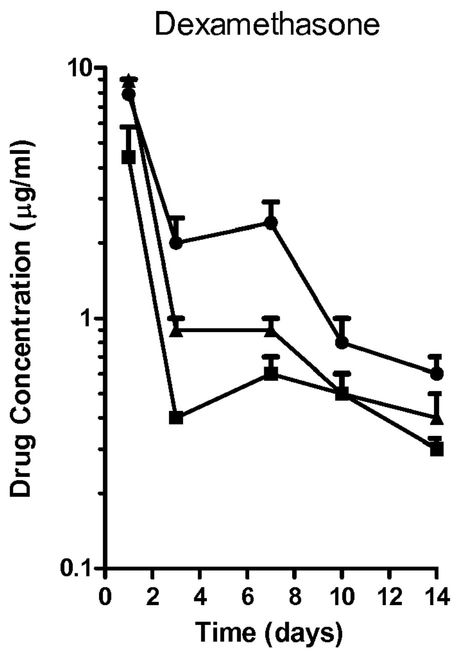
Dexamethasone
Drug Concentration (μg/ml)
10
1
0.1
0 2 4 6 8 10 12 14
Time (days)

Figure 23

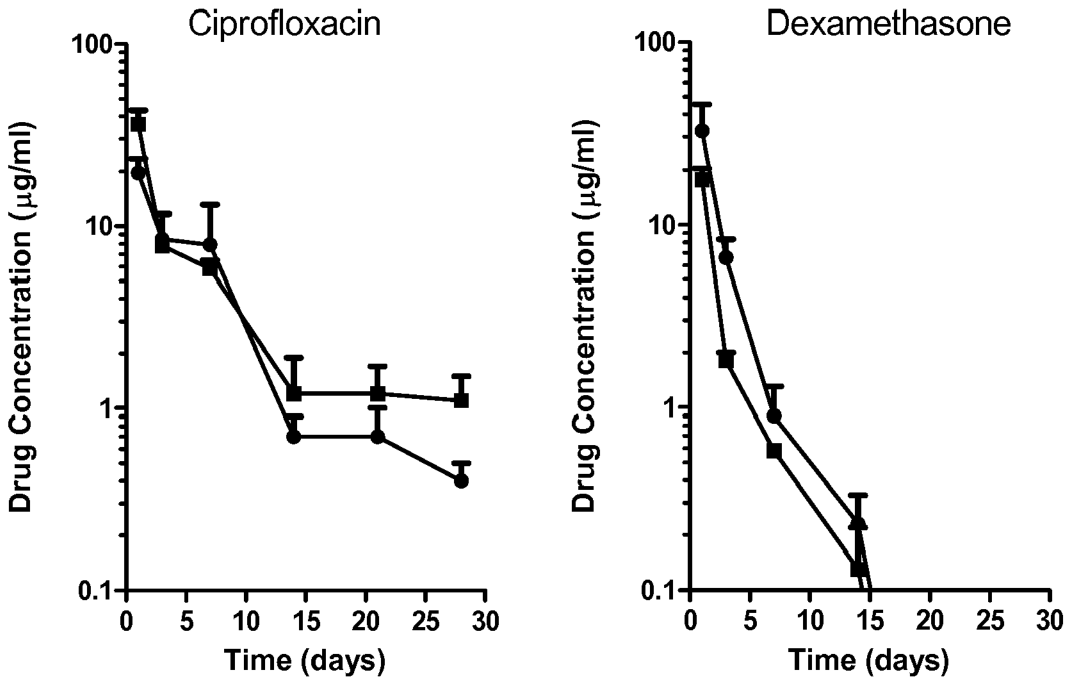
Ciprofloxacin
Drug Concentration (μg/ml)
100
10
1
0.1
0
5
10
15
20
25
30
Time (days)
Dexamethasone
Drug Concentration (μg/ml)
100
10
1
0.1
0
5
10
15
20
25
30
Time (days)

# Figure 24

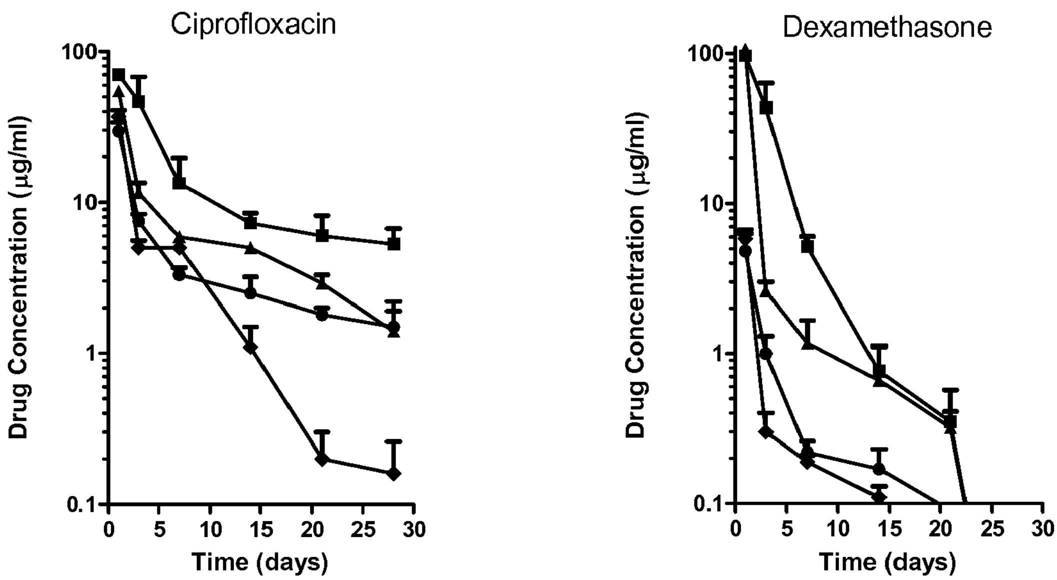
Ciprofloxacin
Drug Concentration (µg/ml)
100
10
1
0.1
0
5
10
15
20
25
30
Time (days)
Dexamethasone
Drug Concentration (µg/ml)
100
10
1
0.1
0
5
10
15
20
25
30
Time (days)

Figure 25

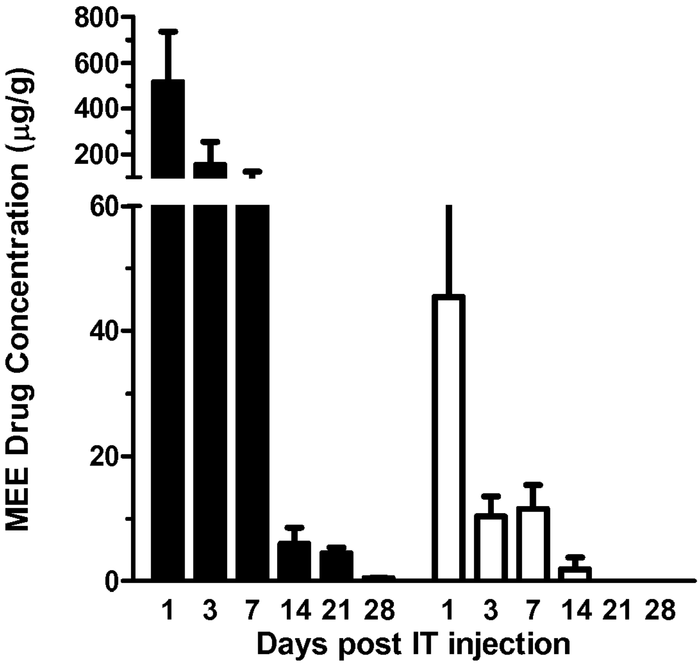
MEE Drug Concentration (μg/g)
800
600
400
200
60
40
20
0
1 3 7 14 21 28 1 3 7 14 21 28
Days post IT injection

Figure 26

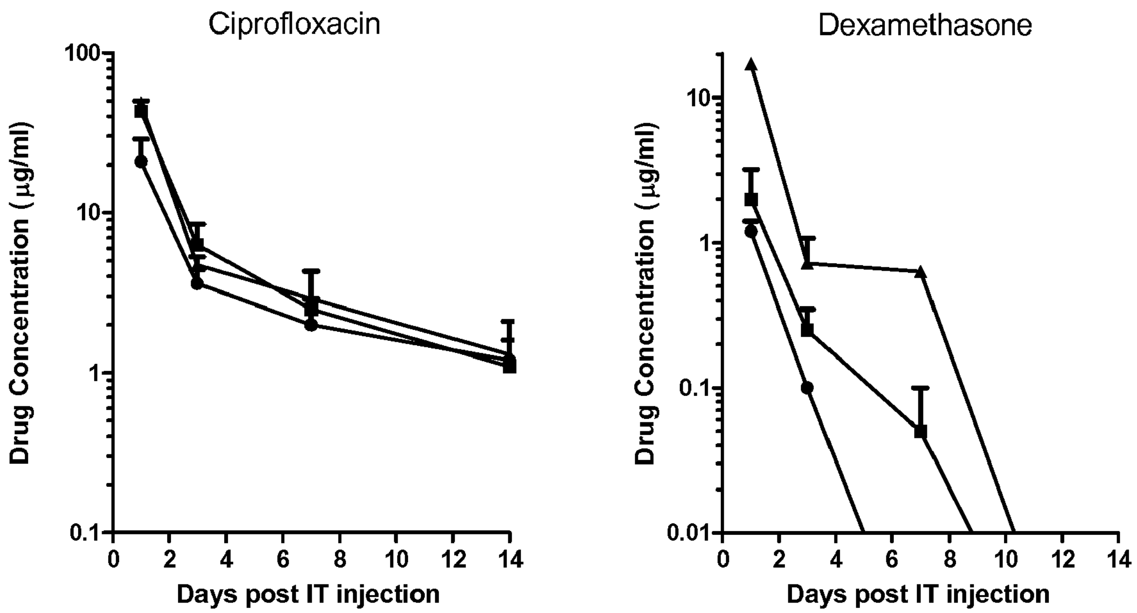
Ciprofloxacin
Drug Concentration (μg/ml)
100
10
1
0.1
0 2 4 6 8 10 12 14
Days post IT injection
Dexamethasone
Drug Concentration (μg/ml)
10
1
0.1
0.01
0 2 4 6 8 10 12 14
Days post IT injection

Figure 27

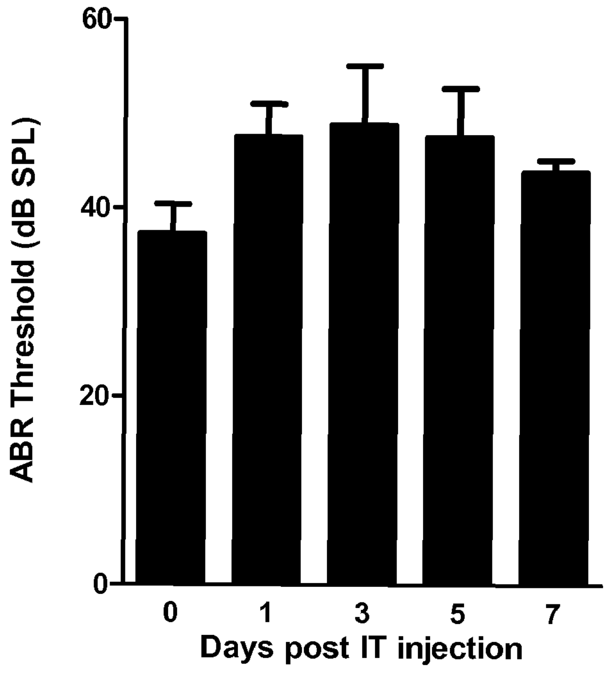

**Figure 28**

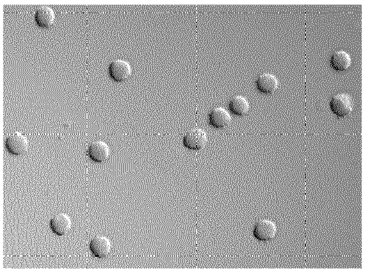

A: 0.45% NaCl Saline

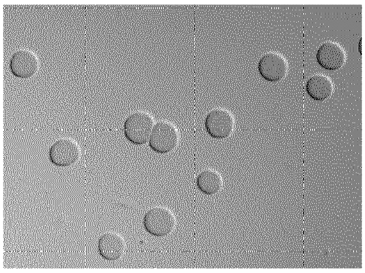

B: 0.9% NaCl Saline

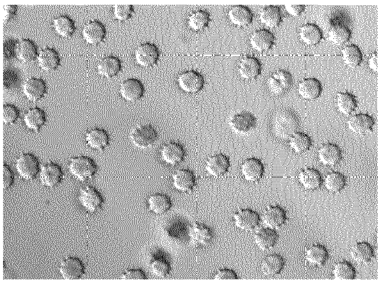

C: 2% NaCl Saline

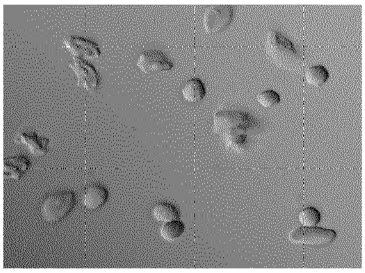

D: 10% NaCl Saline

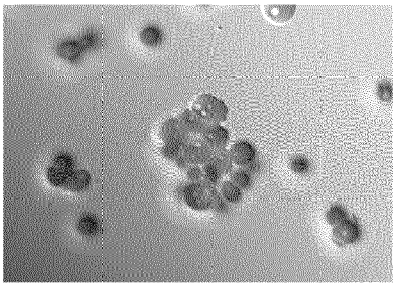

E: P407 in DI Water

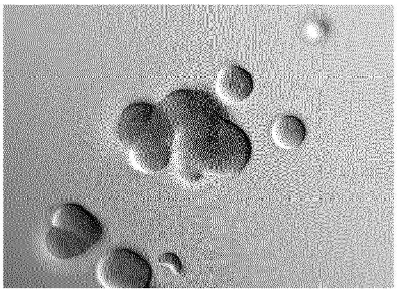

F: P407 in 50 mM TRIS, 0.3% NaCl

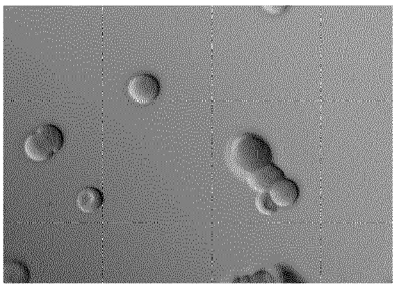

G: P407 in 50 mM TRIS, 0.45% NaCl

Europäisches Patentamt
European Patent Office
Office européen des brevets

# EUROPEAN SEARCH REPORT

Application Number
EP 18 20 4805

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | QI ET AL: "Development of a poloxamer analogs/carbopol-based in situ gelling and mucoadhesive ophthalmic delivery system for puerarin", INTERNATIONAL JOURNAL OF PHARMACEUTICS, ELSEVIER, NL, vol. 337, no. 1-2, 19 May 2007 (2007-05-19), pages 178-187, XP022085267, ISSN: 0378-5173, DOI: 10.1016/J.IJPHARM.2006.12.038<br>* abstract *<br>* page 179, right-hand column, paragraph 2 *<br>* page 180, left-hand column, paragraph 2 - page 180, right-hand column, paragraph 1 *<br>* page 180; table 1 *<br>----- | 1-5,7-9, 12,13 | INV.<br>A61K9/06<br>A61K47/10<br>A61K31/506<br>A61K38/18 |
| X | US 2002/099171 A1 (DE ROBERTIS EDWARD M [US] ET AL) 25 July 2002 (2002-07-25)<br>* page 1, paragraphs 3,10 *<br>* page 5, paragraph 56 *<br>----- | 15 | TECHNICAL FIELDS SEARCHED (IPC)<br>A61K |
| A | RIBEIRO M E N P ET AL: "Solubilisation of griseofulvin, quercetin and rutin in micellar formulations of triblock copolymers E62P39E62 and E137S18E137", INTERNATIONAL JOURNAL OF PHARMACEUTICS, ELSEVIER, NL, vol. 378, no. 1-2, 13 August 2009 (2009-08-13), pages 211-214, XP026348619, ISSN: 0378-5173<br>* the whole document *<br>-----<br>-/-- | 1-15 | |

The present search report has been drawn up for all claims

| Place of search | Date of completion of the search | Examiner |
|---|---|---|
| The Hague | 24 May 2019 | Gómez Gallardo, S |

1

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 18 20 4805

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | E.-S. A. IBRAHIM ET AL: "Formulation and evaluation of quercetin in certain dermatological preparations", JOURNAL OF DRUG DELIVERY SCIENCE AND TECHNOLOGY, vol. 17, no. 6, 1 January 2007 (2007-01-01), pages 431-436, XP055338478, FR ISSN: 1773-2247, DOI: 10.1016/S1773-2247(07)50084-5 * the whole document * ----- | 1-15 | |
| A | US 2004/248803 A1 (NUSSE ROELAND [US] ET AL) 9 December 2004 (2004-12-09) * page 1, paragraph 7-9 * * page 7, paragraph 66 * ----- | 1-15 | |
| A | WO 2008/125872 A1 (RENOVO LTD [GB]; FERGUSON MARK WILLIAM JAMES [GB] ET AL.) 23 October 2008 (2008-10-23) * page 4, paragraph 3 * * claims 1-22 * ----- | 1-15 | TECHNICAL FIELDS SEARCHED (IPC) |
| A | WO 2006/026570 A2 (MASSACHUSETTS EYE & EAR INFIRM [US]; LI HUAWEI [CN] ET AL.) 9 March 2006 (2006-03-09) * page 1, lines 10-13 * * page 5, line 3 - page 6, line 2 * * page 27, lines 13-28 * * claims 13-22 * ----- | 1-15 | |
| A | US 4 188 373 A (KREZANOSKI JOSEPH Z [US]) 12 February 1980 (1980-02-12) * the whole document * ----- -/-- | 1-15 | |

The present search report has been drawn up for all claims

1

| Place of search | Date of completion of the search | Examiner |
|---|---|---|
| The Hague | 24 May 2019 | Gómez Gallardo, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 3

Europäisches Patentamt
European Patent Office
Office européen des brevets

# EUROPEAN SEARCH REPORT

Application Number
EP 18 20 4805

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CHUNG ET AL: "Thermo-sensitive and biodegradable hydrogels based on stereocomplexed Pluronic multi-block copolymers for controlled protein delivery", JOURNAL OF CONTROLLED RELEASE, ELSEVIER, AMSTERDAM, NL, vol. 127, no. 1, 23 December 2007 (2007-12-23), pages 22-30, XP022523522, ISSN: 0168-3659, DOI: 10.1016/J.JCONREL.2007.12.008 * the whole document * ----- | 1-15 | |
| A | LIU J ET AL: "A small-molecule agonist of the Wnt signaling pathway", ANGEWANDTE CHEMIE, INTERNATIONAL EDITION, WILEY-VCH, DE, vol. 44, no. 13, 18 March 2005 (2005-03-18), pages 1987-1990, XP002495891, ISSN: 1433-7851, DOI: 10.1002/ANIE.200462552 * the whole document * ----- | 1-15 | TECHNICAL FIELDS SEARCHED (IPC) |
| X,P | WO 2009/132050 A2 (OTONOMY INC [US]; UNIV CALIFORNIA [US] ET AL.) 29 October 2009 (2009-10-29) * page 3, paragraph 10 - page 4, paragraph 12 * * page 107, paragraph 458 * ----- | 1-14 | |

1

The present search report has been drawn up for all claims

| Place of search | Date of completion of the search | Examiner |
|---|---|---|
| The Hague | 24 May 2019 | Gómez Gallardo, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 3 of 3

## ANNEX TO THE EUROPEAN SEARCH REPORT ON EUROPEAN PATENT APPLICATION NO. EP 18 20 4805

This annex lists the patent family members relating to the patent documents cited in the above-mentioned European search report. The members are as contained in the European Patent Office EDP file on
The European Patent Office is in no way liable for these particulars which are merely given for the purpose of information.

24-05-2019

| Patent document cited in search report | Publication date | Patent family member(s) | Publication date |
|---|---|---|---|
| US 2002099171 A1 | 25-07-2002 | US 2002099171 A1 | 25-07-2002 |
| | | US 2002099172 A1 | 25-07-2002 |
| | | US 2002123613 A1 | 05-09-2002 |
| | | US 2002128439 A1 | 12-09-2002 |
| | | US 2002128440 A1 | 12-09-2002 |
| | | US 2002128441 A1 | 12-09-2002 |
| | | US 2002156249 A1 | 24-10-2002 |
| US 2004248803 A1 | 09-12-2004 | US 2004248803 A1 | 09-12-2004 |
| | | US 2007105776 A1 | 10-05-2007 |
| | | US 2007269890 A1 | 22-11-2007 |
| | | US 2009275134 A1 | 05-11-2009 |
| | | US 2014308744 A1 | 16-10-2014 |
| | | US 2015344846 A1 | 03-12-2015 |
| | | US 2016097031 A1 | 07-04-2016 |
| | | US 2018002661 A1 | 04-01-2018 |
| | | WO 2004091647 A1 | 28-10-2004 |
| WO 2008125872 A1 | 23-10-2008 | NONE | |
| WO 2006026570 A2 | 09-03-2006 | US 2005287127 A1 | 29-12-2005 |
| | | US 2008267929 A1 | 30-10-2008 |
| | | US 2009124568 A1 | 14-05-2009 |
| | | US 2015030568 A1 | 29-01-2015 |
| | | WO 2006026570 A2 | 09-03-2006 |
| US 4188373 A | 12-02-1980 | NONE | |
| WO 2009132050 A2 | 29-10-2009 | AU 2009239429 A1 | 29-10-2009 |
| | | CA 2721927 A1 | 29-10-2009 |
| | | CN 102014957 A | 13-04-2011 |
| | | CN 104491864 A | 08-04-2015 |
| | | EP 2278999 A2 | 02-02-2011 |
| | | HK 1209055 A1 | 24-03-2016 |
| | | IL 208549 A | 30-04-2017 |
| | | JP 6147653 B2 | 14-06-2017 |
| | | JP 6296565 B2 | 20-03-2018 |
| | | JP 2011518195 A | 23-06-2011 |
| | | JP 2014058557 A | 03-04-2014 |
| | | JP 2016026218 A | 12-02-2016 |
| | | JP 2017119723 A | 06-07-2017 |
| | | KR 20100135299 A | 24-12-2010 |
| | | KR 20130097813 A | 03-09-2013 |
| | | KR 20160029870 A | 15-03-2016 |
| | | KR 20190026056 A | 12-03-2019 |
| | | RU 2010147298 A | 27-05-2012 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 18 20 4805

This annex lists the patent family members relating to the patent documents cited in the above-mentioned European search report.
The members are as contained in the European Patent Office EDP file on
The European Patent Office is in no way liable for these particulars which are merely given for the purpose of information.

24-05-2019

| Patent document cited in search report | Publication date | Patent family member(s) | Publication date |
|---|---|---|---|
| | | US 2009306225 A1 | 10-12-2009 |
| | | US 2016095902 A1 | 07-04-2016 |
| | | US 2016228357 A1 | 11-08-2016 |
| | | US 2016243028 A1 | 25-08-2016 |
| | | WO 2009132050 A2 | 29-10-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 61253782 A
- US 61255379 A
- US 61255780 A
- US 61255783 A
- US 61297138 A
- US 61297170 A
- US 61364288 A
- US 61366677 A
- US 20080269449 A
- US 7148320 B
- US 5800711 A
- US 6977045 B
- US 6761824 B
- US 427663 A
- US 12466310 B
- US 12472034 B
- US 12486697 B
- US 12493611 B
- US 12494156 B
- US 12500486 B
- US 12504553 B
- US 12506091 B
- US 12506127 B
- US 12506573 B
- US 12506616 B
- US 12506664 B
- US 472034 A
- US 12427663 B
- US 493611 A
- US 500486 A
- US 506664 A
- US 486697 A
- US 6004573 A
- US 6117949 A
- US 6201072 B
- US 6287588 B
- US 906041 A
- US 09559799 B
- US 10919603 B
- US 5567859 A
- WO 9216484 A
- US 5523492 A
- US 5696298 A
- WO 1998017285 A


### Non-patent literature cited in the description


- *PNAS,* 24 June 2003, vol. 100 (13), 7971-7976
- *Mol. Pharmacol.,* 2003, vol. 64, 731-740
- **SAMBROOK et al.** Molecular Cloning: A Laboratory Manual. 1989
- **SAMBROOK ; RUSSEL.** Molecular Cloning: A Laboratory Manual. 2001
- Current Protocols in Molecular Biology. 1987
- Current Protocols in Nucleic Acid Chemistry. John Wiley & Sons, Inc, 2000
- *Molecular and Cellular Neuroscience,* 2005, vol. 28, 253-263
- **VIEGAS.** *Int. J. Pharm.,* 1998, vol. 160, 157-162
- **BASAVOJU.** norfloxacin is known to form cocrystal. *Crystal Growth & Design,* 2006, vol. 6 (12
- **JEONG et al.** *Nature,* 1997, vol. 388, 860-2
- **JEONG et al.** *J. Control. Release,* 2000, vol. 63, 155-63
- **JEONG et al.** *Adv. Drug Delivery Rev.,* 2002, vol. 54, 37-51
- **LI XIN-YU.** *Acta Pharmaceutica Sinica,* 2008, vol. 43 (2), 208-203
- **CHIU et al.** *American Journal of Rhinology,* 2007, vol. 21, 5-9